(19) Europäisches Patentamt
European Patent Office
Office européen des brevets

(11) Publication number: **0 382 411 B1**

(12) # EUROPEAN PATENT SPECIFICATION

(45) Date of publication of patent specification: **07.12.94**

(51) Int. Cl.$^5$: **A61K 47/48**

(21) Application number: **90301072.6**

(22) Date of filing: **01.02.90**

The file contains technical information submitted after the application was filed and not included in this specification

(54) **Delivery of cytotoxic agents.**

(30) Priority: **02.02.89 US 305824**
**02.02.89 US 305900**

(43) Date of publication of application:
**16.08.90 Bulletin 90/33**

(45) Publication of the grant of the patent:
**07.12.94 Bulletin 94/49**

(84) Designated Contracting States:
**AT BE CH DE ES FR GB IT LI NL SE**

(56) References cited:
**EP-A- 0 055 870** **EP-A- 0 075 452**
**EP-A- 0 251 330** **EP-A- 0 302 473**
**EP-A- 0 392 745** **WO-A-88/07378**

**Lexikon der Biochemie und Molekularbiologie, 1991, Herder. Freiburg-basel-Wien**

(73) Proprietor: **ELI LILLY AND COMPANY**
**Lilly Corporate Center**
**Indianapolis Indiana 46285 (US)**

Proprietor: **HYBRITECH INCORPORATED**
**11095 Torreyana Road**
**San Diego California 92126 (US)**

(72) Inventor: **Chen, Victor John**
**8131 Menlo Court,**
**East Drive**
**Indianapolis, Indiana 46140 (US)**
Inventor: **Jungheim, Louis Nickolaus**
**5706 N Washington Boulevard**
**Indianapolis, Indiana 46220 (US)**
Inventor: **Meyer, Damon Lawrence**
**14360 Janal way**
**San Diego, California 92129 (US)**
Inventor: **Shepherd, Timothy Alan**
**117 East Sherry Lane**
**Indianapolis, Indiana 46227 (US)**

(74) Representative: **Tapping, Kenneth George et al**
**Lilly Industries Limited**
**European Patent Operations**
**Erl Wood Manor**
**Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Over the years, many cytotoxic drugs useful for treating neoplastic diseases have been developed. The chief drawback of all of the useful drugs has been the indiscriminant toxicity of these drugs to all cells of the patient, thus limiting their dosage and consequently the effectiveness of the drugs. The present invention, whereby a latent form of the Cytotoxic Agent is activated at the site of the neoplastic disease, offers a marked advantage in diminishing or possibly eliminating unwanted side effects of the Agent. As a further consequence of reduced deleterious side effects, higher concentrations of the drug at the tumor site may be brought about through administration of the Cytotoxic Agent at increased dosage.

The present invention provides means for a method for the treatment of neoplastic diseases, which comprises:

A) administering a therapeutically effective amount of an Antibody-Enzyme conjugate to the affected host; and then

B) administering a therapeutically effective amount of a Substrate-Cytotoxic Agent to the affected host, wherein the Substrate is a substrate for the Enzyme; wherein the Antibody-Enzyme conjugate and the Substrate-Cytotoxic Agent are as defined below.

Other aspects of the invention include the Antibody-Enzyme conjugates and the Substrate-Cytotoxic Agent compounds as defined below.

One aspect of this invention is an enzyme-antibody conjugate of Formula (I):

Antibody-Enzyme (I)

wherein the Enzyme reacts with a compound of Formula (II):

Substrate-Cytotoxic Agent (II),

which compound is described below, such that the Substrate is separated from the Cytotoxic Agent; and the Antibody complexes with the target tissue.

EP-A-302473 and WO-A-8807378 disclose combinations of an Antibody-Enzyme conjugate and a Substrate-Cytotoxic Agent.

### The Antibody

The antibody of the present invention complexes with one or more antigens of the target cells. The target cells are a part of neoplastic tissues, whether benign or malignant. Examples of such target cells include psoriasis, squamous carcinoma cells, adenocarcinoma cells, small cell carcinoma cells, glioma cells, melanoma cells, renal cell carcinoma cells, transitional cell carcinoma cells, sarcoma cells, cells of supporting tumor vasculature, and cells of lymphoid tumors such as leukemias and lymphomas.

The antibody may be chosen from any class or subclass of immunoglobulin including IgG, IgA, IgM, IgE, and IgD. The species of origin is not critical so long as the antibody complexes with target cells.

In the present state of the art, monoclonal antibodies are most preferred for use in the present invention. However, polyclonal antibodies are not excluded. A newer type of antibody is the chimeric antibody, which is prepared in the laboratory by recombinant technology which permits expression of a modified DNA which encodes the antigen-binding region of any desired antibody, and also encodes any other desired amino acid sequences. Thus, chimeric antibodies of which one portion is derived from one species, and another portion is derived from a different species, may be obtained and used in the present invention. New antibody derivatives secreted from bacteria have been described which also may be used in the present invention. In this regard, see E.S. Ward, et al., Nature 341 544 (1989).

The origin and nature of the antibody is not otherwise critical, so long as it targets the cell to be treated and is not, in itself, toxic to the patient. Those of ordinary skill can readily prepare enzyme conjugates with a candidate antibody and evaluate them. How such antibodies are chosen will be discussed in detail below.

It will be understood that properly chosen fragments of antibodies have the same effect as the antibody. Thus, in the practice of this invention, fragments of antibodies, such as $F(ab')_2$ and especially F(ab') fragments, which recognize an antigen associated with the cell to be treated, may be just as useful as are intact antibodies.

A great number of antibodies are available to immunologists for use in the present invention, and further useful antibodies are being disclosed in every issue of the relevant journals. It is impossible, and entirely unnecessary, to give an exhaustive listing of antibodies which can be applied in the practice of this invention. Immunologists and chemists of ordinary skill are entirely able to choose antibodies from sources such as the catalogue of the American Type Culture Collection, Rockville, Maryland, U.S.A., and Linscott's Directory of Immunological and Biological Reagents, published by Linscott's Directory, 40 Glen Drive, Mill Valley, California, U.S.A., 94941. Thus, it is a simple matter for the artisan in the field to choose an antibody

against virtually any antigen on the present target cells. Alternatively, the necessary hybridomas for the production of such antibodies are obtainable through the ATCC or the Northern Regional Research Laboratories of the U.S. Department of Agriculture, located in Peoria, Illinois, and other cell line collections.

A number of presently known antibodies are particularly interesting for use in the present invention. One preferred specific antibody is L/1C, produced by ATCC hybridoma HB9682 (deposited Apr. 5, 1988). Another preferred is produced by ATCC hybridoma HB 9620 (deposited Jan. 7, 1988), which antibody (designated CEM231.6.7) complexes with a convenient carcinoembryonic antigen expressed by several types of tumor cells. Recently, chimeric antibody CEM231.6.7 has been described in U.S. Patent Application Nos. 07/165,856, corresponding to published EPO Application, EP A 0 332 424 (Sept. 13, 1989). The antibody is also discussed in C.B. Beidler et al., J. Immunology, 141, pp. 4053-4060 (1988). Another Antibody which complexes with the CEA marker is the T101 antibody. The T101 antibody is produced by ATCC hybridoma No. CRL 8023 and is described in U.S. Patent No. 4,675,386. T101 is also available from Boehringer-Mannheim Co. (Indianapolis, IN).

Another interesting antibody is KS1/4, first disclosed by Varki et al., Cancer Research 44, 681-86 (1984). A number of plasmids which comprise the coding sequences of the different regions of monoclonal antibody KS1/4 are now on deposit and can be obtained from the Northern Regional Research Laboratory, Peoria, Illinois, U.S.A. The plasmids can be used by those of ordinary skill to produce chimeric antibodies by recombinant means, which antibodies bind to a cell surface antigen found in high density on adenocarcinoma cells. The construction of such antibodies is discussed in detail in U.S. Patent application 07/184,522, filed April 21, 1988, corresponding to published EPO Application, EP A 0 338 767 (Oct. 25, 1989). The following plasmids relate to KS1/4:

Plasmids pGKC2310, the coding sequence of the light chain, the signal peptide associated with the light chain, and the 5′ and 3′ untranslated regions; isolated from E. coli K12 MM294/pGKC2310, NRRL B-18356 - (deposited April 1, 1988).

Plasmids pG2A52, the coding sequence of the heavy chain, the coding sequence of the signal peptide associated with the heavy chain, and the 5′ and 3′ untranslated regions; isolated from E. coli K12 MM294/pG2A52, NRRL B-18357 (deposited April 1, 1988).

Plasmid CHKC2-6, the coding sequence of the light chain variable region, the coding sequence of the signal peptide associated with the light chain, and a sequence encoding the light chain constant region of a human IgG; isolated from E. coli K12 DH5/CHKC2-6, NRRL B-18358 (deposited April 1, 1988).

Plasmid CHKC2-18, the coding sequence of a derivative light chain variable region, the coding sequence of the signal peptide associated with the light chain, and a sequence encoding the light chain constant region of a human IgG; isolated from E. coli K12 DH5/CHKC2-18, NRRL B-18359 (deposited April 1, 1988).

Plasmid CH2A5, the coding sequence of the heavy chain variable region, the coding sequence of the signal peptide associated with the heavy chain, and a sequence encoding the heavy chain constant region of human IgG1; isolated from E. coli K12 MM294/CH2A5, NRRL B-18360 (deposited April 1, 1988).

Plasmid CH2A5IG2, the coding sequence of the heavy chain variable region, the coding sequence of the signal peptide associated with the heavy chain, and a sequence which encodes the heavy chain constant region of human IgG2; isolated from E. coli K12 DH5/CH2A5IG2, NRRL B-18361 (deposited April 1, 1988).

Plasmid CH2A5IG3, the coding sequence of the heavy chain variable region, the coding sequence of the signal peptide associated with the heavy chain, and a sequence encoding the heavy chain constant region of human IgG3; isolated from E. coli K12 DH5/CH2A5IG3, NRRL B-18362 (deposited April 1, 1988).

Plasmid CH2A5IG4, the coding sequence of the heavy chain variable region, the coding sequence of the signal peptide associated with the heavy chain, and a sequence encoding the heavy chain constant region of human IgG4; isolated from E. coli K12 DH5/CH2A5IG4, NRRL B-18363 (deposited April 1, 1988).

Antibody 5E9C11, produced by available ATCC hybridoma No. HB21, recognizes transferrin receptor, which is expressed by many tumors. An antibody called B72.3, available from the National Cancer Institute, recognizes antigens expressed by both breast and colon carcinoma. Also antibody ZCE 025 is suitable for use in this invention. The antibody was first described by Jean-Pierre Mach and his group (see, for example, Mach, J.P. et al., Int. J. Cancer, 33, pp. 643-649 (1984) and Patt, Y.Z. et al., Cancer Bull., 40:218-221 (1988).

## The Enzyme

The Enzyme for the present conjugate is a beta-lactamase. It recognizes the substrate, and will also recognize that substrate when it is bound to the present Cytotoxic Agent. Furthermore, the Enzyme retains

its activity once conjugated to the present Antibody.

The beta-lactamases that can be used in this invention catalyze a reaction in which the Substrate-Cytotoxic Agent compound is cleaved into its two component parts. The activity of the Enzyme toward the Substrate must be substantially unimpaired by the presence of the Cytotoxic Agent. The Enzyme for this type of reaction is practically insensitive to the presence of the Cytotoxic Agent, regardless of the structure of the Agent. The Enzyme allows that particular conjugate to be used for a variety of Substrate-Cytotoxic Agent combinations. Beta-lactamases recognize as substrates and cephalosporin compounds and separate these substrates from any other molecules to which they are bound. Inherent in these desired properties for the beta-lactamases is the trait that the enzyme reacts only with one particular functional group, i.e., the functional group that links the Substrate and Cytotoxic Agent, such that the Cytotoxic Agent can be substituted with any number of different functional groups that are unaffected by the Enzyme. The penicillins, cephalosporins, cephalosporin sulfoxides, 1-oxadethiacephalosporins, and 1-carbadethiacephalosporins that are its substrates are composed of a number of functional groups that are untouched by the Enzyme. The beta-lactamase simply reacts with the $\beta$-lactam ring of these substrate compounds. As a result of this reaction, the 3′-substituent of the Substrate will often cleaved from the rest of the cephalosporin nucleus.

The purpose of the enzyme is to generate the Cytotoxic Agent in vivo (and in high concentration at the loci of the Antibody-Enzyme conjugate) after the Cytotoxic Agent has been administered to the patient masked in the form of a Substrate-Cytotoxic Agent compound. Enzymes that can be used for the instant invention include the various beta-lactamases, regardless of the species of microorganism from which the enzyme is isolated.

The methods for conjugating the Enzyme and Antibody are well known in the art. See, for example, a listing of the protein-protein coupling reagents commercially available from Pierce Chemical Company, Rockford, Illinois. The various functional groups employed for enzyme-antibody bonding are generally not important for the purposes of this invention. All that is required is that the Antibody retain a substantial part of the immunoreactivity of its unconjugated state. Similarly, all that is required of the conjugated Enzyme is that it retain a substantial portion of its activity from its unconjugated state. Finally, both the conjugate and a newly formed Substrate-Cytotoxic Agent must demonstrate practical pharmacokinetics in the affected host. Reagents that can be used in conjugating the enzyme to the antibody include SMCC, sulfo-SMCC, SPDP, 2-imino-thiolane, MBS, sulfo-MBS, SMPB, Sulfo-SMPB, SIAB, sulfo-SIAB, GMBS, EMCS, N,N′-bis(3-maleimidopropionyl)-2-hydroxy-1,3-propanediamine, N-succinylbromoacetate (Sigma Chemical Co.), BMME (Boehringer Mannheim, Indianapolis, In.) and the like. Depending on the particular functional groups available for reaction on both the antibody and the enzyme, the antibody fragment chosen, and the reactivity of residues in or near the enzyme active site and antibody binding site, these reagents may be used in combination. Also, reagents that crosslink between two amino groups can be used, such as DMA or DSS (Pierce Chemical Co.). Certain enzymes and antibodies may have amino, sulfhydryl, or hydroxyl groups in their active sites or binding sites, respectively. Thus, these reactive functional groups can first be masked with protecting groups such as citraconic anhydride, DTNB, and DNFB (according to procedures in referenced in the Pierce Chemical Co. Catalog, Rockville, Illinois, and Schmuel and Shaltiel, Biochem. and Biophys Res. Comm., 29, pp178-183 (1967)) before they are conjugated with, for example, the reagents listed above. The Pierce Chemical Co. catalog lists references describing the use of many of the reagents above. The use of any remaining reagents may be found in the scientific literature.

Although any combination of Antibody and Enzyme as described above is sufficient for the present invention, the preferred conjugates are ones that are as homogenous as possible given the conjugation technology. Unlike whole antibody, F(ab′) fragments may be derivitized regiospecifically through the thiol residue which is found near the carboxy terminus and away from the antigen binding site, and are preferred in the invention. Furthermore, the conjugate should not be metabolized in organs such as the liver and the spleen, an event that could prevent sufficient conjugate from accumulating at the target tissue, yet, on the other hand, it is desirable that the conjugate be rapidly cleared from the bloodstream after the conjugate has had the opportunity to localize to the target tissue. Both of these pharmacokinetic properties reduce the chance that Cytotoxic Agent will be generated at a site other than the target tissue. These pharmacokinetic properties are facilitated by a conjugate with a low molecular weight, a property supplied by Antibody fragments, and particularly F(ab′) fragments. Also, Enzymes with a low molecular weight (e.g., 50,000 or less) are preferred for the same reason as the Antibody fragments.

The Enzyme of the present Antibody-Enzyme conjugate:

is beta-lactamase.

Preferred embodiments of the present Antibody-Enzyme conjugate include:

C. A conjugate as described above, wherein the Antibody complexes with antigens expressed solely, or in supernatural abundance, by malignant tumor cells;

D. A conjugate of C, wherein the Antibody complexes with carcinoembryonic antigen;

E. A conjugate of C, wherein the Antibody complexes with the KS1/4 antigen;

F. A conjugate of D, wherein the Antibody is designated ZCE025;

G. A conjugate of D, wherein the Antibody is designated CEM 231.6.7;

H. A conjugate of C, wherein the Antibody is designated T101; and

I. A conjugate of C, D, E, F, G, or H, wherein the Antibody is a F(ab') fragment and the Enzyme is a beta-lactamase from Enterobacter cloacae.

A further aspect of this invention are compounds of the Formula (II):

Substrate-Cytotoxic Agent (II)

wherein the Substrate is the Substrate for a beta-lactamase Enzyme, or active fragment thereof, wherein reaction of said Enzyme with the Substrate-Cytotoxic Drug causes the chemical separation of the Substrate from the Cytotoxic Agent; and the Substrate and the Cytotoxic Agent are bonded together through an ether, thioether, ester, amide, amino, hydrazido, carbonate, carbamate, thiocarbamate, thioester, thioamide, or thiocarbonate group formed from the appropiate reactive group on the Substrate and a hydroxy, thioether, amine, amido, hydrazido, carboxy, or carbamato group on the Cytotoxic Agent. Alternatively, the Substrate may be a precursor for a Cytotoxic Agent.

The term "Cytotoxic Agent" means compounds that are useful in the treatment of neoplasms, whether benign or malignant. Such drugs include, in general, alkylating agents, antiproliferative agents, tubulin-binding agents, cytotoxins in general, and the like. Preferred classes of such compounds are the nitrogen mustard agents, the vinca alkaloids, the daunomycin family, the mitomycins, the bleomycins, the cytotoxic nucleosides, the pteridine family of drugs, the podophyllotoxins, the sulfonylureas (as described in European Patent Publication No. 0 222 475, published May 20, 1987), low-molecular weight toxins such as the trichothecenes, and the colchicines. Particularly useful members of these classes may include, for example, doxorubicin, daunorubicin, aminopterin, methotrexate, methopterin, dichloromethotrexate, mitomycin C, porfiromycin, 5-fluorouracil, 6-mercaptopurine, cytosine arabinoside, podophyllotoxin, etoposide, melphalan, vinblastine, vincristine, leurosidine, vindesine, leurosine, trichothecene, desacetylcolchicine, N-(p-tosyl)-N′-(p-chloro-phenyl)urea, N-(((4-chloro-phenyl)amino)carbonyl)-2,3,-dihydro-1H-indene-5-sulfon-amide and the like. Those skilled in the art will understand that minor chemical modifications may be made to the preferred and generally described compounds in order to make reactions of them more convenient for use in the present invention.

It will be understood that preferred Substrate-Cytotoxic Agent compounds are prepared from the preferred Cytotoxic Agent. The preferred embodiments of the compound of Formula (II) are listed below. In the following list, each new preferred embodiment carries all of the definitions and limitations of the preceeding embodiments to which it refers. Also, it will be understood that reference to an Enzyme in the present specification also refers to an active fragment thereof. Thus, preferred embodiments of this aspect of the invention include:

A. A compound of Formula (II), wherein the Substrate is a substrate for a beta-lactamase enzyme; For example, the substrate for a beta-lactamase enzyme could be a penicillin, a penem, a carbapenem, cephalosporin, a cephalosporin sulfoxide, 1-carbadethiacephalosporin,or 1-oxa-dethiacephlosporin.

B. A compound or embodiment A, wherein the Substrate is

a cephalosporin of the formula;

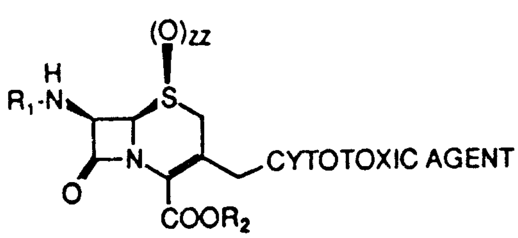

wherein ZZ is 0 or 1, $R_1$ is an acyl group derived from a $C_1$ to $C_{30}$ alkyl group, $R_2$ is hydrogen, an organic or inorganic cation, a carboxy-protecting group, or a non-toxic, metabolically-labile ester-forming group.

C. A compound of embodiment B, wherein the Substrate is a cephalosporin of the formula:

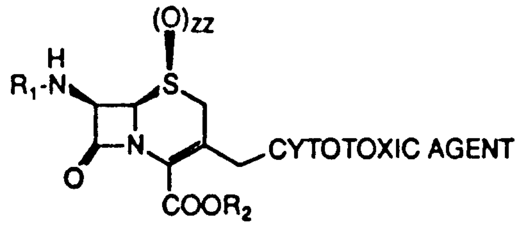

D. A compound of embodiment C, wherein $R_1$ is an acyl group of the formula $-COR_3$ wherein $R_3$ is:

i) $C_1$ to $C_6$ alkyl, $C_1$ to $C_6$ substituted alkyl, $C_1$ to $C_4$ alkoxy, $C_1$ to $C_4$ alkylthio, or a group of the formulae:

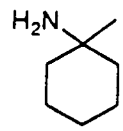 ;

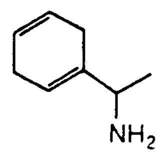 ;

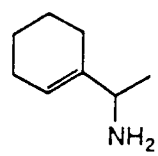 ;

$F_3C-S-CH_2-$ ;

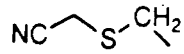 ;

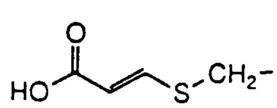 ; or

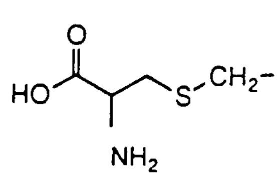 ;

or a protected amino and/or protected carboxy derivative thereof;

ii) a group of the formulae:

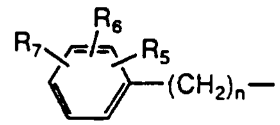

;

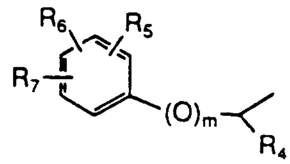

;

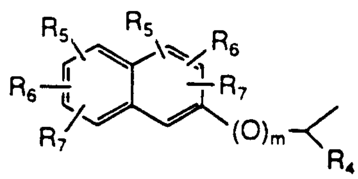

;

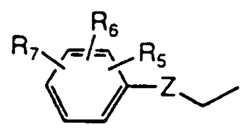

;

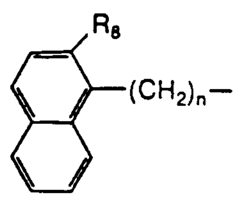

;

wherein each of $R_5$, $R_6$ and $R_7$ is, independently, hydrogen, halo, hydroxy, protected hydroxy, nitro, amino, protected amino, an amine salt, cyano, trifluoromethyl, aminomethyl, protected aminomethyl, N-(methyl- or ethyl-sulfonyl)amino, $C_1$ to $C_6$ alkyl or $C_1$ to $C_4$ alkoxy, $R_4$ is hydroxy, protected hydroxy, formyloxy, amino, protected amino, an amine salt, carboxy, a carboxylate salt, protected carboxy, phenyl carboxylate, (5-indanyl)-carboxylate, sulfonic acid, a sulfonate salt, azido, halo or $C_1$ to $C_6$ alkyl; $R_8$ is $C_1$ to $C_4$ alkoxy; Z is oxygen or sulfur; n is 0, 1, 2 or 3; and m is 0 or 1;

iii) a group of the formulae:

$R_9-(CH_2)_n-$ ;

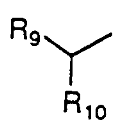

;

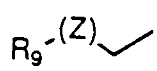

or

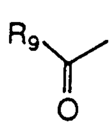

wherein $R_9$ is a heterocyclic ring; $R_{10}$ is hydroxy, protected hydroxy, formyloxy, amino, protected amino, an amine salt, carboxy, a carboxylate salt, protected carboxy, phenyl carboxylate, (5-indanyl)-carboxylate, sulfonic acid, a sulfonate salt, azido, halo or $C_1$ to $C_6$ alkyl; n is 0, 1, 2, or 3; and Z is oxygen or sulfur;

E. A compound of embodiment D, wherein the Cytotoxic Agent is one of the following:

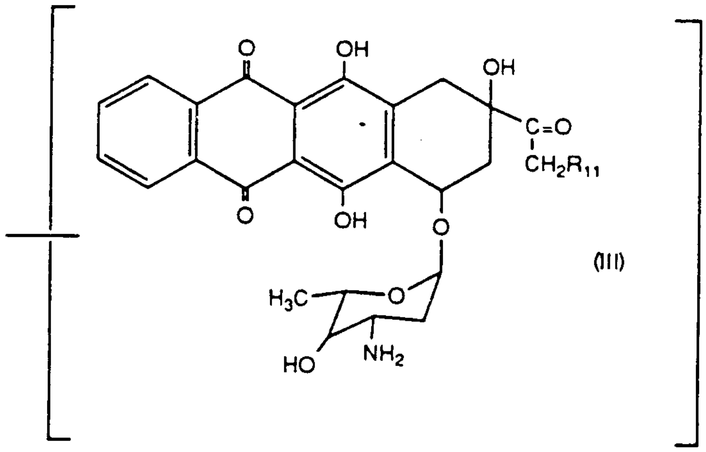

(III)

wherein $R_{11}$ is hydrogen or hydroxy;

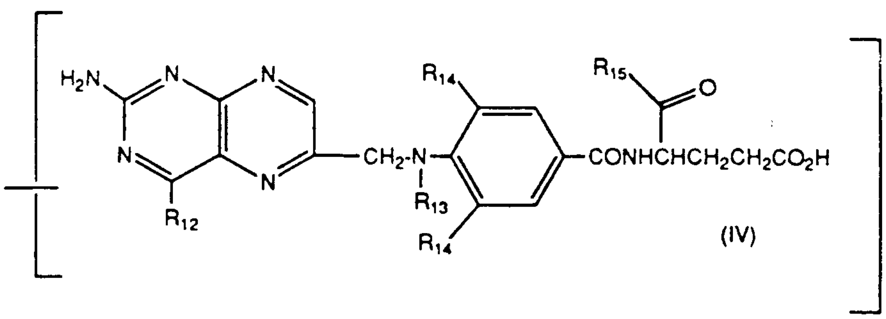

(IV)

wherein $R_{12}$ is amino or hydroxy;
$R_{13}$ is hydrogen or methyl;
$R_{14}$ is hydrogen, fluoro, chloro, bromo, iodo;
$R_{15}$ is hydroxy or a moiety which completes a salt of the carboxylic acid;

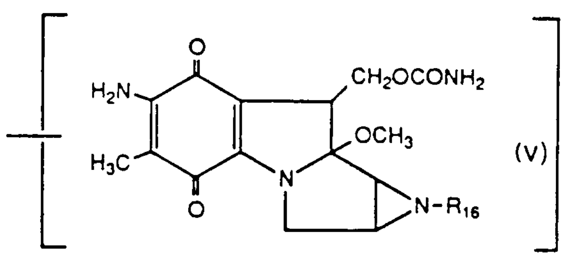

(V)

wherein $R_{16}$ is hydrogen or methyl;

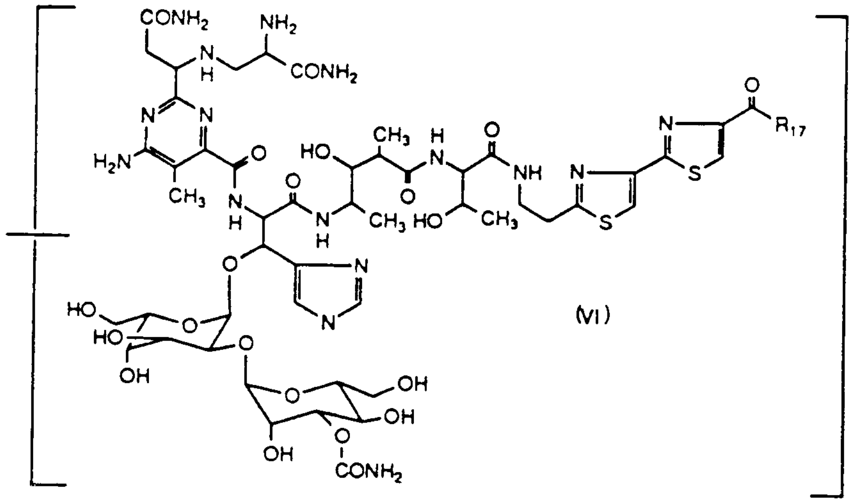

(VI)

wherein $R_{17}$ is amino, $C_1$-$C_3$ alkylamino, di-($C_1$-$C_3$ alkyl)amino, or $C_4$-$C_6$ polymethylene amino;

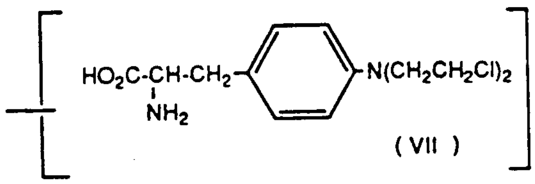

(VII)

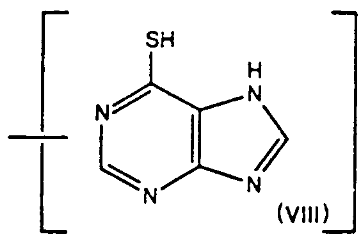

(VIII)

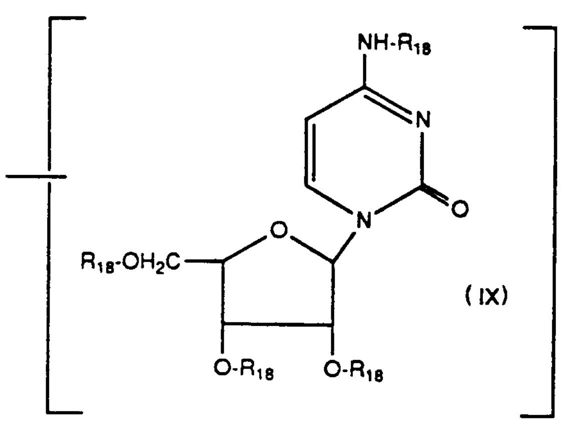

(IX)

wherein one of the $R_{18}$ moieties is a bond and the others are hydrogen;

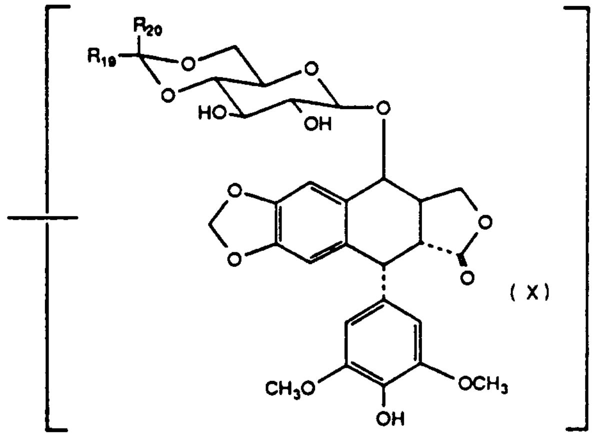

( X )

wherein $R_{19}$ is hydrogen or methyl;
$R_{20}$ is methyl or thienyl;

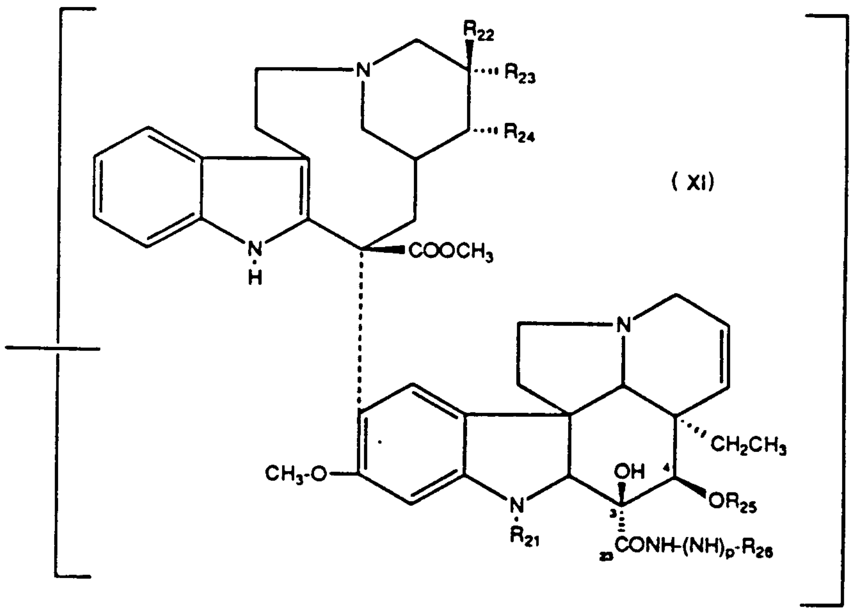

( XI )

wherein $R_{21}$ is H, $CH_3$ or CHO; when $R_{23}$ and $R_{24}$ are taken singly, $R_{24}$ is H, and one of $R_{22}$ and $R_{23}$ is ethyl and the other is H or OH; when $R_{23}$ and $R_{24}$ are taken together with the carbons to which they are attached, they form an oxirane ring in which case $R_{22}$ is ethyl; $R_{25}$ is hydrogen, ($C_1$-$C_3$ alkyl)-CO-, or chloro-substituted ($C_1$-$C_3$ alkyl)-CO;

p is 0 or 1;

$R_{26}$ is a bond, -($C_2$-$C_4$ alkyl)-X, or a group that requires that p is 1 and which is in turn bonded to a carbonyloxy group;

X is -O-, -S-, or -NH-;

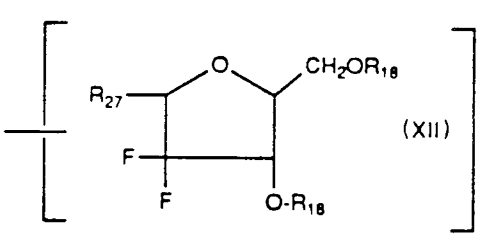

(XII)

wherein $R_{27}$ is a base of one of the formulae:

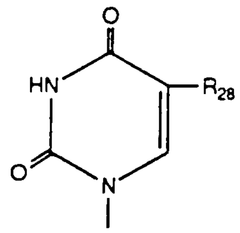

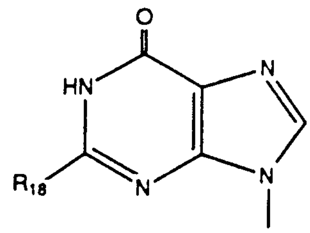

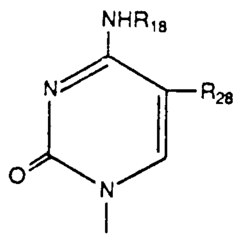

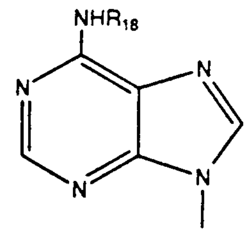

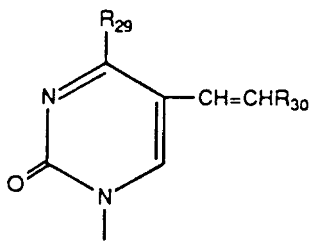

wherein $R_{28}$ is hydrogen, methyl, bromo, fluoro, chloro, or iodo;
$R_{29}$ is $-OR_{18}$ or $-NHR_{18}$;
$R_{30}$ is hydrogen, bromo, chloro, or iodo;

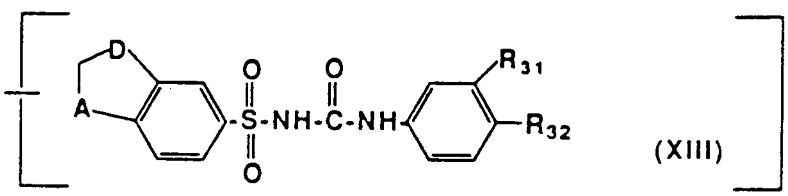

(XIII)

wherein A is -O-, $-NCH_3-$, $-CH_2-$, $-CH_2CH_2-$, or $-CH_2O-$;
D is $-CH_2-$ or -O-;
$R_{31}$ is hydrogen or halo;
$R_{32}$ is halo or trifluoromethyl;

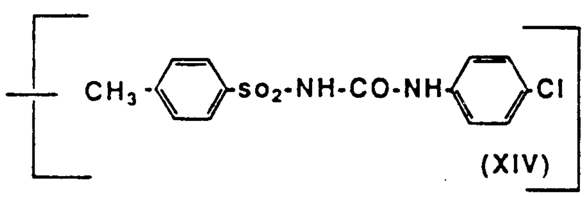

(XIV)

or, 5-uracil.

In the above preferred formulae, compounds of Formula (III) represent the daunomycin (or Adriamycin) group of compounds,; Formula (IV) represents the methotrexate group of compounds; Formula (V) represents the mitomycins; Formula (VI) represents the bleomycins; Formula (VII) represents melphalan; Formula (VIII) represents 6-mercaptopurine; Formula (IX) represents cytosine arabinoside; Formula (X) represents the podophyllotoxins; Formula (XI) represents the vinca drugs; Formula (XII) represents the difluoronucleosides and Formulae (XIII) and (XIV) represent the sulfonylurea compounds.

F. A compound of embodiment E, wherein $R_1$ of the substrate is acetyl, propionyl, isopropionyl, pivaloyl, butanoyl, methoxymethylacetyl, phenylacetyl, phenoxyacetyl, 2-(aminomethyl)phenylacetyl, 2-phenyl-2-hydroxy-acetyl, 2-phenyl-2-(sodium sulfonato)-acetyl, 2-phenyl-2-2-carboxyacetyl, 2-(4-hydroxyphenyl)-2-carboxyacetyl, 2-phenyl-2-aminoacetyl, 2-(4-hydroxyphenyl)-2-amino-acetal, 2-(3-(N-(methyl-sulfonylamino))phenyl)-2-aminoacetyl, 2-phenyl-2-(5-indanyl carboxylate)acetyl, 2-phenyl-2-(phenyl carboxylate)acetyl, 2-phenyl-2-azido-acetyl, 2-phenoxypropionyl, 2,5-dimethoxybenzoyl, 2-(formyloxy)-2-phenylacetyl, 2-(2-ethoxynaphth-1-yl)acetyl, 2-(naphth-1-yl)-2-aminoacetyl, 2-(naphth-2-yl)-2-aminoacetyl, 2-(2,5-dichlorophenylthio)acetyl, 2-(3,4-dichlorophenylthio)acetyl, 2-(1-tetrazolyl)acetyl, 2-(N-(3,5-dichloropyrid-4-oxyl))acetyl, 2-(2-aminothiazol-4-yl)acetyl, 2-(2-thienyl)acetyl, 2-(4-pyridylthio)acetyl, 2-(N-methyl-4-pyridiniumthio)acetyl, 2-(2-amino-4-phenylthiazol-5-yl)acetyl, 2-(3-hydroxy-4-carboxyisothiazol-5-ylthio)acetyl, 3-phenyl-5-methylisoxazolyl-3-formyl, 3-(2-chlorophenyl)-5-methylisoxazolyl-3-formyl, 3-(2,5-dichloroph.enyl)-5-methylisoxazolyl-3-formyl, 3-(2-fluoro-5-chlorophenyl)-5-methylisoxazolyl-3-formyl, 2-(2-thienyl)-2-aminoacetyl, 2-(2-thienyl)-2-(sodium carboxylate)acetyl, 2-(N-(4-pyridinium))acetyl, 2-(2-benzothienyl)acetyl, 2-(3-benzothienyl)acetyl, 2-(2-benzofuryl)acetyl, and 2-(3-benzofuryl)acetyl;

G. A compound of embodiment F, wherein the Cytotoxic Agent is methotrexate, 5-fluorouracil, desacetylvinblastine hydrazide, desacetylvinblastine aminoethanethiol, a desacetylvinblastine hydrazidocarboxy moiety, a 7-(carboxyamino)desacetyl colchicine moiety, N-(p-tosyl)-N′-(p-chlorophenyl) urea, or N-(((4-chlorophenyl)amino)-carbonyl)-2,3-dihydro-1H-indene-5-sulfonamide;

H. A compound of embodiment G, wherein $R_1$ is 2-(thien-2-yl)acetyl and ZZ is zero;

I. A compound of embodiment H, wherein the Cytotoxic Agent is methotrexate;

J. A compound of embodiment I, wherein methotrexate is bonded through its carboxylate group to the cephalosporin Substrate;

K. A compound of embodiment H, wherein the Cytotoxic drug is 5-fluorouracil;

L. A compound of embodiment K, wherein the 5-fluorouracil is bonded through its N-1 nitrogen to the cephalosporin Substrate;

M. A compound of embodiment H, wherein the Cytotoxic Agent is desacetylvinblastine hydrazide;

N. A compound of embodiment M, wherein the desacetylvinblastine hydrazide is bonded to the cephalosporin Substrate through the nitrogen atom of the hydrazide moiety that is not bonded to the vinblastine ring system;

O. A compound of embodiment H, wherein the Cytotoxic Agent is desacetylvinblastine aminoethanethiol;

P. A compound of embodiment O, wherein the desacetylvinblastine aminoethanethiol is bonded through the thiol group of its aminoethanethiol moiety to the cephalosporin moiety;

Q. A compound of embodiment H, wherein the Cytotoxic Agent is a desacetylvinblastine hydrazidocarboxy moiety;

R. A compound of embodiment Q , wherein the desacetylvinblastine moiety is bonded through an oxygen atom of the hydrazidocarboxy group to the cephalosporin substrate;

S. A compound of embodiment H, wherein the Cytotoxic Agent is N-(p-tosyl)-N′-(p-chlorophenyl) urea;

T. A compound of embodiment S, wherein N-(p-tosyl)-N′-(p-chlorophenyl)urea is bonded through the p-tosyl nitrogen to the cephalosporin Substrate;

U. A compound of embodiment G, wherein $R_1$ is 2-(thien-2-yl)acetyl and ZZ is 1;

V. A compound of embodiment U, wherein the Cytotoxic Agent is a 7-(carboxyamino)desacetylcolchicine moiety;

W. A compound of embodiment V, wherein the 7-(carboxyamino) desacetylcolchicine moiety is bonded through an oxygen atom of the 7-(carboxyamino) group to the cephalosporin Substrate;

X. A compound of embodiment U, wherein the Cytotoxic Agent is desacetylvinblastine aminoethanethiol;

Y. A compound of embodiment X, wherein the Cytotoxic Agent is desacetylvinblastine aminoethanethiol;

Z. A compound of embodiment U, wherein the Cytotoxic Agent is the desacetylvinblastine hydrazidocarboxy moiety;

AA. A compound of embodiment Z, wherein the desacetylvinblastine hydrazidocarboxy moiety is bonded through an oxygen atom of the hydrazidocarboxy group to the cephalosporin moiety;

BB. A compound of embodiment G, wherein $R_1$ is phenoxyacetyl and ZZ is 1;

CC. A compound of embodiment BB, wherein the Cytotoxic Agent is the desacetylvinblastine hydrazidocarboxy moiety;

DD. A compound of embodiment CC, wherein the desacetylvinblastine hydrazidocarboxy moiety is bonded through an oxygen atom of the aminocarboxy group to the cephalosporin Substrate.

EE. A compound of embodiments I through DD, wherein $R_2$ is hydrogen, a sodium cation, or a potassium cation.

It will be understood that the compounds of Formula (II) may also exist as solvates and hydrates. Thus, these compounds may crystallize with, for example, waters of hydration, or one, or a number of, or any fraction thereof of molecules of the mother liquor solvent. The solvates and hydrates are included within the scope of this invention.

Similarly, the compounds of Formula (II) can exist as pharmaceutically-acceptable salts. The compounds include salts of both the acidic functions, such as carboxy and sulfonate groups, and the basic functional groups, such as amino groups. Such salts include the organic and inorganic cations discussed below, plus the salts formed from acid-base reactions of basic groups with acids such as hydrochloric, sulfuric, phosphoric, acetic, succinic, citric, lactic, maleic, fumaric, palmitic, cholic, pamoic, mucic, D-glutamic, d-camphoric, glutaric, phthalic, tartaric, lauric, stearic, salicylic, methanesulfonic, benzenesulfonic, sorbic, picric, benzoic, cinnamic, and like acids.

As used in the above preferred embodiments, the term "acyl group derived from a $C_1$ to $C_{30}$ carboxylic acid" respresented by $R_1$ refers to the acyl moieties which have been bonded to the C-6 amino group of penicillins, the C-7 amino group of cephalosporins, 1-sulfoxide cephalosporin, 1-oxadethiacephalosporins or 1-carbacephalosporins and the C-3 amino of monocyclic $\beta$-lactams (such as the azthreonam series). The "acyl group derived from a $C_1$ to $C_{30}$ carboxylic acid" can be optionally interrupted by heteroatoms. Examples of such acyl groups can be found in references such as "Cephalosporins and Penicillins, Chemistry and Biology" edited by Edwin W. Flynn, Academic Press, New York, 1972 and "Chemistry and Biology of $\beta$-lactam Antibiotics" edited by Robert B. Morin and Marvin Gorman, Vols. 1,2, and 3, Academic Press, New York, 1982.

Examples of acyl groups at $R_1$ can also be found in M. Yoshioka et al., U.S. Patent No. 4,478,997, issued October 23, 1984, B.R. Belleau et al., U.S. Patent No. 4,172,199, issued October 23, 1979, T. Kamiya et al., U.S. Patent No. 4,472,300, issued September 18, 1984, (especially columns 25 through 36). Additional examples of "acyl groups derived from a $C_1$ to $C_{30}$ carboxylic acid" can be found in Koster et al., U.S. Patent No. 4,478,749, issued October 23, 1984.

The term "organic or inorganic cation" refers to counter-ions for the carboxylate anion of a carboxylate salt. The counter-ions are chosen from the alkali and alkaline earth metals, such as lithium, sodium, potassium, barium and calcium; ammonium; and organic cations such as dibenzylammonium, benzylammonium, 2-hydroxyethylammonium, bis(2-hydroxyethyl)ammonium, phenylethylbenzylammonium, dibenzylethylenediammonium and like cations. Other cations encompassed by the above term include the protonated form of procaine, quinine and N-methylglucosamine, and the protonated forms of basic amino acids such as glycine, ornithine, histidine, phenylglycine, lysine and arginine. Furthermore, any zwitterionic form of the instant compounds formed by a carboxylic acid and an amino group is referred to by this term.

The term "carboxy-protecting group" as used in the specification refers to one of the ester derivatives of the carboxylic acid group commonly employed to block or protect the carboxylic acid group while reactions are carried out on other functional groups on the compound. Examples of such carboxylic acid protecting groups include 2-nitrobenzyl, 4-nitrobenzyl, 4-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylenedioxybenzyl, benzhydryl, 4,4′-dimethoxybenzhydryl, 2,2′,4,4′-tetramethoxybenzhydryl, t-butyl, t-amyl, trityl, 4-methoxytrityl, 4,4′-dimethoxytrityl, 4,4′,4″-trimethoxytrityl, 2-phenylprop-2-yl, trimethylsilyl, t-butyldimethylsilyl, phenacyl, 2,2,2-trichloroethyl, $\beta$-(trimethylsilyl)ethyl, $\beta$-(di(n-butyl)methylsilyl)ethyl, p-toluenesulfonylethyl, 4-nitrobenzylsulfonylethyl, allyl, cinnamyl, 1-(trimethylsilylmethyl)prop-1-en-3-yl, and like moieties. The species of carboxyl-protecting group employed is not critical so long as the derivatized carboxylic acid is stable to the condition of subsequent reaction(s) on other positions of the cephalosporin molecule and can be removed at the appropriate point without disrupting the remainder of the molecule. A preferred carboxylic acid protecting group is the allyl group. Similar carboxy-protecting groups used in the cephalosporin, penicillin and peptide arts can also be used to protect carboxy group substituents of the instant cephalosporin substrate. Further examples of these groups are found in E. Haslam, "Protective Groups in Organic Chemistry", J.G.W. McOmie, Ed., Plenum Press, New York, N.Y., 1973, Chapter 5, and T.W. Greene, "Protective Groups in Organic Synthesis", John Wiley and Sons, New York, N.Y., 1981, Chapter 5. A related term is "protected carboxy", which refers to a carboxy group substituted with one of the above carboxy-protecting groups.

The term "non-toxic, metabolically-labile, ester-forming group" refers to those biologically active ester forms which induce increased blood levels and prolong the efficacy of the corresponding non-esterified forms of the compounds. Such ester groups include the lower alkoxymethyl groups, for example, methoxymethyl, ethoxymethyl, iso-propoxymethyl and the like; the α-($C_1$ to $C_4$) alkoxyethyl groups, for example, methoxyethyl, ethoxyethyl, propoxyethyl, iso-propoxyethyl, and the like; the 2-oxo-1,3-dioxolen-4-ylmethyl groups, such as 5-methyl-2-oxo-1,3-dioxolen-4-ylmethyl, 5-phenyl-2-oxo-1,3-dioxolen-4-ylmethyl, and the like; the $C_1$ to $C_3$ alkylthiomethyl groups, for example methylthiomethyl, ethylthiomethyl, iso-propylthiomethyl, and the like; the acyloxymethyl groups, for example, pivaloyloxymethyl, α-acetoxymethyl, and the like; the ethoxycarbonyl-1-methyl group; the α-acyloxyα-substituted methyl groups, for example α-acetoxyethyl; the 3-phthalidyl or 5,6-dimethylphthalidyl groups; the 1-($C_1$ to $C_4$ alkyloxycarbonyloxy)eth-1-yl groups such as the 1-(ethoxycarbonyloxy)eth-1-yl group; and the 1-($C_1$ to $C_4$ alkylaminocarbonyloxy)eth-1-yl groups such as the 1-(methylaminocarbonyloxy)eth-1-yl group.

Several terms used in conjunction with embodiment D above should be defined. Thus, the term "$C_1$ to $C_6$ alkyl" denotes such radicals as methyl, ethyl, n-propyl, iso-propyl, n-butyl, sec-butyl, tert-butyl, amyl, tert-amyl, hexyl and the like.

The term "$C_1$ to $C_6$ substituted alkyl" denotes the above $C_1$ to $C_6$ alkyl groups that are substituted by one or two halogen, hydroxy, protected hydroxy, amino, protected amino, $C_1$ to $C_7$ acyloxy, nitro, carboxy, protected carboxy, carbamoyl, carbamoyloxy, cyano, methylsulfonylamino or $C_1$ to $C_4$ alkoxy groups. The substituted alkyl groups may be substituted once or twice with the same or with different substituents.

The term "$C_1$ to $C_4$ alkoxy" as used herein denotes groups such as methoxy, ethoxy, n-propoxy, isopropoxy, n-butoxy, t-butoxy and like groups.

The term "protected amino" as employed in the above definition has reference to an amino group substituted with one of the commonly employed amino blocking groups such as the tert-butoxycarbonyl group (t-BOC), the benzyloxycarbonyl group, the 4-methoxybenzyloxycarbonyl group, the 2,2,2-trichloroethoxycarbonyl group, the trimethylsilyl group, and like amino protecting groups. The nature of such amino protecting groups is not critical so long as the protected amino functionality is stable under the reaction conditions described hereinafter.

The term "protected hydroxy" has reference to any group stable under the reaction conditions of the subsequent step in the synthesis of the cephalosporin substrate compounds, but readily cleavable thereafter. Such groups include the formyloxy group, the chloroacetoxy group, the benzhydryloxy group, the trityloxy group, the trimethylsilyl group, and the like.

In the foregoing definitions, hydroxy, amino, and carboxy protecting groups are not exhaustively defined. The function of such groups is to protect the reactive functional groups during the preparation of the desired products and then to be removed without disrupting the remainder of the molecule. Many such protecting groups are well known in the art and the use of other groups equally applicable to the process and compounds of the present invention, such as those described in J.F.W. McOmie, "Protective Groups in Organic Chemistry", Plenum Press, 1973, and T.W. Greene, "Protective Groups in Organic Chemistry", John Wiley and Sons, New York, N.Y., 1981, will be recognized as suitable. Thus, there is no novelty or inventiveness asserted with regard to the "protecting groups" alluded to in this specification.

The term "heterocyclic ring", when used in conjunction with the term "acyl group derived from a $C_1$ to $C_{30}$ carboxylic acid", refers to the rings in W. Durckheimer et al., U.S. Patent No. 4,278,793. Particular examples are unsubstituted or substituted rings such as tetrazolyl, oxazolyl, isoxazolyl, thienyl, furyl, thiazolyl, thiadiazolyl, isothiazolyl, pyridyl, 4-pyridonyl, pyrididyl, benzthienyl, benzfuryl or indolyl. Of course, these examples can be substituted with the substituents discussed in the Durckheimer et al., supra.

This invention provides a means for a method of treatment of neoplastic diseases which comprises:

1) administering to the affected host a therapeutically effective amount of the Antibody-Enzyme conjugate compound of Formula (I); i.e. an Antibody-Enzyme conjugate compound wherein the Enzyme is a beta-lactamase; then

2) administering to the affected host a therapeutically effective amount of the Substrate-Cytotoxic Agent of Formula (II) above; i.e. a Substrate-Cytotoxic agent wherein the Substrate is a substrate for beta-lactamase.

It will be understood that the term "therapeutically-effective", as it relates to the Substrate-Cytotoxic Agent, will be an amount sufficient to cause some target neoplastic cell death, and may further refer to the use of this drug to keep a neoplastic disease in remission, or, in general, in an amount sufficient for prophylaxis.

In general, the term "therapeutically effective" means at least the same molar equivalent amount of the Substrate-Cytotoxic Agent as would be given for the Cytotoxic Agent alone for the particular purpose at hand. Larger amounts of the Substrate-Cytotoxic Agent may be given in the instant invention because the

toxicity of the Cytotoxic Agent is initially masked by the attached Substrate but subsequently unmasked upon reaction with the Antibody-Enzyme conjugate at its point of delivery, thus relieving the patient of the side effects normally associated with that amount of Cytotoxic Agent alone.

A therapeutically effective dose of the Antibody-Enzyme conjugate is the amount that delivers between 1 through 500, and preferrably, 10 through 50, milligrams of Antibody (or fragments thereof) to the affected host.

Preferred embodiments for the aforementioned method are listed below. As with the compound aspects of the invention, the embodiments listed below contain the limitations and definitions of the embodiment it refers to. Examples of the preferred embodiments of the present method include:

C. A method wherein the Antibody complexes with antigens that are present solely, or are in supernatural abundance, on malignant tumor cells;

D. A method of embodiment C, wherein the Substrate is a cephalosporin of the formula:

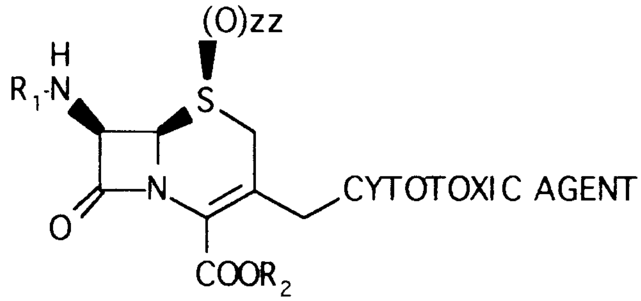

wherein ZZ is 0 or 1; $R_1$ is an acyl group derived from a $C_1$ to $C_{30}$ alkyl group, $R_2$ is hydrogen, an organic or inorganic cation, a carboxy-protecting group, or a non-toxic, metabolically-labile ester-forming group; as those terms are defined regarding preferred embodiment B of the Substrate-Cytotoxic Agent aspect of this invention;

E. A method of embodiment D, wherein the Antibody complexes with carcinoembryonic antigen;

F. A method of embodiment D, wherein the Antibody complexes with the KS1/4 antigen;

G. A method of embodiment E, wherein $R_1$ is the same as $R_1$ in the preferred embodiment D of the compounds of Formula (II);

H. A method of embodiment G, wherein the Cytotoxic Agent is a compound of the formula listed in the preferred embodiment E of the compounds of Formula (II);

I. A method of embodiment H, wherein the $R_1$ is the same as for preferred embodiment F of the compounds of Formula (II);

K. A method of embodiment I, wherein the Cytotoxic Agent is methotrexate, 5-fluorouracil, desacetylvinblastine hydrazide, desacetylvinblastine aminoethanethiol, a desacetylvinblastine aminoacarboxy moiety, a 7-(carboxyamino) desacetylcolchicine moiety; N-(p-tosyl)-N′-(p-chlorophenyl)urea; or N-(((4-chlorophenyl)-amino)carbonyl)-2,3-dihydro-1H-indene-5-sulfonamide;

L. A method of embodiment K, wherein $R_2$ of the cephalosporin Substrate is 2-(thien-2-yl)acetyl and ZZ is 0;

M. A method of embodiment L, wherein the Antibody is designated CEM 231.6.7;

N. A method of embodiment M, wherein the Cytotoxic Agent is methotrexate;

O. A method of embodiment N, wherein the methotrexate is bonded through a carboxy group to the cephalosporin Substrate;

P. A method of embodiment M, wherein the Cytotoxic Agent is 5-fluorouracil;

Q. A method of embodiment P, wherein 5-fluorouracil is bonded through its N-1 nitrogen to the cephalosporin substrate;

R. A method of embodiment M, wherein the Cytotoxic Agent is desacetylvinblastine hydrazide;

S. A method of embodiment R, wherein the desacetylvinblastine hydrazide is bonded through the nitrogen atom of the otherwise unbonded amine portion of the hydrazide moiety;

T. A method of embodiment M, wherein the Cytotoxic Agent is desacetylvinblastine aminoethanethiol;

U. A method of embodiment T, wherein the desacetylvinblastine aminoethanethiol is bonded through the thiol group of the aminoethanethiol moiety to the cephalosporin moiety; and

V. A method of treatment of embodiment M, wherein the Cytotoxic agent is N-(p-tosyl)-N′-(p-chlorophenyl)urea.

W. A method of treatment of embodiment V, wherein the N-(p-tosyl)-N′-(p-chlorophenyl)urea is bonded through the (p-tosyl) nitrogen.

X. A method of treatment of embodiment M, wherein the Cytotoxic Agent is a desacetylvinblastine aminocarboxy moiety.

Y. A method of treatment of embodiment X, wherein the desacetylvinblastine hydrazidocarboxy moiety is bonded through an oxygen atom of the hydrazidocarboxy group to the cephalosporin Substrate.

Z. A method of treatment of embodiment K, wherein $R_1$ is 2-(thien-2-yl) acetyl and ZZ is 1.

AA. A method of treatment of embodiment Z, wherein the Cytotoxic Agent is a 7-(carboxyamino)-desacetylcolchicine moiety.

BB. A method of treatment of embodiment AA, wherein the Cytotoxic Agent is 7-(carboxyamino)-desacetylcolchicine moiety.

CC. A method of treatment of embodiment Z, wherein the Cytotoxic Agent is desacetylvinblastine aminoethanethiol.

DD. A method of treatment of embodiment CC, wherein the desacetylvinblastine aminoethanethiol is bonded through the thiol group to the cephalosporin Substrate.

EE. A method of treatment of embodiment Z, wherein the Cytotoxic Agent is a desacetylvinblastine hydrazidocarboxy moiety.

FF. A method of treatment of embodiment EE, wherein the desacetylvinblastine hydrazidocarboxy moiety is bonded through an oxygen atom of the hydrazidocarboxy group to the cephalosporin Substrate.

GG. A method of treatment of embodiment K, wherein $R_1$ is (phenoxy)acetyl and ZZ is 1.

HH. A method of treatment of embodiment GG, wherein the Cytotoxic Agent is a desacetylvinblastine hydrazidocarboxy moiety.

II. A method of treatment of embodiment HH, wherein the desacetylvinblastine hydrazidocarboxy moiety is bonded through an oxygen atom of the hydrazidocarboxy group to the cephalosporin Substrate.

JJ. A method of embodiments M through II; wherein the Antibody is a F(ab′) fragment.

The Substrate-Cytotoxic Agent compounds of the instant invention can be synthesized by a variety of methods that are known. Thus, a nucleophilic group of the Substrate could be used to displace a leaving group at an electrophilic center of the Cytotoxic Agent under $S_n1$ or $S_n2$ conditions, or vice versa. Carboxy, amino, thiol or hydroxy groups on the Substrate and the Cytotoxic Agent that would interfere with the reaction of desired functional groups could be masked with protecting groups before the reaction, then deprotected after the reaction. The use of suitable protecting groups is described, among other places, in the works of McOmie and Greene as set forth above. All that is required of the instant Cytotoxic Agent compounds is that the Cytotoxic Agent be a potent antineoplastic agent after separation from the Substrate as is effected by the enzyme, and that the derivatization of the Substrate with Cytotoxic Agent does not significantly decrease the reactivity of the Substrate with Enzyme. It is preferred that the SubstrateCytotoxic Agent compound mask the toxicity of the Cytotoxic Agents or that the cytotoxicity be no more than 70% of the cytotoxicity of the unsubstituted Cytotoxic Agent.

For the purposes of the present method, it is desirable that Enzyme have a reasonable catalytic rate. In light of the meager concentrations of Substrate (in the form of the Substrate-Cytotoxic Agent compounds) that the enzyme will encounter, it is especially important that the enzyme-substrate combination have a high $k_{cat}/K_m$ ratio. Thus, it is desirable to choose an enzyme that has a $k_{cat}/K_m$ ratio of at least $1 \times 10^6\ M^{-1}\ s^{-1}$. This same ratio is desirable in the Enzyme once it is conjugated to the Antibody. Similarly, it is preferred for the Antibody to have high affinity for the antigen on the target neoplastic cell, preferably about $1 \times 10^6$ - (liter/mole) or above. It is also desirable that the conjugated Antibody retain about 80% of its unconjugated activity, thus desirably exhibiting a 60% immunoreactivity once conjugated.

Conjugating an Enzyme to an Antibody is accomplished by methods standard in the protein chemistry art. Heterobifunctional and homobifunctional reagents can be used. Thus, the present conjugates can be formed using MBS, Sulfo-MBS, SMCC, Sulfo-SMCC, SIAB, SPDP, Sulfo-SMPB, DIDS, DFDNB, BMH, (which are commercially available) and the like. Purification of the resulting conjugate may be accomplished by appropriate, well-known chromatographic methods such as gel permeation or ion exchange. Thus, nothing is novel in the instant invention in the way the Enzyme and the Antibody are conjugated. It is preferred that the stoichiometry and other conditions of the conjugation reaction (and purification) be adjusted such that a 1:1 Antibody : Enzyme conjugate results, although conjugates with disproportionately larger amounts of either moiety in the conjugate are encompassed in the present invention.

After choosing the Antibody and Enzyme, one skilled in the art must decide whether to use the whole Antibody or a fragment thereof. The methods for making fragments, using pepsin, papain, or lysyl endopeptidase from Achromobacter lyticus (Wako Chemicals USA, Inc., 1600 Bellwood Road, Richmond, VA), for instance, are elaborated in M. Brennan et al., Science, 229, pp. 81-83 (1985), and M. Glennie et al.,

J. Immunology, 139, pp. 2367-2375 (1987), and will not be discussed here, other than to mention that F(ab′) is the preferred form for the Antibody of this invention.

After isolating an Enzyme with the criteria detailed above, conjugating it to the appropriate Antibody as described above, and purifying the conjugate, it is useful to analyze the conjugate by gel electrophoresis. Alternatively, high pressure liquid chromatography (HPLC) may be used to analyze the conjugate.

The activity of the Enzyme of the conjugate is again measured, this time employing Substrate bound to a molecule that imparts to the Substrate (or the molecule itself) a measurable spectrophotometric change upon cleavage to allow one to measure the $k_{cat}$ and the $K_M$. This measurement will demonstrate whether or not the enzyme has retained its activity through the conjugation step. The next parameter to be measured is the immunoreactivity of the conjugate in vitro. This measurement is usually made by first radioactively labelling the conjugate (with, for example, $^{125}I$) coating a solid surface (e.g., small plastic beads or microtiter plate wells) with the target antigen. This measurement will detect if the Antibody portion of the conjugate has been unacceptably altered in the conjugation step. The first step toward determining whether the components of the instant method function in the presence of the target antigen comes when the $k_{cat}$ and the $K_m$ of the conjugate are measured with the Substrate-Cytotoxic Agent. Release by the enzyme of free Cytotoxic Agent can be shown chromatographically. The conjugate is then studied to assess if it will localize to tumors in vivo. This is typically accomplished by injecting radiolabelled conjugate into tumor-bearing mice, which tumors express the target antigen, then sacrificing the mice and detecting the amount of radioactivity present in the tumor(s) and the various organs. This study will demonstrate that the conjugate localizes to the tumor, or is unacceptably targeting an undiseased organ, or furthermore is metabolized becasuse it is a foreign protein.

Once determined that the conjugate localized to the tumor, it is necessary to demonstrate that the Substrate-Cytotoxic Agent will be cleaved by the localized conjugate. Thus, target-antigen expressing cells are suspended and incubated either with conjugate or, for a control, enzyme only. The cells so treated are resuspended in another amount of buffer, then incubated with a Substrate that is coupled with a compound such that the pair absorb at different wavelengths in the visible spectrum before and after cleavage. The supernatant of these suspensions then is measured to determine if the conjugate-containing suspensions demonstrate a time and concentration dependence on the amount of conjugate added. Thus, if the control samples are exhibiting such a dependence, some condition other than the conjugate is causing the cleavage. Assuming that the components of the present invention demonstrate in the preceding test that localized conjugate is indeed the agent causing the Substrate-Cytotoxic Agent cleavage, the serum kinetics of the conjugate in a test animal such as mice should be measured to determine if and when the Substrate-Cytotoxic Agent can be administered to the affected host after administration of the conjugate. It is desirable that the conjugate demonstrate a short serum half-life, so that the Substrate Cytotoxic Agent molecule can be administered within a reasonable time (e.g., 48 hours) after administration of the conjugate. Once the serum half-life of the conjugate has been found to be acceptable, the Enzyme activity of the conjugate localized in vivo in test animals, such as the mice, is determined. The conjugate is administered to the target-tumor-bearing mice, and after sufficient time has passed for the conjugate to localize, the mice are sacrificed and their tumors are excised. The tumor is minced and incubated with a chromogenic substrate. The supernatants of these suspensions are measured for signs of enzymatic activity.

If the components of the present method have successfully overcome these hurdles, the in vitro cytotoxicity of the components is measured.

Target cells are then suspended and incubated with conjugate. The treated cells are then resuspended and incubated with Substrate-Cytotoxic Agent. Cell growth is monitored by the uptake of radiolabelled nutrients subsequently added to the culture media. The final step in evaluating the present method is to evaluate the efficacy of the components in a nude mouse tumor model study.

A further aspect of this invention is the pharmaceutical formulations of the Antibody-Enzyme conjugate of Formula (I) and the Substrate-Cytotoxoic Agent compounds of Formula (II). In particular, these pharmaceutical formulations are useful for the treatment of neoplastic diseases of the present invention and comprise a pharmaceutically acceptable carrier, diluent or excipient and a therapeutically effective amount of either a conjugate of Formula (I) or a compound of Formula (II). As used, "pharmaceutically acceptable" refers to those agents, well known to those skilled in the art, which are useful in the preparation of formulations useful for the treatment of a warm-blooded animal.

With regard to compositions for oral administration (e.g., tablets and capsules), the term "pharmaceutically acceptable carrier, diluent or excipient" means common excipients such as binding agents, for example, syrup, acacia, gelatin, sorbitol, tragacanth, polwinylpyrrolidine (Povidone), methylcellulose, ethylcellulose, sodium carboxymethylcellulose, hydroxypropylmethylcellulose, surcrose and starch; fillers and carriers, for example corn starch, gelatin, lactose, sucrose, microcrystalline cellulose, kaolin, mannitol,

dicalcium, phosphate, sodium chloride and alginic acid; disintegrators such as croscarmellose sodium, microcrystalline cellulose, corn starch, sodium starch glycolate, alginic acid and mutable wetting agents such as sodium lauryl sulfate; and lubricants such as magnesium stearate and other metallic stearates, stearic acid, silicone fluid, talc, waxes oils and colloidal silica. Flavoring agents such as peppermint, oil of wintergreen, cherry flavoring or the like can also be used. It may be desirable to add a coloring agent to make the dosage form more aesthetically pleasing in appearance or to help identify the product. The tablets may also be coated by methods well known in the art.

The pharmaceutical compositions of the present invention may also be in the form of oral liquid preparations, which may be either a) aqueous or oily suspensions, solutions, emulsions or syrups; or b) a dry powder to be reconstituted with water or another suitable vehicle before use. When used in conjunction with such oral liquid preparations, the term "pharmaceutical acceptable carrier, diluent or excipient" means conventional additives such as suspending agents, for example, sorbitol, syrup, methyl cellulose, glucose/sugar syrup, gelatin, hydroxyethylcellulose, carboxymethyl cellulose, aluminum stearate gel or hydrogenated edible oils, for example almond oil, fractionated coconut oil, oily esters, propylene glycol or ethyl alcohol; and preservatives such as methyl or propyl p-hydroxybenzoates or sorbic acid.

The pharmaceutical composition can also be for intravenous ("IV") use. Specifically, a water soluble form of the conjugate or Substrate-Cytotoxic Agent can be dissolved in one of the commonly used intravenous fluids and administrated by infusion. When used in conjunction with compositions for IV use, the term "pharmaceutically acceptable carrier diluent, carrier or excipient" means fluids such as physiological saline, Ringer's solution or 5% dextrose solution.

For intramuscular preparations a sterile formulation of a suitable salt form of the conjugate or Substrate-Cytotoxic Agent compound (for example, the hydrochloride salt or sodium salt) can be formulated with a "pharmaceutically acceptable carrier, diluent or excipient". Examples of such sterile formulations are a suitable salt form either dissolved in a pharmaceutical diluent (for example, Water-for-Injection, physiological saline, 5% glucose) or suspended in an aqueous base or a pharmaceutically acceptable oil base (for example, an ester of a long chain fatty acid such as ethyl oleate).

Topical compositions can be formulated with suitable vehicles such as hydrophobic or hydrophilic bases. Such bases include ointments, creams or lotions.

Veterinary pharmaceutical compositions of the conjugates and the Substrate-Cytotoxic Agent compounds may be administered in the feed or the drinking water of farm animals. Alternatively, the conjugate or Substrate-Cytotoxic Agent compounds can be formulated as intramammary preparations with suitable vehicles such as long-or quick-release bases.

The Antibody-Enzyme conjugate of Formula (I) and the Substrate-Cytotoxic Agent compounds of Formula (II) can also be formulated in unit dosage form in sterile vials, sterile plastic pouches containing a port with a septum, or sterile, hermetically sealed ampoules. The conjugate or compound (or the corresponding pharmaceutically-acceptable salt) may be a dry powder or in crystalline or lyophilized form. The amount of the conjugate or Substrate-Cytotototoxic Agent compound per unit dosage may vary from about 5 milligrams to about 10 grams.

In a further embodiment of the invention, there is provided a kit adapted for the treatment of a neoplastic disease which comprises an Antibody-Enzyme Conjugate of Formula (I) and a Substrate-Cytotoxic Agent compound of Formula (II). These compounds can be formulated as outlined above and may be administered in accordance with the method described earlier.

The following non-limiting Experiments and Procedures are provided to further illustrate the present invention. Abbreviations used below have the meanings commonly associated with them in the relevant art unless noted otherwise. The N.M.R. spectra were taken on General Electric G.E.-300 instrument. The infrared spectra were obtained on a Perkin-Elmer 281 instrument. Ultraviolet spectra were obtained on a Cary 118 instrument (except for Example 9 and Procedures 6 through 13, where the U.V. spectra were obtained on a Hewlett-Packard 8451A instrument.) Fast atom bombardment mass spectra were obtained on a VG ZAB-3 instrument.

Procedure 1

Allyl 7-β-(2-(Thien-2-yl)acetamido)-3-Acetoxymethylene-3-Cephem-4-Carboxylate

Sodium 7-β-(2-(thien-2-yl)acetamido)-3-(acetoxymethylene)-3-cephem-4-carboxylate (11.0 g) was dissolved in a 1:1 mixture of DMF and water. Allyl bromide (3.6g) was added and the reaction mixture was stirred for 48 h at room temperature under a nitrogen atmosphere. The reaction mixture was diluted with ethyl acetate and then 0.1N hydrochloric acid. The organic layer was separated and dried over magnesium

sulfate, filtered, and concentrated in vacuo to give an orange oil. The oil was flash chromatographed on a 3 inch column with a 100-200 mesh activated silica support eluted with ethyl acetate. White crystals formed from some of the product-containing fractions on standing. These crystals were collected by filtration then recrystallized from 1:1 chloroform:ether and dried to give 5.5 g of the title product. N.M.R. (300 MHz, $CDCl_3$) δ 7.25 (d,1,J = 7); 7.0 (m,2); 6.27 (br d, 1, J = 9), 6.0-5.8 (m,1); 5.83 (dd, 1, J = 5,9); 5.4-5.2 (m,2); 5.06 (1/2 of ABq, 1, J = 14); 4.96 (d, 1, J = 5); 4.81 (1/2 of ABq, 1, J = 14); 4.74 (br d, 2, J = 6); 3.85 (s, 2); 3.55 (1/2 of ABq, 1 J = 18); 3.33 (1/2 of ABq, 1, J = 18); 2.06 (s, 3).

Procedure 2

Allyl 7-β-(2-(Thien-2-yl)acetamido)-3-(Iodomethyl)-3-Cephem-4-Carboxylate

Allyl 7-β-(2-thien-2-yl)acetamido)-3-acetoxymethyl-3-cephem-4-carboxylate (5.01g, 11.5 mmol) was dissolved in methylene chloride (55 ml). TMSI (3.27 ml) was added and the resultant solution was stirred at room temperature for 70 min. The solution was diluted with ethyl acetate, then washed sequentially with ice cold 10% sodium thiosulfate, saturated aqueous sodium bicarbonate solution, and brine. The solution was dried over sodium sulfate and concentrated in vacuo to give 4.6g, 79% yield of a yellow-orange tinted foam of the title product: N.M.R. (300 MHz, $CDCl_3$): δ 7.25 (d, 1,J = 7); 7.0 (m,2); 6.42 (d,1,J = 9); 6.0-5.85 (m,1) ; 5.78 (dd, 1, J = 5,9); 5.4-5.2 (m,2); 4.95 (d,1,J = 5); 4.74 (d,2,J = 6); 4.38 (ABq, 2, J = 10); 3.85 (s, 2); 3.72 (1/2 of ABq, 1, J = 18); 3.44 (1/2 of ABq, 1, J = 18).

Example 1

Allyl 7-β-(2-(Thien-2-yl)acetamido)-3-((Methotrexate-gamma-Ester)methylene)-3-Cephem-4-Ester

Allyl 7-β-(2-(Thien-2-yl)acetamido)-3-(iodomethyl)-3-cephem-4-carboxylate (4.6g, 9.1 mmol), methotrexate-gamma-ester (4.6g, 9.1 mmol, Sigma), DMF (50 ml), and sodium bicarbonate (1.54g, 18.25 mmol) were combined. The solution was stirred at room temperature for 18 h then added dropwise to a solution of acetic acid (4 ml) in water (400 ml). The resultant precipatate was collected and dried in vacuo to yield approximately 7g of powder. The powder was flash chromatographed over a silica gel column eluted with a solution of 15% methanol : 2% acetic acid in chloroform, to give approximately 2.1g of yellow-orange solid. The solid was flash chromatographed on silica gel eluted with a gradient of 10% methanol : 1% acetic acid in chloroform to 2% acetic acid in chloroform then by a 15% methanol and 2% acetic acid in chloroform to give 1.06g ($R_f$ = 0.5 in 2% HOAc, 15% MeOH in $CHCl_3$) of the title product: IR (KBr): 1782, 1730, 1607, 1562, 1513 $cm^{-1}$. FAB MS: calcd. for $C_{37}H_{38}N_{10}O_9S_2Na$: 853.2162; found 853.2106. UV (EtOH) $\lambda_{max}$ 370 (3290); 297 (12000); 260 (13300). N.M.R. (300 MHz, DMSO-$d_6$) δ 9.42 (bd, 1, J = 8); 9.13 (d, 1, J = 9); 8.52 (s, 1), 7.45-6.7 (m, 5); 6.6 (bs, 2); 5.88 (m, 1, allyl CH); 5.39 (dd, 1, J = 5, 8); 5.35-4.55 (m, 7) with 4.7 (s, 2) superimposed; 4.16 (m, 1); 3.70 (s, 2); 3.50 (AB q, 2, J = 10); 3.2 (s, 3); 2.1 (m, 2); 1.9 (m, 2).

Example 2

7-β-(2-(Thien-2-yl)acetamido)-3-((Methotrexate-gamma-Ester)methylene)-3-Cephem-4-Carboxylic Acid

Palladium (II) acetate (3 mg, 0.013 mmol) and triphenylphosphine (14 mg, 0.053 mmol) were combined in ethyl acetate (0.5 ml). Allyl 7-β-(2-(thien-2-yl)acetamido)-3-((Methotrexate ester)methylene)-3-cephem-4-carboxylate (130 mg, 0.15 mmol) was suspended in a mixture of 1:1:1 ethyl acetate : methanol : acetic acid followed by the addition of triethylsilane (0.1 ml). The resultant reaction mixture was stirred for 16 h then concentrated in vacuo. The concentrate was diluted with water (15 ml) and the pH of the solution was adjusted to 7 by the addition of $NaHCO_3$. The resultant solution was layered with ethyl acetate and the mixture was stirred for 1 h. The mixture was filtered, the phases were separated and the aqueous phase was extracted with additional ethyl acetate then freeze dried.

The freeze-dried solid was subjected to medium pressure liquid chromatography on a $C_{18}$ reverse phase column. The column was first equilibrated with a solution of 15% acetonitrile in water. Then the freeze dried material was loaded on the column and eluted first with 30% acetonitrile : 1% acetic acid in water (600 ml); then 40% acetonitrile : 1% acetic acid in water. Fractions containing the peak eluting at 4.8 min (Waters $C_{18}$ μ bondpak analytical column, eluted with 30% acetonitrile, 1% HOAc, water, 2 ml/min) were combined and freeze dried to give the title product: N.M.R. (300 MHz, DMSO-$d_6$) δ 9.0 (d, 1, J = 9); 8.52 (s,1); 7.7 (d, 2, J = 10); 7.64 (br s, 2); 7.30 (m, 1); 6.88 (m, 2); 6.75 (d, 2, J = 10); 6.55 (br s, 2); 5.42 (dd,

1, J=4.5,9); 4.94 (d, 1, J=11.6, 1/2 of ABq); 4.89 (d, 1, J=4.5); 4.80 (d, 1, J=11.5, 1/2 of ABq); 4.72 (s, 2); 4.06 (m,1); 3.60 (s, 2); 3.15 (s, 3); 2.1-1.7 (m, 4); I.R. (KBr) 1763, 1757, 1606, 1559 $cm^{-1}$. FABMS: found: 791.2037

Example 3

Allyl 7-β-(2-(Thien-2-yl)acetamido)-3-((N-1-(5-fluorouracil)yl)methylene)-3-Cephem-4-Carboxylate

Allyl 7-β-(2-(thien-2-yl)acetamido)-3-(acetoxymethylene)-3-cephem-4-carboxylate (1.0 g) was dissolved in methylene chloride (5 ml) then trimethylsilyl iodide (0.65 ml, 5 mmol) was added to the solution. The dark brown solution was stirred at room temperature for 90 min under a nitrogen atmosphere. The reaction mixture was diluted with ethyl acetate, then washed sequentially with cold 1N sodium thiosulfate, saturated aqueous sodium bicarbonate solution, and brine. The layers were separated and the organic layer was dried over sodium sulfate and concentrated in vacuo.

5-Fluorouracil (0.30 g, 2.3 mmol) was dissolved in dry DMF. Triethylamine (0.40 ml, 2.3 mmol) then the 3-(iodomethylene) cephem compound from above, dissolved in DMF, was added to the 5-fluorouracil solution. The resultant solution was stirred at room temperature for 2 hours then concentrated in vacuo. The concentrate was diluted with cold 1N HCl and ethyl acetate. The ethyl acetate layer was separated and dried over magnesium sulfate and concentrated in vacuo to give 0.70g of a brown solid. The brown solid was flash chromatagraphed over silica gel eluted with 5% methanol in methylene chloride. N.M.R. (300 MHz, $CDCl_3$) δ 10.10 (br s, 1), 7.81 (d, 1, J=5), 7.20 (m, 1); 7.05 (d, 1, J=7); 6.92 (m, 2), 6.0-5.8 (m, 2); 5.40-5.25 (m, 2,); 4.95 (d, 1, J=5); 4.85 (d, 1, J=9); 4.75 (d, 2, J=5); 4.41 (d, 1, J=9); 3.80 (s, 2); 3.45 (d,l, J=$l_2$); 3.30 (d, 1, J=12). IR (chloroform): 1792, 1720, 1704, 1671, 1507 $cm^{-1}$; UV (EtOH); $\lambda_{max}$ 272 ($\epsilon$ = 14,100), 238 ($\epsilon$ = 12,900). FABMS: M+1 = 507.

Example 4

7-β-(2-(Thien-2-yl)acetamido)-3-((N-1(5-Fluorouracil)yl)methylene)-3-Cephem-4-Carboxylic Acid

Tetrakis[triphenylphosphine] palladium [0] (8 mg, 0.006 mmol), and triphenylphosphine (3 mg, 0.01 mmol) were combined in a 1:1 mixture of methylene chloride and ethyl acetate (2 ml). Allyl 7-β-(2-(Thien-2-yl)acet-amido)-3-((N-1(5-fluorouracil)yl)methylene)-3-cephem-4-carboxylate (0.112g, 0.221 mmol) was also dissolved in a 1:1 mixture of methylene chloride and ethyl acetate and the resultant brown solution was added to the catalyst-containing solution at room temperature. To this solution was added sodium 2-ethylhexanoate ( 0.040 g, 0.243 mmol). The resultant solution was stirred for 1.5 hours and the precipitate was isolated by first adding acetone to reaction mixture and transferring the mixture to a centrifuge tube. After centrifugation, the supernatant was discarded and the beige precipitate washed with ether and dried in a vacuum descicator. While drying, the beige solid turned brown. The brown solid was dissolved in water, washed with ether, and freeze-dried to give 85 mg of off-white solid. The solid was subjected to reverse-phase HPLC (Waters Associates C-18 column) eluted with 15% acetonitrile/1% acetic acid/water. The product-containing fractions were combined and freeze-dried to give 15 mg of the title product: N.M.R. (DMSO-$d_6$, 300 MHz) δ 9.08 (d, 1, J = 8.3); 8.24 (bd, 1, J= 5.8); 7.30 (m, 1); 6.88 (m, 2); 5.59 (dd, 1, J = 4.9 and 3.4); 4.97 (d, 1, J= 4.9); 4.72 ( d, 1, J= 14.6); 4.23 (d, 1, J=14.6); 3.68 (s, 2); 3.50 (d, 1, J=14.6); (other doublet obscured by water in DMSO at 3.3 δ). IR ($CHCl_3$) 1782, 1695, 1666 $cm^{-1}$. UV (ethanol): $\lambda_{max}$ 271 nm ($\epsilon$ = 10,800), 237 ($\epsilon$ = 10,200). FABMS : Found: 467.0489.

Procedure 3

N-t-Butoxycarbonyl-2-Aminoethanethiol

2-Aminoethanethiol hydrochloride (9.9 g, 0.08 mol) was suspended in acetonitrile (approximately 25 ml) under a nitrogen atmosphere. Diisopropylamine (14.9 ml) was added as the mixture was kept at 0°C during the addition. After 10 minutes at 0°, di(t-butyl)dicarbonate (20.0ml) was added and the resultant mixture was stirred at 0°C for 15 minutes then at room temperature for another 45 minutes. The reaction mixture was concentrated in vacuo and a 40:60 mixture of ethyl acetate:hexane was added. The white solid thus formed was removed by filtration. The filtrate was flash chromatographed over a 17.8 cm (7 inch) silica gel column eluted with a 40:60 mixture of ethyl acetate:hexane. The product-containing fractions were combined and concentrated in vacuo to give 15 g of a colorless oil of the title product. N.M.R. (300 MHz,

$CDCl_3$): δ 4.92 (bs, 1, NH); 3.28 (q, 2, J=6.3); 2.62 (q, 2, J=6.9); 1.42 (s, 9); 1.33 (t, 1, J=8.5).

Procedure 4

7-β-(2-(Thien-2-yl)acetamido)-3-((N-(t-Butoxycarbonylamino)-2-ethylsulfide)methylene)-3-Cephem-4-Carboxylic Acid

Sodium 7-β-(2-(Thien-2-yl)acetamido)-3-(acetoxymethylene)-3-cephem-4-carboxylate (2.0 g, 5.05 mmol) was stirred in distilled water, followed by the addition of potassium iodide (8 g) and N-t-butoxycarbonyl-2-aminoethanethiol (0.87 g). The mixture was heated slowly to 65°C and held at this temperature for 2 hours, then at 70°C for an additional 2 hours. The reaction mixture was cooled and poured into a mixture of ice water and ethyl acetate with stirring. 1N Hydro-chloric acid was added to the mixture to adjust the pH to 2. The ethyl acetate layer was collected, dried over magnesium sulfate, filtered and concentrated in vacuo to give 2 g of a white solid of the title product. N.M.R. (300 MHz, DMSO-$d_6$) δ 9.10 (d, NH, J=9); 7.30 (m, 1); 6.88 (m, 2); 5.59 (m, 1); 5.05 (d, 1, J=5); 3.71 (s, 2); 3.60 (m, 4); 3.26 (bs, 2); 3.02 (m, 2); 2.45 (m, 2, overlap with DMSO signal).

Procedure 5

Allyl 7-β-(2-(Thien-2-yl)acetamido)-3-(((1-(t-Butoxycarbonylamino)-2-ethylsulfide)methylene)-3-Cephem-4-Carboxylate

7-β-(2-(Thien-2-yl)acetamido)-3-(((1-(t-butoxycarbonylamino)-2-ethylsulfide)methylene)-3-cephem-4-carboxylate (0.50 g, 0.975 mmol), allyl bromide (0.25 ml, 2.92 mmol) and sodium bicarbonate (0.4 g) were combined in 5 ml DMF and stirred overnight at room temperature under a nitrogen atmosphere. The reaction mixture was diluted with ethyl acetate and 0.1 N hydrochloric acid. The organic layer was separated, dried over magnesium sulfate, filtered, and concentrated in vacuo to give 0.5 g of a yellow oil. The oil was flash chromatographed over 6 inches of silica gel eluted with 1:1 ethyl acetate:hexane and the product-containing fractions were combined and concentrated in vacuo to give 200 mg of the title product. N.M.R. ($CDCl_3$, 300 MHz) δ 7.20 (m, 1); 6.92 (m, 2); 5.90 (m, 1); 5.75 (dd, 1, J=5,9); 5.30 (m, 2); 4.98 (d, 1, J=5); 4.68 (d, 2); 3.80 (s, 2); 3.55 (q, 2, J=12); 3.4 -3.1 (m, 4); 2.6 (t, 2, J=5); 1.40 (s, 9). IR (KBr) 1771, 1756, 1714, 1680, 1670, 1531 $cm^{-1}$. FDMS: M+1 = 554.

Example 5

Allyl 7-β-((2-Thien-2-yl)acetamido)-3-(((1-(Desacetylvinblastine)amino)-2-ethylsulfide)methylene)-3-Cephem-4-Carboxylate

Allyl 7-β-((2-thien-2-yl)acetamido)-3-(((1-(t-butoxycarbonylamino)-2-ethylsulfide)methylene)-3-cephem-4-carboxylate (0.46 g, 0.823 mmol) was dissolved in methylene chloride (5 ml) and cooled to 0°C under a nitrogen atmosphere. Trifluoroacetic acid (neat, 2 ml) was added and the resultant mixture was stirred for 1 hr. The reaction mixture was concentrated in vacuo, diluted with toluene and concentrated in vacuo twice, then diluted with $CHCl_3$ and concentrated in vacuo twice to give the deprotected amino compound.

The deprotected amino compound was dissolved in methylene chloride (3 ml) and the solution was cooled to 0°C. N-Methylmorpholine (0.3 ml) was added to the mixture followed by the slow addition of a methylene chloride solution (5 ml) of desacetylvinblastine azide (preparation described in U.S. Patent No. 4,203,898, Example 5, Column 20) (0.47 g, 0.543 mmol). The resultant mixture was stirred at 0° for 30 min, the reaction mixture was concentrated to 1/2 of its original volume then stirred overnight at room temperature. The reaction mixture was diluted with water, the layers were separated and the organic layer was dried over sodium sulfate, filtered and concentrated in vacuo. The concentrate was flash chromatgraphed over silica gel eluted with 4% methanol in methylene chloride. The product-containing fractions were combined and concentrated to give 100 mg of the title product. N.M.R. ($CDCl_3$) δ (mixture of Δ-2 and Δ-3 isomers) 9.68 (s, 1); 9.58 (s, 1); 8.60 (d, 1, J=9): 8.05 (s, 1); 7.55 (d, 1, J=9): 7.25-7.10 (m,6); 7.0 (m,2); 6.90 (s, 1): 6.60 (s, 1): 6.10 (s,1); 6.0-5.6 (m,5), 5.60 (s, 1); 5.40-5.3 (m, 2); 4.8-4.6 (m,3); 3.86 (s, 2); 3.80 (s,3); 3.75 (s, 2); 3.62 (s, 3): 2.80 (s, 3); 0.90 (two overlapping triplets, 6); remainder of protons are a mixture of multiplets from 4.0-1.0. UV (ethanol) $\lambda_{max}$ 265 nm (ε=0.6609), 214 (ε=1.8358). FABMS: M+1 = 1190.4720.

Example 6

7-β-(2-(Thien-2-yl)acetamido)-3-(((1-(desacetylvinblastine)amino)-2-ethylsulfide)methylene)-3-Cephem-4-Carboxylic Acid

Under a nitrogen atmosphere, tetra-kis[triphenylphosphine] palladium [0] (5.8 mg) and triphenylphosphine (1.8 mg) were dissolved in 1:1 ethyl acetate:hexane. Allyl 7-beta-(2-(thien-2-yl)acetamido)-3-(((1-(des-acetylvinblastine)amino)-2-ethylsulfide)methylene)-3-cephem-4-carboxylate (0.20 g, 0.168 mmol) was added to the solution followed by the addition of triethylsilane (40.3 μl, d = 0.728). The reaction mixture was stirred at room temperature. After 1 hour, glacial acetic acid (2 drops) was added and the mixture was stirred for an additional hour. Ether was added to the reaction mixture to precipitate the product, and the reaction mixture was centrifuged to collect the resultant beige precipitate (85 mg). The precipitate was chromatographed by reverse phase HPLC using a Waters $C_{18}$ column eluted at 10 ml/min with 30% acetonitrile, 0.2% formic acid in water, monitored at 254 nm. The product-containing fractions were combined and concentrated to give 30 mg of white solid title product. N.M.R. (DMSO-$d_6$, 300 MHz) δ 9.30 (s, 1); 9.0 (d, 1, J = 9); 8.45 (m, 1); 8.17 (brs, 1); 7.9-7.6 (m, 3); 7.31 (m, 2); 7.20 (d, 1, J = 9); 7.0-6.8 (m, 4); 6.39 (s, 1); 6.14 (s, 1); 5.7-5.5 (m, 2); 5.40 (dd, 1, J = 5,9); 4.92 (d, 1, J = 5); 3.90 (s, 1); 3.70 (s, 2); 3.67 (s, 3); 3.49 (s, 3); 2.67 (s, 3); 0.74 (t, 3, J = 7); 0.68 (t, 3, J = 7); remainder of protons are several multiplets from δ 4.9-1.1. IR (KBr) 1768, 1764, 1648, 1630, 1616 $cm^{-1}$; UV (ethanol) $\lambda_{max}$ 267 nm (ε = 22,900), 215 (ε = 53,800); FABMS: 1150.44435.

Example 7

Allyl 7-β-(2-(Thien-2-yl)acetamido)-3-((Desacetylvinblastinehydrazido)methylene)-3-Cephem-4-Carboxylate

Allyl 7-β-((2-(Thien-2-yl)acetamido)-3-(acetoxymethylene)-3-cephem-4-carboxylate (872 mg, 2 mmol) was dissolved in methylene chloride (20 ml). To this solution was added trimethylsilyl iodide (0.57 ml, 4 mmol). The resulting solution was stirred at room temperature for 1 h then diluted with ethyl acetate, washed with ice cold 1N sodium thiosulfate, saturated aqueous sodium bicarbonate solution and brine. The layers were separated and the organic layers were dried over sodium sulfate and concentrated in vacuo.

The residue was dissolved in DMF (4 ml). To this solution was added solid sodium bicarbonate (0.504 g, 6 mmol) then desacetylvinblastine hydrazide (preparation described in U.S. Patent No. 4,203,898) (1.74 g, 2 mmol) was washed into the solution with DMF (8 ml). The resulting solution was stirred at room temperature for 5 h. Water (100 ml) was added to give a yellowish precipitate, which was collected by filtration. The precipitate was dissolved in chloroform, dried over sodium sulfate, filtered, and concentrated in vacuo to give 1.9 g of a foam. The foam was flash chromatogrphed on silica eluted with 10% isopropanol in chloroform to give 470 mg of the title product. N.M.R. (300 MHz, $CDCl_3$) δ 9.6 (br s, 1); 8.28 (d, 1, J = 6); 8.04 (s, 1); 7.55 (d, 1, J = 7); 7.3-7.0 (m, 6); 6.60 (s, 1); 6.35 (d, 1, J = 9); 6.1 (s, 1); 5.95 (m, 1); 5.84 (dd, 1, J = 5,9); 5.75 (d, 1, J = 9); 5.30 (m, 2); 5.01 (d, 1, J = 5); 5.0-4.65 (m, 3); 4.20 (m, 1); 3.87 (s, 2); 3.80 (s, 3); 3.62 (s, 3); 3.60 (ABq, 2, J = 17), 2.75 (s, 3); 0.95 (2 overlapping triplets, 6, J = 7); the remainder of the protons generate signals in an envelope of multiplets from 4.0-1.0. IR (KBr): 1783, 1724, 1674, 1616, 1504, 1459, 1225 $cm^{-1}$. UV (EtOH) $\lambda_{max}$265 nm (ε = 23,500), 215 (ε = 60,800). FABMS: M + 1 = 1145.4825.

Example 8

7-β-(2-(Thien-2-yl)acetamido)-3-((Desacetyl-vinblastinehydrazido)methylene)-3-Cephem-4-Carboxlic Acid

Allyl 7-β-(2-(Thien-2-yl)acetamido)-3-((Desacetylvinblastinehydrazido)methylene)-3-cephem-4-carboxylate (340 mg, 0.3 mmol) was dissolved in methylene chloride (6 ml). To this solution was added glacial acetic acid (0.16 ml, 2.8 mmol) then bis[triphenylphosphine]palladium [II] chloride (21 mg, 0.03 mmol) and triethylsilane (0.16 ml, 1mmol). The resultant solution was stirred at room temperature for 22 hrs under $N_2$. The reaction mixture was loaded directly on a flash chromatography column and the column was eluted with 20% isopropanol in chloroform. The product-containing fractions were combined and concentrated in vacuo to give 80 mg of the title product. N.M.R. (300 MHz, DMSO-$d_6$) δ 9.9 (s, 1); 9.4 (br s, 1); 9.15 (d, 1, J = 9); 8.75 (br s, 1); 7.4 (m, 1); 7.36 (dd, 1, J = 1,5); 7.27 (d, 1, J = 9); 7.05-6.9 (m, 4); 6.43 (s, 1); 6.20 (s, 1); 5.75 (dd, 1 J = 5,9); 5.70 (dd, 1, J = 4, 10); 5.58 (d, 1, J = 10); 5.10 (d, 1, J = 5); 4.30 (d, 1, J = 7); 4.12 (ABq, 2, J = 17); 3.76 (s, 2); 3.70 (s, 3): 3.53 (s, 3); 2.83 (s, 3); 0.8 (t, 3, J = 7); 0.72 (t, 3, J = 7); the remainder of the protons generated signals that were in an envelope of multiplets from δ 3.8-1.1. IR (KBr) 1786, 1720,

1684, 1460 $cm^{-1}$; UV (EtOH) $\lambda_{max}$ 264 nm ($\epsilon$ = 23,200); 215 ($\epsilon$ = 62,000). FABMS: M-17 = 1087.

Example 9

Synthesis of Antibody (F(ab')) Enzyme Conjugate

A solution of Sulfo-SMCC (Pierce, Rockford, Ill.) was prepared in isotonic phosphate buffered saline, pH 7.2 (PBS) at a concentration of approximately 35 mM. The true concentration of the solution was determined by measuring the absorbance at 260 nm of an aliquot before and after hydrolysis with 0.3 M Na2$CO_3$, using a molar extinction coefficient of 8,000.

Beta-Lactamase ("B-L") from the P99 strain of Enterobacter cloacae (available from Sigma, St. Louis, Mo.) was purified according to the method of Cartwright and Waley (Biochem J., 221, pp.505-512 (1984)), then dialyzed with distilled water and lyophilized. A 20 mg portion of the powder was dissolved in 2 mL 50 mM sodium borate, 100 mM sodium chloride, pH 8.0 (BBS) containing 0.01% Nonidet P-40 (Sigma, St. Louis, Mo.), and its exact concentration measured by absorbance at 280 nm (extinction 1 mg/mL = 1.4, Cartwright and Waley, Biochem., 26, pp.5329-5337 (1984)).

A volume of Sulfo-SMCC solution containing 2.0 molar equivalents (~35 µL) was added to the B-L solution, and the pH was adjusted to 8.0 with 0.3 M $Na_2CO_3$. The unstirred solution was left at room temperature for 1 hour. The reaction mixture was then eluted through a 50 mL P6DG (Bio-Rad, Richmond, Ca) column with 50 mM ammonium citrate, 1 mM DTPA, 100 mM NaCl, pH 6.2.

The protein containing peak (monitored at 280 nm) was collected in a single fraction, and its concentration determined by measurement of the Absorbance at 280 nm of an aliquot of the solution. The maleimide content of the protein solution was determined by reaction of an aliquot with a twofold excess of cysteine (10 min, room temperature), and detection of the residual cysteine with DTNB: molar extinction coefficient at 412 nm of released thionitro-benzoic acid = 13,600. Under the stated reaction conditions, an average molar ratio of ~0.9 maleimide/B-L is obtained. The derivatized protein was used within two hours.

F(ab') fragments of the anti-carcinoembryonic antigen antibody, CEM231, were prepared by pepsin digestion followed by cysteine reduction according methods known to the art and described by Breman et al., and Glennie et al. cited above. The ratio of free thiol/F(ab') was determined by measuring the Absorbance at 280 nm of an aliquot of the protein solution (extinction of a 1 mg/mL solution = 1.4), and quantitating the thiol concentration with DTNB. Typical average thiol/F(ab') ratios are in the range of 2.0 to 2.5.

Molar equivalents of maleimide derivatized B-L and CEM231 F(ab') at a concentration of 1 to 5 mg/mL each were reacted at room temperature, unstirred, for 1 hour in 50 mM ammonium citrate, 1 mM DTPA, 100 mM NaCl, pH 6.2. At the end of the reaction period, excess N-ethyl maleimide was added to stop the reaction.

The reaction mixture was then applied directly to a 500 mL Sephadex® G-150 Superfine column and eluted with BBS buffer. Product fractions were those determined by PAGE to contain pure material of molecular weight ~90,000. Pooled product fractions were then concentrated to ~1.5 mg/mL, sterile filtered, and stored in sterile septum capped vials.

Procedure 6

Determination of Beta-Lactamase Enzymatic Activity

Enzymatic activity was quantified using either cephalothin (Sigma Chemical, St. Louis, Mo.) or PADAC (CalBiochem, San Diego, CA) as substrate, in a Hewlett-Packard 8451A Spectrophotometer equipped with a stirred thermostable cell holder, monitored at 264 or 570 nm, respectively. In a typical assay, substrate, at an assay concentration of 50 µM, was added to a stirred, referenced solution of ~5 nM P-99 E. cloacae beta-lactamase Enzyme ("B-L") in an appropriate buffer (PBS, HEPES, etc.) at an appropriate pH (generally 7.2) equilibrated at 37°C. (The B-L Enzyme (also referred to as "pencillinase") can be obtained both commercially (e.g. Sigma Chemical Co., St. Louis, Mo.) and by known published procedures. The change in absorbance was then determined by the spectrophotometer at intervals of 5 or more seconds.

Determination of $K_m$ and $V_{max}$ for B-L Enzyme and Conjugates

A stock solution of cephalothin was prepared by dissolving 5.5 mg in 1.0 mL 10 mM sodium phosphate, 150 mM sodium chloride, pH 7.2 (PBS): 13 mM. A stock solution of P-99 E. cloacae beta-lactamase ("B-L") enzyme was determined by $A_{280}$ to be 1.3 µM. B-L solution, 7.7 µL, was added to a cuvette containing stirred, temperature equilibrated PBS, such that a final volume of 2.0 mL would be obtained, and the spectrophotometer was referenced. Substrate stock solution was then added to obtain assay concentrations between 5 and 130 µM. The spectrophotometer was instructed to wait 5 sec. for initial substrate mixing, then record the absorbance at 264 nm every 10 sec for 2 min.

The results were converted to change in absorbance from the initial value, and plotted using the RS/3 program (BBN Software Products Co., Cambridge, Mass.). Inspection of the graphs showed that the initial rates were identical above 50 µM substrate concentration, and that initial rates could be estimated from times prior to 25 sec. (In subsequent determinations, absorbance readings were collected every 5 sec). The Δ-OD values after 20 sec. of reaction were converted to Δ-[substrate]/sec. and were correlated with substrate concentration in a double reciprocal plot: 1/V vs 1/[S] ( wherein V = Δ[substrate]/second ), from which $K_m$ and $V_{max}$ were determined with their associated error limits. $K_{cat}$ was calculated based on the relation that: $k_{cat} = V_{max}/[E]$ (The substrate concentrations were calculated from the 264 nm absorbance at the 5 sec time point using a molar extinction coefficient of 8,540 for cephalothin.)

Similar experiments were performed on two separate lots of B-L-CEM231 conjugate. The values for $K_m$ were4.8±0.1, 4.4±0.3, and 5.2±0.4 µM; and for $k_{cat}$ 54±0.7, 35±1.4, and 49±2.8 sec-1, for B-L and the two lots of conjugate, respectively.

Procedure 7

Demonstration of Immunoreactivity of Beta-Lactamase Conjugated CEM231

Iodination: 10 µg conjugate at 1 µg/µL was treated with 200 µCi $^{125}I$ in the presence of Biorad Enzymobeads (Biorad, Richmond, CA) and 25 µL 1% β-D-glucose at room temperature for 20 min in 0.2 M sodium phosphate, pH 7.1. The mixture was quenched with 50 µL of a solution of PBS containing 0.1% sodium azide and 0.35 mg/mL sodium metabisulfite, pH 7.2. Carrier was added in the form of 100 µL of PBS containing 0.1% sodium azide, 0.1% gelatin, and 1% sodium iodide.

The mixture was purified on a 5 mL Sephadex® G-25 column equilibrated with PBS, 0.1% sodium azide, 0.1% gelatin solution, and eluted with PBS containing 0.1% sodium azide, pH 7.5, into tubes containing 100 µL of a 0.1% gelatin solution in 0.5 mL fractions. The fractions were counted for radioactivity in an ISODATA 20/20 Series Gamma Counter (RIA Data Systems, Rolling Meadows, Ill.), and the first peak of radioactivity was the labeled conjugate.

Immunoreactivity: Polystyrene beads were prepared with carcinoembryonic antigen (CEA) or with irrelevant protein (for non-specific binding control) by soaking the beads in a solution of the desired protein at a concentration of 150 ng/bead in 10% horse serum. Iodinated conjugate was diluted to about 200 cpm/µL in 10% horse serum. Three each antigen positive and antigen negative beads were placed in individual gamma counting tubes, washed, and treated with 200 µL labeled conjugate. The beads were incubated at 37°C overnight. Total counts were measured in each tube; supernatants were transferred to tubes containing a second bead of the same type. Finally, all beads were washed, and the fraction of counts on each bead was determined. Immunoreactivity was calculated as:

$$A_+ + B_+(1-A_+),$$

and Non-specific Binding was calculated as:

$$A_- + B_-(1-A_-),$$

where $A_+$ and $B_+$ are the fractions bound on the antigen positive beads in the first and second rounds, respectively, and $A_-$ and $B_-$are the fractions bound on the antigen negative beads in the first and second rounds, respectively.

Immunoreactivity for the B-L-CEM231 conjugate was found to be about 80%, which compares well with values obtained for unmodified F(ab′) fragments of CEM231. Non-specific binding had also not increased by conjugation with beta-lactamase.

Procedure 8

Demonstration of Release of Substituents from the 3′-Methylene Group of Cephalosporins on Treatment with Beta-Lactamase

Release of substituents (Cytotoxic Agents) from the 3-position of cephalosporins can be demonstrated by chromatographic (especially HPLC) analysis of the reaction mixture, or by spectrophotometric assay of the released functional group. (If the detection method is not instantaneous, the enzyme can be stopped at the desired time by addition of an inhibitor such as cloxacillin (Sigma, St. Louis, Mo.) at its effective concentration: 5 µM for cloxacillin.)

For instance, a model system for the present method employs the expulsion of aminoethanethiol from 7-β-(2-(thien-2-yl)acetamido)-3-((2-aminoethyl-1-sulfide)methylene)-3-cephem-4-carboxylic acid. A typical enzyme assay was performed (following the 264 nm absorbance), but in 50 mM Tris, 100 mM NaCl buffer, pH 8.0. Quantitation of the release of thiol was achieved in a subsequent assay by including 250 mM DTNB (dithionitrobenzoic acid) in the reaction mixture, and observing the change in absorbance at 412 nm. Using molar extinction coefficients of 8,540 for the 264 nm absorbance, and 13,600 for the 412 nm absorbance, the rates of the two reactions can be compared (reaction of thiol with DTNB is instantaneous under these conditions). The graphs of the results showed that thiol is released as a result of beta-lactamase catalyzed cleavage of the cephalosporin, but that the release rate is slower than the catalytic rate.

To demonstrate the expulsion of 5-fluorouracil from 7-β-(2-(thien-2-yl)acetamido)-3-((N-1(5-fluorouracil)-yl)methylene)-3-cephem-4-carboxylic acid ("ceph-5-FU"), a stock solution of the cephalosporin was prepared (1 mg of cephalosporin in 2 ml of 20% N,N-dimethylacetamide in pH 7.0 HEPES buffer, final concentration of 500 µg/ml). A stock solution of P-99 E. cloacae beta-lactamase Enzyme was prepared. To the stock solution of the cephalosporin Substrate (90 µl) was added stock beta-lactamase solution (10 µl). The reaction was monitored by HPLC (on a Waters Associates $C_{18}$ µ Bondapak column) eluted with 25% acetonitrile/ 1% acetic acid in water (monitered at 254 nm absorbance). The retention time for 5-fluorouracil was 1.9 minutes and was 5.3 minutes for the cephalosporin - (5-fluorouracil) compound. Approximately 30 seconds after mixing, a 5 µl aliqout of the reaction mixture was injected into the HPLC. The HPLC trace indicated that the cephalosporin had been consumed, and a new compound with a retention time of 4.3 minutes appeared, along with a small amount of 5-fluorouracil with a retention time of 1.9 minutes. Twenty-one minutes after mixing the two stock solutions, the compound eluting at 4.3 minutes was diminished and a large amount of compound eluting at 1.9 minutes was observed. Thirty-six minutes after mixing the stock solutions the amount of compound eluting at 4.3 minutes was further diminished, and the amount of compound eluting at 1.9 minutes was correspondingly larger by intergration of the peak areas.

Thus, the Enzyme caused conversion of the cephalosporin-(5-fluorouracil) Substrate-Cytotoxic Agent to the Cytotoxic Agent.

Procedure 9

Tumor Localization of Iodinated B-L-CEM231 Conjugate

Conjugate was labeled with $^{125}I$ as described for the immunoreactivity assay. Twelve nude mice in which LS174T tumor cells (available from the ATCC under accession number ATCC CL188) had been injected subcutaneously and allowed to reach a size of about 0.4 g were injected in the tail vein with 50 µg of the labeled conjugate in 100 µL BBS. At 4 and 24 hours 6 mice were sacrificed, dissected, and the weight and amount of radioactivity in each organ was quantitated. The results are reported as % of injected dose/gm tissue.

The results are presented in the following Table:

Tumor Localization Studies

| Blood | Bone | Heart | Kidney | Liver | Lung | Muscle | Skin | Spleen | Tumor | GI |
|---|---|---|---|---|---|---|---|---|---|---|
| 4 HOUR | | | | | | | | | | |
| 15.8 | 2.4 | 4.3 | 7.4 | 3.7 | 8.1 | 1.3 | 3.8 | 4.2 | 13.9 | 3.1 |
| 24 HOUR | | | | | | | | | | |
| 1.5 | 0.2 | 0.4 | 0.7 | 0.6 | 1.0 | 0.1 | 0.8 | 0.4 | 7.5 | 0.7 |

These results show that the conjugate does accumulate in tumor preferentially, and that it shows no untoward accumulation in critical organs. It also shows a rapid clearance from the serum which is desirable from the standpoint of conversion of Substrate-cytotoxic Agent to Cytotoxic Agent specifically at the target. The results are comparable to those seen for murine hybrid antibodies of the same approximate molecular weight, and show no unusual metabolic treatment of the bacterial protein.

Procedure 10

Enzymatic Assay of Conjugate Bound to Antigen Expressing Cells

In order to show that beta-lactamase activity could be bound to tumor cells via the prepared B-L-CEM231 conjugate, CEA expressing LS174T cells and CEA negative MOLT4 cells were prepared as single cell suspensions in Autopow media (GIBCO, Grand Island, NY). Both suspensions were at a density of $2x10^7$ cells/mL; LS174T were 23% viable, MOLT4 80% viable. Suspensions were maintained on ice until use.

Cells (1 mL) were incubated for either 1 or 10 min. with 10 or 50 pmol of conjugate or unconjugated beta-lactamase. Cells were then centrifuged, the supernatant discarded, re-suspended in buffer, centrifuged again, then suspended in 1.0 mL of buffer containing PADAC (CalBiochem, San Diego, CA) at a known concentration (determined spectrophotometrically) around 30 µM. After incubating 10 min. with substrate, the cells were centrifuged, and the concentration of PADAC in the supernatant determined. Results are reported as % of original absorbance remaining after incubation at the PADAC peak absorbance wavelength of 570 nm.

| Results: | | | |
|---|---|---|---|
| Conjugate | Quantity pmol/ml | Binding Time min | %$OD_{570}$ Remaining after incubation |
| | LS174T Cells | | |
| B-LCEM231 | 10 | 10 | 46 |
| B-LCEM231 | 50 | 10 | 23 |
| B-L | 10 | 10 | 95 |
| B-L | 50 | 10 | 87 |
| B-LCEM231 | 10 | 1 | 62 |
| B-LCEM231 | 50 | 1 | 36 |
| | MOLT4 Cells | | |
| B-LCEM231 | 10 | 10 | 94 |
| B-LCEM231 | 50 | 10 | 93 |

These results show that beta-lactamase activity can be bound to antigen positive cells in a specific manner, that the cells have no such activity in the absence of the conjugate, that binding does not impede the catalytic activity of the enzyme, and that the binding is time and concentration dependent.

Procedure 11

PADAC Assay of Serum Kinetics of B-L-CEM231 Conjugate

B-L-CEM231 conjugate (50 µg) in 50 mM sodium borate, 100 mM NaCl (BBS) was injected into the tail vein of each of 18 tumor-free nude mice. Three mice each at 1, 24, 48, 72, 96, and 120 hours after injection were bled, and their sera pooled and frozen. The serum samples were assayed for B-L activity using the standard assay conditions. Appropriate quantities of serum were diluted to 2.0 mL with PBS, and the time of the assay was varied as necessary to detect activity. PADAC (20 µL of an EtOH solution prepared by dissolving 2.4 mg/mL) was used as substrate to avoid interference from serum proteins.

Linear portions of the Δ-absorbance vs time plots were determined by inspection and those points were fitted by linear regression (RS/3, BBN Software Products). The slopes of these lines were compared to the slope obtained for a known amount of conjugate under identical conditions, and corrected for dilution, to calculate the concentration of conjugate present in the serum.

| Results: | | | |
|---|---|---|---|
| Time (hr) | Slope x$10^4$ | µL Serum in Assay | µg B-LCEM231/mL Serum |
| 1 | 27.0 | 10 | 20 |
| 24 | 13.3 | 100 | 1.0 |
| 48 | 2.2 | 50 | 0.33 |
| 72 | 4.3 | 200 | 0.16 |
| 96 | 5.3 | 600 | 0.06 |
| 120 | 5.2 | 600 | 0.06 |

These results show that no beta-lactamase activity exists in normal serum of these mice, and that the serum level drops rapidly, so that Substrate-Cytotoxic Agent treatment could begin relatively soon (48 hr) after injection of conjugate (in nude mice). Similar experiments would show when sufficient serum clearance

had occurred in humans. Comparison of the enzymatically determined level of conjugate with the level determined by radiodetection of iodinated conjugate, (measured at 4 and 24 hours after injection), shows that enzyme is not inactivated or inhibited in the serum. (See Procedure 9)

Procedure 12

Beta-Lactamase Activity Bound to Antigen Expressing Tumor Tissue In Vivo

Nude mice implanted with LS174T or MOLT4 cells which had been allowed to develop into 0.5 to 1.0 g tumors were injected in the tail vein with 50 µg of B-L-CEM231 conjugate diluted in 100 µL BBS. Twenty four hours after injection the tumors were excised and cut into ~100 mg chunks.

The chunks were minced, then incubated 10 min. with a 1 mL PBS solution to which had been added 10 µL of a stock solution made by dissolving 1 mg of PADAC in 400 µL 100% EtOH. Exact concentrations of PADAC in the assay solutions were determined spectrophotometrically ($\epsilon_{570\ nm}$ 48,000). The samples were then centrifuged (10 min, 1000x g), and the supernatants examined spectrophotometrically. Because the supernatants were very cloudy, quantitation was impossible, but the color change from purple to orange was clearly visible only in the LS174T tumor sample. The result was recorded photographically. Identical positive results were obtained in sections of LS174T tumors from two different nude mice, and negative results from sections of MOLT4 tumors from two different nude mice.

Procedure 13

In vitro Cytotoxicity Determination

Target cells (antigen positive or antigen negative) are resuspended in 75% leucine deficient EBSS-MEM (GIBCO, Grand Island, NY) + 10% dialyzed fetal bovine serum (FBS) + gentamicin (GIBCO) at 200,000 cells/ml. Aliquots of this cell suspension, 0.2 ml, are seeded into 96 well plates and incubated overnight at 37°C, 5% $CO_2$. To the supernatants is added the antibody-enzyme conjugate to a final concentration of 25 µg/ml (unconjugated enzyme, unconjugated antibody, no treatment). The supernatants are removed after 1 hr incubation, and the cells rinsed once with media. The media is then replaced with the same media, but containing a Substrate-Cytotoxic Agent at concentrations between 0.001 and 10 µg/ml. The cells are incubated with the Substrate-Cytotoxic Agent compound for a period of time ranging from 3 to 48 h at 37°C, 5% $CO_2$. Media containing 4 µCi of $^3$H-leucine are added per well. The cells are incubated 24 hr with the labeled leucine, then harvested. Uptake of labeled leucine is determined by liquid scintillation counting. (All Samples are run in triplicate.) Results are reported as the Substrate-Cytotoxic Agent concentration at which leucine incorporation is reduced to 50% of the control value ($ID_{50}$).

As an example of the above general procedure, the cytotoxicity of 7-β-(2-(thien-2-yl)acetamido)-3-((((1-(desacetylvinblastine)amino)-2-ethylsulfide)methylene)-3-cephem-4-carboxylic acid ("COMPOUND") towards CEA - bearing LS174T tumor cells that were either treated or untreated with B-L-CEM231 conjugate ("conjugate") was measured. Thus, the appropiate wells were exposed to the B-L-CEM 231 conjugate at 25 µg/ml for 1 hour at 37°C, 5% $CO_2$. The cells were then rinsed and resuspended in media containing the compound. At the 24 hour time point, the appropriate wells were washed and 0.2 ml of fresh media was added to complete the 48 hour incubation. The rest of the experiment was carried using the above standard procedure.

The results of this experiment are presented in the following Table:

**In Vitro Cytotoxicity Data**

| Cells Treated With[1]: | COMPOUND treatment time: 6 Hours | 48 Hours |
|---|---|---|
| 1) COMPOUND and conjugate: | 0.265 | <0.001 |
| 2) COMPOUND alone: | .154 | <0.001 |
| 3) Cytotoxic Agent[2] alone: | 0.26 | <0.001 |

[1] values are $ID_{50}$ ($\mu g/ml$)
[2] Desacetylvinblastine aminoethanethiol

These data show that the COMPOUND is much less toxic to the tumor cells than the Cytotoxic Agent from which it is derived, but that when the cells are first treated with the conjugate, the toxicity of the COMPOUND is dramatically increased.

Procedure 14

N-(p-toluylsulfonyl)-N'-(p-Chlorophenyl)urea

Tosyl isocyanate (34.6g, 0.175 mol) was added to toluene ( 300 ml) under a nitrogen atmosphere. 4-Chloroaniline (22.4g, 0.176 mol) in toluene (200 ml) was added to the isocyanate solution over a period of ten minutes. A precipitate was formed soon after addition of the aniline solution. The resultant reaction

mixture was stirred overnight at room temperature under nitrogen.

The resultant precipitate was collected by filtration. The collected solid was washed with toluene (2x, 100 ml) then dried in vacuo at 60 °C to give 49.3 g of the title product. Elemental Analysis: Cal'd: C, 51.77; H, 4.04; N, 8.63. Found: C, 52.00; H, 4.09; N, 8.54.

Example 10

Allyl-7-β-(2-(Thien-2-yl)acetamido)-3-(N-((N-(p-tosyl)-N′-(p-Chlorophenyl)ureido)methylene)-3-Cephem-4-Carboxylate

Allyl 7-β-(2-(thien-2-yl)acetamido)-3-(acetoxymethylene)-3-cephem-4-carboxylate (2.0g, 4.59 mmol) was converted to the 3-iodomethylene derivative using trimethylsilyl iodide (0.98 ml) as described above in Procedure 2. N-(p-Tosyl)-N′-(p-chlorophenyl)urea (1.0 g, 3.08 mmol) was dissolved in dried DMF (5 ml). Sodium hydride (0.15g of a 60% dispersion) was added to the solution at room temperature. The resultant reaction mixture was heated to 65 °C under nitrogen. After 10 minutes, a DMF solution of the 3-iodomethyl cephalosporin was added over a period of 2 minutes. The resultant solution was stirred at 50 °C for 30 minutes then cooled and poured into water. The reaction mixture was extracted with ethyl acetate and brine then dried over magnesium sulfate (filtered and concentrated in vacuo.) The concentrate was flash chromatographed over silica gel eluted with a 3% methanol in methylene chloride mixture to yield 0.55g of the title product: N.M.R. ($CDCl_3$, 300 $MH_z$) δ 7.95 (d, 2, J = 8 Hz); 7.32 (d, 2, J = 8); 7.24-7.16 (m, 3); 7.0-6.9 (m, 4); 5.75-5.62 (m, 1); 5.6 (dd, 1, J = 4,8); 5.3-5.2 (m, 2); 5.08 (d, 1, J = 4); 4.6 (s, 2); 4.2-3.9 (m, 4); 3.80 (s, 2); 2.41 (s, 3); IR ($CHCl_3$) 1783, 1741, 1494 $cm^{-1}$: UV (EtOH) $\lambda_{max}$ 234 (ε = 26,000); FABMS: $M^+$ 701, 520.

Example 11

7-β-(2-(thien-2-yl)acetamido)-3-(N-(N-(p-tosyl)-N′-(p-chlorophenyl)ureido)methylene)-3-cephem-4-carboxylic acid

Allyl 7-β-(2-(thien-2-yl)acetamido)-3-(N-(N-(p-tosyl)-N′-(p-chlorophenyl)ureido)methylene)-3-cephem-4-carboxylate (0.10g, 0.143 mmol) was dissolved in methylene chloride (5 ml). The solution was stirred at room temperature under nitrogen while bis(triphenylphosphine)dichloro palladium (II) (10mg) then glacial acetic acid (2 drops) was added. Tributyl tin hydride (0.1 ml) was then added and the resultant reaction mixture was stirred for 30 minutes at room temperature. A mixture of concentrated hydrochloric acid (2 drops) and acetonitrile (2 ml) was added to the reaction mixture and the mixture was then diluted with diethylether, and concentrated to approximately 1/2 volume in vacuo. The concentrate was rediluted with a 1:1 mixture of diethylether and hexane then centrifuged to give a light yellow solid. A portion (60mg) was chromatographed on a preparatory-scale column (reverse phase $C_{18}$, 900 psi, 50 ml/min, 35% acetonitrile/ 0.5% ammonium acetate/water) to give 5 mg of the title product: N.M.R. (DMSO-$d_6$ 300 MHz); δ 9.32 (d, 1, J = 8.3 Hz); 7.81 (d, 2, J = 8.1 Hz); 7.46-7.32 (m, 5); 7.12 (d, 2, 8.6 Hz); 6.90 (s, 2); 5.40 (dd, 1, J = 4.4, 8.1); 4.94 (d, 1, 4.3 Hz); 4.32 (s, 1); 4.07 (d, 1, 10.3); 3.98 (d, 1, 10.3 Hz); 3.74 (s, 2); 3.65 (d, 2, 13.0 Hz); 2.37 (s, 3); IR ($CHCl_3$) 1757, 1736, 1731, 1618 $cm^{-1}$; UV (EtOH), $\lambda_{max}$ 231 (ε = 25,400); FABMS: Cal'd For (M + 1) = 661.06518 Found: 661.06861.

Procedure 15

t-Butyl 7-β-(2-(Phenoxy)acetamido)-3-((p-Nitrophenylcarbonato)methylene-3-cephem-4-carboxylate

t-Butyl 7-β-(2-(phenoxyacetamido)-3-(hydroxymethylene)-3-cephem-4-carboxylate (0.50g, 1.15 mmol) was dissolved in dry tetrahydrofuran (5 ml). The resultant solution was cooled to 0 °C under nitrogen. To the cooled solution was added (p-nitrophenyl)chloroformate (0.35g, 1.72 mmol) then dimethylaminopyridine (DMAP, 2 mg) and finally 2,6-lutidine (0.20 ml)(slowly). The resultant mixture, exhibiting a heavy precipitate, was allowed to stand overnight in an ice bath. Additional THF was added and the precipitate was broken up with a spatula. The mixture was filtered then flash chromatographed over silica gel eluted with a 1:1 mixture of methylene chloride and ethyl acetate to yield 0.40 g of the title product: N.M.R. (CDCl3, DMSO-$d_6$ 300 $MH_z$) δ 8.01 (d, 2, J = 8.9); 7.75 (d, 1, J = 10.2); 7.25-7.00 (m, 3); 6.80-6.62 (m, 4); 5.86 (dd, 1, J = 4.9, 10.2); 5.28 (d, 1, J = 13.3); 4.67 (d, 1, J = 13.3); 4.56 (d, 1, J = 4.6); 4.33 (s, 2); 3.65 (d, 1 J = 18.6); 3.32 (d, 1, J = 18.5) 1.30 (s, 9); IR ($CHCl_3$) 3020, 1807, 1771, 1725, 1696 $cm^{-1}$; UV (EtOH), $\lambda_{max}$ 392 (ε = 1100), 268(ε = 13,500); FABMS (M + H) 602, 546, 363.

Example 12

t-Butyl 7-$\beta$-(2-(phenoxy)acetamido)-3-((Desacetylvinblastinehydrazido)carbonyloxymethylene)-3-cephem-4-carboxylate

The free base of desacetylvinblastine hydrazide sulfate was made by dissolving the sulfate salt (900 mg) in aqueous sodium bicarbonate solution then extracting the solution with methylene chloride. The organic layer was dried over sodium sulfate, filtered and concentrated in vacuo to give a white solid. The free base was dissolved in dry pyridine (6 ml) and added to t-butyl 7-$\beta$-(2-(phenoxy)acetamido)-3-(O-(p-nitrophenyl)carbonato)methylene)-3-cephem-4-carboxylate (0.40g, 0.666 mmol). The addition flask was washed with another portion (2 ml) of pyridine and added to the reaction mixture. A small amount of N,N-Diisopropylethylamine (two drops) was added to the reaction mixture and the resultant mixture was stirred overnight at room temperature under nitrogen. The reaction mixture was diluted with ethyl acetate then concentrated in vacuo. The concentrate was flash chromatographed over silica gel eluted with 90/10 methylene chloride/methanol to give 0.54g of the title product: n.m.r ($CDCl_3$, 300 mHz) $\delta$ 10.0 (s, 1); 8.70 (s, 1); 8.02 (s, 1); 7.93 (d, 1, J = 12); 7.50 (d, 1, J = 12), 7.38-6.90 (m, 10); 6.10 (s) overlapping 6.12 (m, 1); 5.80 (d, 1, J = 12); 5.65 (d, 1, J = 12); 4.38 (s, 2); 4.02 (d, 1, J = 12); 3.75 (s, 3); 3.58 (s, 3); 2.8 (brs, 3); 1.52 (s, 9); 0.90 (brs, 6) remainder are mutiplets from 4.0-1.1 ppm.; IR ($CHCl_3$) 1802, 1721, 1696, 1515 $cm^{-1}$; UV (EtOH), $\lambda_{max}$ 267 ($\epsilon$ = 23,800), 214 ($\epsilon$ = 53,000); FABMS Cal'd (M + H) = 1231.53853 Found: 1231.54230.

Example 13

7-$\beta$-(2-(Phenoxy)acetamido)-3-((Desacetylvinblastinehydrazido)carbonyloxymethylene)-3-cephem-4-carboxylic acid Trifluoroacetic Acid Salt

t-Butyl 7-$\beta$-(2-(phenoxy)acetamido)-3-((desacetylvinblastinehydrazido)carbonyloxymethylene)-3-cephem-4-carboxylate (123 mg, 0.1 mmol) was dissolved in methylene chloride (3 ml). The solution was cooled to 0 °C and triethylsilane (1 ml) then trifluoroacetic acid (1 ml) was added. The resultant solution was stirred for 30 minutes. Additional identical portions of triethylsilane and trifluoroacetic acid were added. The ice bath was removed and the reaction mixture was stirred at room temperature for 3 hours. The mixture was concentrated in vacuo, diluted with cold acetonitrile (3 volumes), and again concentrated in vacuo. Carbon tetrachloride was added and the mixture was again concentrated in vacuo to yield 120 mg of the title product: n.m.r (DMSO-d6 , 300MHz) $\delta$ 9.76 (br s, 1); 9.46 (br s, 1); 9.41 (br s, 1); 8.14 (d, 1, J = 9.3); 7.47 (d, 1, J = 7.9Hz); 7.35-7.21 (m, 4); 7.18 - 6.80 (m, 6); 6.70 (s, 1); 6.30 (s, 1); 6.00 (dd, 1, J = 4.8, 9.5); 5.75 (s, 2); 5.15 (d, 1, J = 12); 4.92 (d, 1, J = 4.8); 4.6 (s. overlapping broad $H_2O$ peak); 3.72 (s, 3), 3.53 (s, 3); 2.79 (brs, 3); 0.82 (t, 3, J = 7); 0.70 (t, 3, J = 7); remainder are multiplets ranging from 4.0 - 1.1; IR (KBr) 1788, 1720, 1685, 1677 $cm^{-1}$; UV (EtOH), $\lambda_{max}$ 311 ($\epsilon$ = 3800), 268 ($\epsilon$ = 18,500), 215 ($\epsilon$ = 45,100); FABMS: Cal'd (M + H) = 1175.47593 Found: 1175.47600.

Procedure 16

Benzhydryl 7-$\beta$-(2-(Thien-2-yl)acetamido)-3-(Hydroxymethyl)-2-Cephem-4-Carboxylate

7-$\beta$-(2-(Thien-2-yl)acetamido)-3-(Hydroxymethyl)-2-cephem-4-carboxylic acid (5.0g, 14.1 mmol), obtained as described in S. Kukolja, J. Med Chem., 1114 (1970), was dissolved in a 1/1 mixture of acetone and acetonitrile. To this solution was added in a dropwise fashion an acetonitrile solution of diphenyldiazomethane (2.7g, 13.9 mmol). The resultant reaction mixture was stirred at room temperature for 1 hour then concentrated in vacuo. Acetonitrile was added to the concentrate and the resultant biege solid (4.4g), the title product, was collected by filtration. N.M.R. (CDCl3, 3OOMHz) $\delta$ 7.40-7.23 (m, 11); 7.02-6.95 (m, 2); 6.88 (s, 1); 6.45 (d, 1, J = 7.6); 6.25 (s, 1); 5.61 (dd, 1, J = 5,8); 5.20 (d, 1, J = 5); 5.15 (s, 1); 4.10 (q, 2, J = 8); 3.82 (s, 2); IR ($CHCl_3$) 1778 $cm^{-1}$; 1743, 1683; EA: Cal'd: C, 62.29, H, 4.64, N, 5.38 Found: C, 62.07, H, 4.73, N, 5.51.

Procedure 17

Benzhydryl 7-β-(2-(Thien-2-yl)acetamido)-3-(Hydroxymethylene)-3-Cephem-1-β-Sulfoxide-4-Carboxylate

Benzyhydryl 7-β-(2-(Thien-2-yl)acetamido)-3-(hydroxymethylene)-2-cephem-4-carboxylate (2.0g, 3.85 mmol) was dissolved in a 4/1 mixture of methylene chloride/isopropanol (125 ml) and the solution was cooled to -10°C. m-Chloroperbenzoic acid (1.21g) was dissolved in isopropanol (25 ml) and added to the previous cooled solution in a dropwise fashion over a period of 30 minutes. The resultant reaction mixture was taken to dryness in vacuo. Benzene was added and the mixture was taken to dryness in vacuo. Ethyl Acetate was added to the solid, and the tan solid was collected by filtration and dried to yield 1.5g of the title product: N.M.R. (300MHz, $CDCl_3$ + DMSO-$d_6$) δ 7.4-7.0 (m, 11); 6.80 (m, 2); 6.70 (s, 1); 5.82 (dd, 1, J=4.6, 9.4); 4.42 (d, 1, J=4.7); 4.37 (d, 1, J=15.0); 4.18 (d, 1, J=15.0); 3.84 (d, 1, J=19.2); 3.67 (s, 2), 3.22 (d, 1, overlaps with $H_2O$); (OH and NH not identified); UV (EtOH), $\lambda_{max}$ 258nm (ε=6540); IR (KBr) 1654 cm-1, 1721, 1755, 1773, 1785; E.A., Cal'd: C, 60.43, H, 4.51, N, 5.22; Found: C, 60.57, H, 4.37, N, 5.01; FABMS: M+H = 537.

Procedure 18

Benzhydryl 7-β-(2-(Thien-2-yl)acetamido-3-(O-(p-Nitrophenyl)carbonato)methylene)-3-Cephem-1-β-Sulfoxide-4-Carboxylate

Benzhydryl 7-β-2-(Thien-2-yl)acetamido-3-(hydroxymethyl)-3-cephem-1-β-sulfoxide-4-carboxylate (1.0g, 1.87 mmol) was partially dissoved in dry THF (20 ml) and the mixture was cooled to 0°C under nitrogen. First (p-nitrophenyl)chloroformate (0.49g, 2.43 mmol), then dimethylaminopyridine (5 mg), and finally dry 2,6 lutidine (0.28 ml) was added to the cooled solution. The resultant solution was stirred for 30 minutes at 0°C then for 1 hour at room temperature. The mixture was filtered, the filtered solid was washed with ethyl acetate, and the filtrate was concentrated in vacuo. The resultant solid was flash chromatographed over silica gel eluted with a 60/40 mixture of methylene chloride/ ethyl acetate to yield 0.32 g of the colorless title product: N.M.R. (300MHz, DMSO-d6) δ 8.49 (d, 1, J=8.3 Hz); 8.27 (d, 2, J=9.2); 7.5-7.2 (m, 13); 6.92 (m, 3); 5.94 (dd, 1, J=4.9, 8.2); 5.25 (d, 1, J=13.0); 4,93 (d, 1, J=4.4); 4.85 (d, 1, J=13.0); 4.04 (d, 1, J=18.7); 3.88 (d, 1, J=15.3); 3.78 (d, 1, J=15.3); 3.66 (d, 1, J=18.7); UV (EtOH) λ 265 nM (ε=3570); IR (KBr) 1720 $cm^{-1}$, 1765, 1658; EA, Cal'd: C, 58.20, H, 3.88, N, 5.99; Found: C, 58.48, H, 4.02, N, 5.90.

Example 14

Benzhydryl 7-β-(2-(Thien-2-yl)acetamido)-3-((Desacetylvinblastinehydrazido)carbonyloxy)methylene)-3-Cephem-1-β-Sulfoxide-4-Carboxylate

The free base of desacetylvinblastinehydrazide sulfate (0.50g) was made as described above in Example 12. The free base was dissolved in dry pyridine (10 ml) and added to benzhydryl 7-β-(2-(thien-2-yl)acetamido)-3-(O-(p-nitrophenyl)carbonato)methylene)-3-cephem-1-β-sulfoxide (0.32 g, 0.456 mmol). The mixture was allowed to stir at room temperature under nitrogen after N,N-diisopropylethylamine (2 drops) was added. The resultant reaction mixture was stirred overnight at room temperature under nitrogen, then diluted with ethyl acetate and concentrated in vacuo. The solid thus obtained was flash chromatographed over silica gel eluted with a 90/10 mixture of methylene chloride/methanol to yield 0.34 g of an off-white solid that was the title product: N.M.R. ($CDCl_3$, 300MHz) δ 9.91 (s, 1); 8.66 (br s, 1); 8.01 (s, 1); 7.53-6.86 (m, 23); 6.55 (s, 1); 6.08 (s, 1); 6.05 (dd, 1, J=4.7, 9.8); 5.8-5.6 (m, 2); 5.3-5.2 (m, 1); 4.85 (brs, 1); 4.47 (d, 1, J=4.4); 3.83 (s, 2); 3.74 (s, 3); 3.57 (s, 3); 0.87 (t, 6, J=7.2) remainder are buried multiplets from 4.1-1.1 ppm.; UV (EtOH), $\lambda_{max}$ 268 (ε=25200); IR (CHCl3) 1690 $cm^{-1}$, 1730, 1803; FABMS Cal'd For (M+H): $C_{71}H_{79}N_8O_{14}S_2$ 1331.51569 Found: 1331.51224.

Example 15

7-β-(2-(Thien-2-yl)acetamido)-3-((Desacetylvinblastinehydrazido)carbonyloxy)methylene)-3-Cephem-1-β-Sulfoxide-4-Carboxylic Acid, Trifluoroacetic Acid Salt

Benzhydryl 7-β-(2-(Thien-2-yl)acetamido)-3-((desacetylvinblastinehydrazido)carbonyloxy)methylene)-3-cephem-1-β-sulfoxide-4-carboxlate (105 mg, 0.00789 mmol) was dissolved in methylene chloride (3 ml).

The solution was cooled to 0 °C under nitrogen, then triethylsilane (0.5 ml) followed by trifluoroacetic acid (0.5 ml) was added. After 15 minutes the solution was diluted with acetonitrile and concentrated in vacuo twice. The solid was dissolved first in chloroform then in diethyl ether, each time followed by concentration in vacuo. The resultant solid was dried under vaccuum at room temperature to yield 100 mg of the title product: N.M.R. (300MHz, DMSO$d_6$) δ 9.76 (s, 1); 9.46 (s, 1); 9.40 (s, 1); 8.43 (d, 1, J=8.1); 7.47 (d, 1, J=8.0), 7.4-6.9 (m, 16); 6.68 (s, 1); 6.28 (s, 1); 5.80 (dd, 1, J=5.8) overlapping with 5.75 (s, 1); 5.16 (d, 1, J=12.7); 4.85 (m, 1); 4.64 (d, 1, J=13.2); 3.88 (s, 2); 3.78 (s, 2); 3.71 (s, 3); 3.53 (s, 3); 2.78 (br s, 3); 0.80 (t, 3, J=7); 0.70 (t, 3, J=7); remainder are multiplets ranging from 4.3-1.0; UV (EtOH), $\lambda_{max}$ 266 nM (ε=19,300), 214 (ε=49,800); FABMS Cal'd For (M+H) $C_{58}H_{69}N_8O_{14}S_2$: 1165.43744, Measured mass: 1165.43290.

Example 16

Benzhydryl 7-β-(2-(Thien-2-yl)acetamido)-3-(((Desacetylvinblastinehydrazido)carbonyloxy)methylene)-3-Cephem-4-Carboxylate

Benzhydryl 7-β-(2-(thien-2-yl)acetamido)-3-(((desacetylvinblastinehydrazido)carbonyloxy)methylene)-3-cephem-1-β-sulfoxide (0.24 g, 0.180 mmol) and stannous chloride (0.10 g, 0.451 mmol) were dissolved in dimethylformamide (DMF, 3 ml). The solution was cooled with an ice bath and acetyl chloride (0.32 ml) was added slowly with a syringe. The resultant reaction mixture was stirred for two minutes with the ice bath in place then for twenty minutes at room temperature. The reaction mixture was poured into cold water (20 ml). The pH of the aqueous layer was taken to 8 with cold aqueous sodium bicarbonate solution. The resultant mixture was extracted with ethyl acetate, and the organic layer was dried over sodium sulfate, filtered, and taken to dryness in vacuo. The solid was flash chromatographed over silica gel eluted with a 90/10 mixture of methylene chloride/methanol to yield 47 mg of the title product: N.M.R. (300MHz, $CDCl_3$) δ 10.30 (s, 1); 9.90 (s, 1); 9.20 (s, 1); 8.70 (s, 1); 8.02 (d, 2, J=8Hz); 7.6-6.8 (m, 25); 6.58 (br s, 1); 6.05 (s, 1); 5.83 (dd, 1, J=5,8); 5.8-5.7 (m, 2); 5.12 (d, 1, J=13); 4.92 (d, 1, J=5); 4.10 (d, 1, J=8); 3.78 (s, 2); 3.72 (s, 3); 3.55 (s, 3); 2.77 (s, 3); 0.88 (t, 6, J=7); Remainder are buried multiplets from 4.0 to 1.1 ppm.; FABMS: Calculated for $C_{71}H_{79}N_8O_{13}S_2$: 1315.52077 Found: 1315.51781

Example 17

7-β-(2-(Thien-2-yl)acetamido)-3-(((desacetylvinblastinehydrazido)carbonyloxy)methylene)-3-Cephem-4-Carboxylic Acid, Trifluoroacetic Acid Salt

Benzhydryl 7-β-(thien-2-yl)acetamido)-3-(((Desacetylvinblastinehydrazido)carbonyloxy)methylene)-3-cephem-4-carboxylate (46 mg, 0.0035 mmol) was dissolved in methylene chloride ( 2 ml). The solution was cooled to 0 °C under nitrogen, then triethylsilane (0.5 ml) followed by trifluoroacetic acid (0.5 ml) were added. The resultant reaction mixture was stirred for 30 minutes under nitrogen a 0 °C, cold acetonitrile was added and the solution was taken to dryness in vacuo. The procedure with acetonitrile was repeated, then the solid was taken up in diethyl ether and taken to dryness in vacuo to leave an off-white solid (42 mg after drying) of the title product: N.M.R. (300MHz, DMSO-$d_6$) δ 9.78 (s, 1): 9.46 (s, 1); 9.35 (s, 1): 9.10 (d, 1, J=8); 7.47 (d, 1, J=8); 7.4-6.8 (m, 16); 6.68 (s, 1); 6.29 (s, 1); 5.75 (s, 1); 5.65 (dd, 1, J=5,8); 5.2-4.9 (m, 2); 3.85 (s, 2), 3.72 (s, 3); 3.50 (s, 3); 2.75 (br s, 3); 0.80 (t, 3, J=7); 0.65 (t, 3, J=7) remainder are mutiplets ranging from 4.2-1.0; FABMS - Calculated for $C_{58}H_{69}N_8O_{13}S_2$: 1149.44252 Found: 1149.44007.

Procedure 19

Allyl 7-β-(2-(Thien-2-yl)acetamido)-3-((1-(t-Butoxycarbonylamino)-2-ethylsulfide)methylene)-3-Cephem-1-β-Sulfoxide-4-Carboxylate

Allyl 7-β-(thien-2-yl)acetamido)-3-((1-(t-butoxycarbonylamino)-2-ethylsulfide)methylene)-3-cephem-4-carboxylate( 1.0g, 1.81 mmol) was dissolved in methylene chloride (20 ml) and the solution was cooled to -78 °C under nitrogen. Meta-chloroperbenzoic acid (0.37g) was dissolved in methylene chloride (20ml) and the solution was added dropwise to the cold cephalosporin solution. The reaction mixture was stirred under nitrogen for 15 minutes, then warmed to 10 °C using an ice/brine bath. After 15 minutes, the reaction mixture taken to dryness in vacuo. The solid was flash chromatographed over silica gel eluted with a 2% methanol in methylene chloride solution to give 0.60 g of the title product: N.M.R. (DMSO-$d_6$, 300MHz) δ

8.40 (d, 1, J = 8.3Hz); 7.32 (m, 1); 6.90 (m, 2); 6.80 (t, 1, J = 4.8); 6.0-5.8 (m, 1); 5.78 (dd, 1, J = 4.8, 8.3); 5.34 (d, 1, J = 17.2); 5.21 (d, 1, J = 10.5); 4.91 (d, 1, J = 4.4); 4.71 (d, 1, J = 5.4); 4.0-3.4 (m, 6); 3.0 (m, 2); 2.5 (m, 2), 1.32 (s, 9); $^{13}C$ (DMSO-$d_6$, 270MHz) 28.19; 30.68; 32.70; 35.67; 46.42; 58.05; 66.06; 66.54: 77.69; 118.76; 122.93; 123.10; 125.04; 126.44; 126.66; 131.78; 136.78; 155.50: 160.65; 164.12; 170.00; UV (EtOH); 271 ($\epsilon$ = 8,330), 234 ($\epsilon$ = 11,000); IR ($CHCl_3$) 1801, 1728, 1700, 1696 $cm^{-1}$; FABMS: M + H = 570.

| | | | | |
|---|---|---|---|---|
| EA: | Cal'd:<br>Found: | C, 50.60,<br>C, 50.83, | H, 5.48,<br>H. 5.55, | N, 7.38;<br>N, 7.32. |

Example 18

Allyl 7-$\beta$-(2-(Thien-2-yl)acetamido)-3-(((1-(Desacetylvinblastine)amino)-2-ethylsulfide)methylene)-3-Cephem-1-$\beta$-Sulfoxide-4-Carboxylate

The procedure of Example 5 was repeated, substituting the cephalosporin-1-$\beta$-sulfoxide starting material of Procedure 19 (0.34 g, 0.598 mmol) for the cephalosporin sulfide of Example 5. Also, the following amounts of other reagents were used:

Desacetylvinblastine hydrazide (0.46 g, 0.60 mmol);
N-Methylmorpholine (0.20 ml, 1.8 mmol);
Sodium Nitrite (0.083g, 1.2 mmol).

The reaction was stirred overnight at room temperature under nitrogen, instead of 30 minutes at 0 °C in Example 5. The product was flash chromatographed over silica gel eluted with a gradient of 95/5 to 90/10 methylene chloride/methanol, to yield 0.30g of the title product as a yellow solid: N.M.R. ($CDCl_3$, 300MHz) $\delta$ 9.48 (s, 1); 8.01 (s, 1); 7.5-6.9 (m, 12); 6.52 (s, 1); 6.02 (s, 1); 5.95 (dd, 1, J = 4.2, 9.5Hz); overlapping 5.92 (m, 1,); 5.70 (s, 2); 5.35 (d, 1, J = 17.1); 5.25 (d, 1, J = 7.5); 4.73 (br s, 2); 4.62 (d, 1, J = 4.2); 3.71 (s, 2); 3.67 (s, 3); 3.66 (s, 3); 3.55 (s, 3); 2.73 (s, 3); 0.88 (t, 3, J = 7); overlapping 0.85 (t, 3, J = 7); remainder are various multiplets from 4.1-1.1 ppm.; UV (EtOH), $\lambda_{max}$ 268 ($\epsilon$ = 22,600), 214 ($\epsilon$ = 57,000); IR (CHCl3) 1799, 1730, 1669, 1615$cm^{-1}$; FABMS: Cal'd For $C_{62}H_{76}N_7O_{12}S_3$: 1206.47139 Measured mass: 1206.47138.

Example 19

7-$\beta$-(2-(Thien-2-yl)acetamido)-3-(((1-(Desacetylvinblastine)amino)-2-Ethylsulfide)methylene)-3-Cephem-1-$\beta$-Sulfoxide-4-Carboxylic Acid

Allyl 7-$\beta$-(2-(Thien-2-yl)acetamido)-3-(((1-(Desacetylvinblastine)amino)-2-ethylsulfide)methylene)-3-cephem-1-$\beta$-sulfoxide-4-carboxylate (0.30g, 0.249 mmol) was dissolved in methylene chloride (3 ml). The solution was added to a methylene chloride (1 ml) solution of tetrakis[triphenyl-phosphine] palladium (0) (11.4 mg, 0.0099 mmol) and triphenylphosphine (2.6 mg, 0.0099 mmol). The resultant reaction mixture was stirred at room temperature under nitrogen for 10 minutes then triethylsilane (4 drops) and glacial acetic acid (2 drops) were added. The reaction mixture was then stirred overnight at room temperature under nitrogen. An identical portion of the palladium, phosphine, silane and acetic acid reagents were added to the reaction mixture and the mixture was stirred for another 3 hours. Diethyl ether was added and the resultant solid was collected using a centrifuge. The collected off-white solid was dried under vacuum at room temperature to yield 183 mg of the title product. A portion (100 mg) of this material was purified by HPLC ($C_{18}$ 50 x 350 column, 1000 psi, 50 ml/min, eluted with 15% acetonitrile/ 1% formic acid /84% water for 10 min, a linear gradient over 18 min to 30% acetonitrile/69% water/ 1% formic acid until completion.) N.M.R. (DMSO-$d_6$, 300MHz) $\delta$ 9.40 (s, 1); 8.32 (d, 1, J = 8Hz); 8.0-7.6 (m, 4); 7.4-7.2 (m, 4); 7.0-6.8 (m, 4); 6.39 (s, 1); 6.15 (s, 1); 5.75-5.50 (m, 3); 4.81 (d, 1, J = 4); 3.75 (s, 2); 3.65 (s, 3); 3.50 (s, 3); 2.62 (s, 3); 0.75 (t, 3, J = 7); 0.65 (t, 3, J = 7); remainder are multiplets ranging from 4.0-1.1; UV (EtOH), $\lambda_{max}$ 266 ($\epsilon$ = 25,600), 215 ($\epsilon$ = 59,200); IR (CHCl3) 1792, 1623, 1616, 1602$cm^{-1}$; FABMS: Cal'd For $C_{59}H_{72}N_7O_{12}S_3$: 1166.44009 Measured Mass: 1166.43436.

Procedure 20

Benzhydryl 7-β-(2-(Thien-2-yl)acetamido)-3-(((O-(p-Nitrophenyl)carbonato)methylene)-2-Cephem-4-Carboxylate

Benzhydryl 7-β-(2-(thien-2-yl)acetamido)-3-(hydroxymethyl)-2-cephem-4-carboxylate (3.0g, 5.77 mmol) was dissolved in dry tetrahydrofuran (THF, 5 ml). The solution was cooled to 0 °C under nitrogen then (p-nitrophenyl)chloroformate (1.74g, 8.65 mmol) and dimethylaminopyridine (2 mg) were added. To this reaction mixture dry 2,6-lutidine (1.0 mL, 8.65 mmol) was added in a dropwise fashion then the reaction was stirred overnight under nitrogen gradually allowing to warm to 23 °C. The reaction mixture was gravity-filtered, and the filtrate was concentrated in vacuo. The concentrate was flash chromotagraphed over silica gel eluted with 95/5 methylene chloride/ethyl acetate. The chromatography yielded 2.9g of a white foam of the title product: N.M.R. ($CDCl_3$, 300MHz); 8.28 (d, 2, J = 9.1); 7.42-7.28 (m, 13); 7.05-7.00 (m, 2); 6.92 (s, 1); 6.55 (s, 1); 6.35 (d, 1, J = 8.7); 5.65 (dd, 1, J = 4.0, 8.7Hz); 5.24 (d, 1, J = 4.0); 5.21 (S, 1); 4.82 (d, 1, J = 12.4); 4.72 (d, 1, J = 12.5); 3.88 (s, 2); IR ($CHCl_3$) 1777, 1749, 1686, 1528 $cm^{-1}$; UV (EtOH), $\lambda_{max}$ 244 ($\epsilon$ = 16,800); FABMS: Cal'd (M + H): 686.12669 Found: 686.12451.

Example 20

Benzhydryl 7-β-(2-(Thien-2-yl)acetamido)-3-((Desacetylcolchicine-7-amino)carbonyloxy)methylene)-3-Cephem-4-Carboxylate

Desacetylcolchicine (70 mg, 0.196 mmol) was made according to the procedure described in Raffauf, J. Amer. Chem. Soc,, p5292 (1953). The desacetylcolchicine thus prepared was dissolved in dry pyridine under nitrogen at room temperature and the solution was added to benzhydryl 7-β-(2-(thien-2-yl)acetamido)-3-((O-(p-nitrophenyl)carbonato)methylene)-2-cephem-4-carboxylate (130 mg, 0.196 mmol), followed by the addition of N,N-diisopropylethylamine (1 drop). The reaction mixture was stirred overnight at room temperature under nitrogen. The reaction mixture was diluted with ethyl acetate then concentrated in vacuo. The dilution/concentration was repeated and the concentrate was flash chromatographed over silica gel eluted with 10% methanol in methylene chloride. The chromatography yielded 0.17 g of a yellow oil of the title products: N.M.R. (CDCl3, 300 MHz), 1:1 mixture of Δ-2 :Δ-3); 8.78 (br s, 1); 8.10 (d, 1, J = 6); 7.6-7.1 (m, 13); 7.0-6.8 (m, 4); 6.52 (s, 1); 6.38 (s, 1); 5.8 (dd, 1, J = 4,8); 5.7 (d, 1, J = 6); 5.55 (dd, 1, J = 4,8); 5.30 (d, 1, J = 6); 5.16 (d, 1, J = 4); 5.10 (s, 1); 4.95 (d, 1, J = 12); 4.90 (d, 1, J = 4); 4.65 (d, 1, J = 12); 4.4-4.2 (m, 1); 3.92 (s, 3); 3.90 (s, 3); 3.88 (s, 3); 3.60 (s, 3); 3.42 (d, 1, J = 12); 3.23 (d, 1, J = 12); 2.58-2.45 (m, 1); 2.40-2.18 (m, 2); UV (EtOH) 341 ($\epsilon$ = 81,600), 219 ($\epsilon$ = 214,000), 202 ($\epsilon$ = 311,000); FABMS: (M + H) 904.

Example 21

Benzyhydryl 7-β-(2-(Thien-2-yl)acetamido)-3-(((Desacetylcolchicine-7-Amino)carbonyloxy)methylene)-3-Cephem-1-β-Sulfoxide-4-Carboxylate

Benzhydryl 7-β-(2-(Thien-2-yl)acetamido)-3-(((desacetylcolchicine-7-amino)carbonyloxy)methylene)-2,3-cephem-4-carboxylate (0.17g, 0.188 mmol) was dissolved in methylene chloride and the solution was cooled to 0 °C under nitrogen. meta-Chloroperbenzoic acid (0.038g, 0.188 mmol) was dissolved in methylene chloride (2 ml) and this solution was slowly added to the cephalosporin solution. The reaction mixture was stirred at 0 °C under nitrogen for one hour. The reaction mixture was taken to dryness in vacuo then the solid was flash chromatographed over silica gel eluted with a 90/10 methylene chloride/methanol mixture to yield 70 mg of the title product: N.M.R. ($CDCl_3$, 300 MHz); 8.08 (d, 1, J = 6.2Hz); 7.48-7.10 (m, 13); 7.0-6.8 (m, 4); 6.51 (s, 1), 6.0 (m, 1); 5.15 (d, 1, J = 13); 4.62 (d, 1, J = 13); 4.45 (d, 1, J = 4); 4.32 (m, 1); 3.95 (s, 2); 3.92 (s, 3); 3.90 (s, 3); 3.82 (s, 3); 3.60 (s, 3); overlapping 3.6 (d, 1, J = 13); 3.15 (d, 1, J = 13); 2.50-2.18 (m, 3); 1.78 (m, 1); UV (EtOH), $\lambda_{max}$ 346 ($\epsilon$ = 14,500); FABMS: Cal'd (M + H): 920.25228 Found: 920.25448.

Example 22

7-β-(2-(Thien-2-yl)acetamido)-3-(((Desacetylcolchicine-7-Amino)carbonyloxy)methylene)-3-Cephem-1-β-Sulfoxide-4-Carboxylic Acid

Benzhydryl 7-β-(2-(Thien-2-yl)acetamido)-3-(((desacetylcolchicine-7-amino)carbonyloxy)methylene)-3-cephem-1-β-sulfoxide-4-carboxylate (70 mg) was dissolved in methylene chloride (3 ml) and cooled to 0°C under nitrogen. To this cooled solution was added triethyl-silane (1 ml) then trifluoroacetic acid (1 ml). The resultant reaction mixture was stirred at 0°C under nitrogen for 30 minutes. The reaction mixture was diluted with cold acetonitrile and taken to dryness in vacuo. Cold acetonitrile was added to the residue and the resultant solution was again taken to dryness in vacuo. The residue was taken up in diethyl ether and the solution was taken to dryness in vacuo giving a yellow solid. After drying, the preceeding procedure afforded 70 mg of the title product: N.M.R. (DMSO-d6, 300 MHz); δ 8.37 (d, 1, J=8.4); 8.13 (d, 1, 7.9); 8.06 (d, 1, J=9.1); 7.38-6.80 (m, 6); 6.70 (s, 1); 5.74 (dd, 1, J=4,8); 5.07 (d, 1, J=13.2); 4.82 (d, 1. J=4): 4.42 (d, 1, J=13.2); 4.05 (m, 1); 3.88 (s, 2); 3.82 (s, 3); 3.77 (s, 3); 3.73 (s, 3); 3.46 (s, 3); with 2 buried protons 2.58-2.40 (m, 1); 2.20-1.90 (m, 2); 1.83-1.70 (m, 1); IR (KBr) 1788, 1721, 1693, 1592 $cm^{-1}$; UV (EtOH), $\lambda_{max}$ 345 (ε = 14,200), 240 (ε = 33,000); FABMS: Cal'd (M+H): 754.17403 Found: 754.17598.

Procedure 21

Rat Hematology Protocol

The purpose of the following procedure was to compare the toxicity of the Substrate-Cytotoxic Agent to the toxicity of Cytotoxic Agent alone.

1. Day 0: 25 male Fischer 344 rats (~100g) are pre-bled (eyebleed) and injected i.v. as follows:

Group A: 5 rats + vehicle control: 0.5 ml 10 mM HEPES, 150 NaCl, pH 7.1 buffer containing 10% ethanol.

Group B: 5 rats + 1 mg/kg 4 desacetylvinblastine-3-carboxhydrazide (DAVLBHYD). Dissolve 1 mg DAVLBHYD in 0.5 ml absolute ethanol and add 4.5 ml HEPES-NaCl buffer. Inject each rat with 0.5 ml.

Group C: 5 rats + 1 mg/kg ("LY262758") (7-β-2-(phenoxyacetamido)-3-(((desacetylvinblastine)-aminocarboxy)methylene)-3-cephem-1-β-sulfoxide-4-carboxybiacid, trifluoroacetic acid salt) (vinca content), (58% vinca). Dissolve 2 mg as above and inject 0.5 ml.

Group D: 5 rats + 1mg/kg ("LY266494"), (7-β-(2-(thien-2-yl)acetamido-3-((-(desacetylvinblastinehydrazido)carbonyloxy)methylene)-3-cephem-4-carboxylic acid, hydrochloride salt (55.8% vinca). Dissolve 2 mg and inject 0.5 ml.

Group E: 5 rats + 1 mg/kg ("LY266070") 7-β-(2-(thien-2-yl)acetamido-3-((-(desacetylvinblastinehydrazido)carbonyloxy)methylene)-3-cephem-1-β-sulfoxide-4-carboxylic acid, trifluoroacetic acid salt. Dissovle 2 mg and inject 0.5 ml.

All animals were indentifed by ear punch and were bled on days 3 and 7 post treatment for white blood cell (WBC) determination. The raw data are on the following page. The DAVLBHYD treated animals exhibited a significant WBC suppression on day 3 compared to all other groups. The WBC values of the groups were indistinguishable at day 7 (and lower than the pre-bleed which may have been due to using a different instrument for the WBC determination).

RAT HEMATOLOGY DATA

Values x 1000 = WBC's

| Group | | Day 0 | Day 3 | Day 7 |
|---|---|---|---|---|
| Control | rat #1 | 13.2 | 14.2 | 6.0 |
| | rat #2 | 12.4 | 16.4 | 9.3 |
| | rat #3 | 13.1 | 13.7 | 6.8 |
| | rat #4 | 10.4 | 10.3 | 10.1 |
| | rat #5 | 10.1 | 13.1 | |
| | Mean | 11.8 | 13.5 | 8.1 |
| | ±SD | 1.5 | 2.2 | 2.0 |
| DAVLB-Hyd | rat #1 | 17.4 | ** | 5.0 |
| | rat #2 | 10.9 | 4.4 | 2.8 |
| | rat #3 | 10.2 | 9.0 | 19.8 |
| | rat #4 | 12.7 | 5.4 | ** |
| | rat #5 | 17.6 | 10.6 | ** |
| | Mean | 13.8 | 7.4 | 9.2 |
| | ±SD | 3.5 | 2.9 | 9.2 |
| LY262758 | rat #1 | 13.3 | 13.3 | 14.0 |
| | rat #2 | 14.3 | 14.6 | 13.0 |
| | rat #3 | 9.4 | 15.0 | 12.5 |
| | rat #4 | 10.2 | 16.3 | 9.0 |
| | rat #5 | 19.7 | 14.7 | 7.7 |
| | Mean | 13.4 | 14.8 | 11.2 |
| | ±SD | 4.1 | 1.1 | 2.7 |
| LY266494 | rat #1 | 15.6 | 19.2 | 8.3 |
| | rat #2 | 16.5 | 16.2 | 6.8 |
| | rat #3 | 14.3 | 14.3 | 11.4 |
| | rat #4 | ** | 13.3 | 13.2 |
| | rat #5 | 16.0 | 12.1 | 20.1 |
| | Mean | 15.6 | 15.0 | 12.0 |
| | ±SD | 0.9 | 2.8 | 5.2 |
| LY266070 | rat #1 | 14.1 | 15.9 | 7.0 |
| | rat #2 | 15.0 | 12.8 | 14.5 |
| | rat #3 | 9.9 | 15.1 | 9.6 |
| | rat #4 | 9.9 | 14.5 | 9.6 |
| | rat #5 | 16.2 | 11.3 | 7.1 |
| | Mean | 13.0 | 13.9 | 9.6 |
| | ±SD | 2.9 | 1.9 | 3.0 |

**Sample clotted--could not determine WBC count.

## Claims

**Claims for the following Contracting States : AT, BE, CH, DE, FR, GB, IT, LI, NL, SE**

**1.** A compound of Formula (II):

Substrate-Cytotoxic Agent (II)

wherein the Substrate denotes a molecule that is a substrate for a a beta-lactamase enzyme, wherein said enzyme cleaves, and thus separates, the substrate from the Cytotoxic Agent.

**2.** A compound as claimed in Claim 1, wherein the Substrate is :
a cephalosporin of the formula:

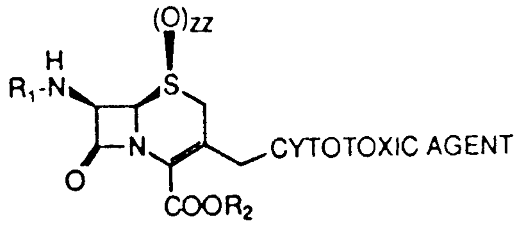

wherein ZZ is 0 or 1, $R_1$ is an acyl group derived from a $C_1$ to $C_{30}$ alkyl group, and $R_2$ is hydrogen, an organic or inorganic cation , a carboxy - protecting group, or a non-toxic, metabolically-labile ester-forming group.

**3.** A compound of Claim 1, wherein $R_1$ is an acyl group of the formula $-COR_3$, wherein $R_3$ is:
i) $C_1$ to $C_6$ alkyl, $C_1$ to $C_6$ substituted alkyl, $C_1$ to $C_4$ alkoxy, $C_1$ to $C_4$ alkylthio, or a group of the formulae:

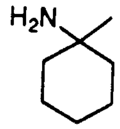

;

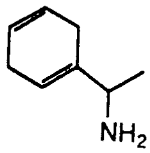

;

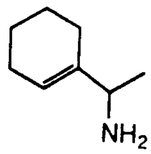

;

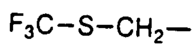

;

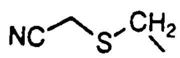

;

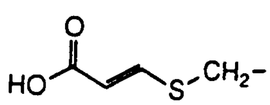

; or

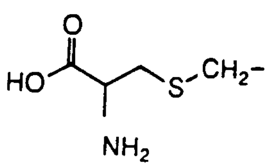

;

or a protected amino and/or protected carboxy derivative thereof;

ii) a group of the formulae:

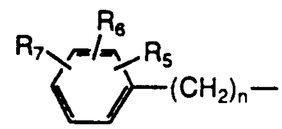

;

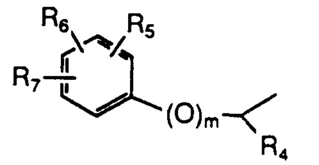

;

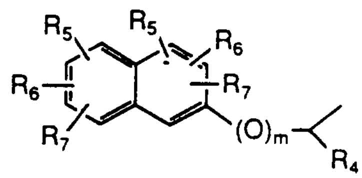

;

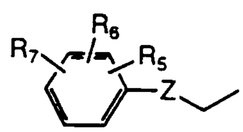

;

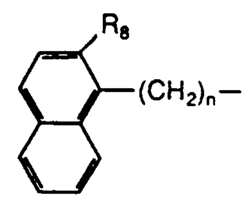

;

wherein each of $R_5$, $R_6$ and $R_7$ is, independently, hydrogen, halo, hydroxy, protected hydroxy, nitro, amino, protected amino, an amine salt, cyano, trifluoromethyl, amino-methyl, protected aminomethyl, N-(methyl- or ethyl-sulfonyl)amino, $C_1$ to $C_6$ alkyl or $C_1$ to $C_4$ alkoxy, $R_4$ is hydroxy, protected hydroxy, formyloxy, amino, protected amino, an amine salt, carboxy, a carboxylate salt, protected carboxy, phenyl carboxylate, (5-indanyl)-carboxylate, sulfonic acid, a sulfonate salt, azido, halo or $C_1$ to $C_6$ alkyl; $R_8$ is $C_1$ to $C_4$ alkoxy; Z is oxygen or sulfur; n is 0, 1, 2 or 3; and m is 0 or 1;

iii) a group of the formula:

$R_9-(CH_2)_n-$ ;

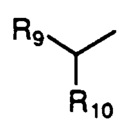

;

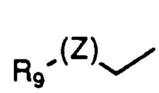

or

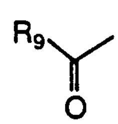

wherein $R_9$ is a heterocyclic ring; $R_{10}$ is hydroxy, protected hydroxy, formyloxy, amino, protected amino, an amine salt, carboxy, a carboxylate salt, protected carboxy, phenyl carboxylate, (5-indanyl)-carboxylate, sulfonic acid, a sulfonate salt, azido, halo or $C_1$ to $C_6$ alkyl; n is 0, 1, 2, or 3; and Z is oxygen or sulfur;

**4.** A compound of Claim 3, wherein the Cytotoxic Agent is:

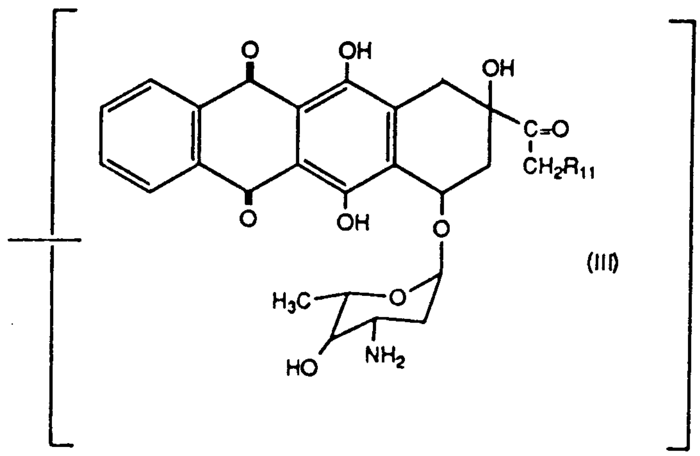

(III)

wherein $R_{11}$ is hydrogen or hydroxy;

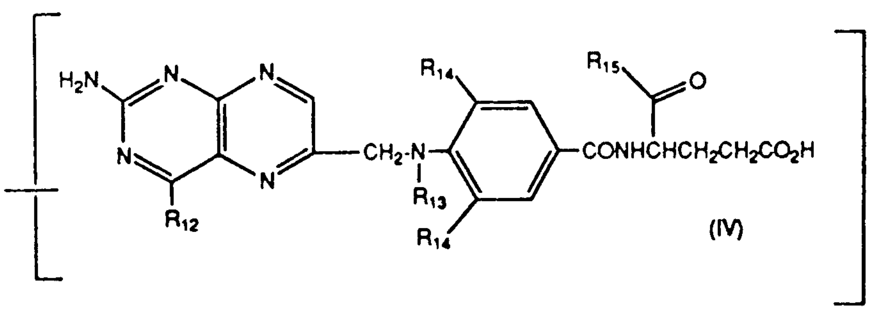

(IV)

wherein $R_{12}$ is amino or hydroxy;
$R_{13}$ is hydrogen or methyl;
$R_{14}$ is hydrogen, fluoro, chloro, bromo, iodo;
$R_{15}$ is hydroxy or a moiety which completes a salt of the carboxylic acid;

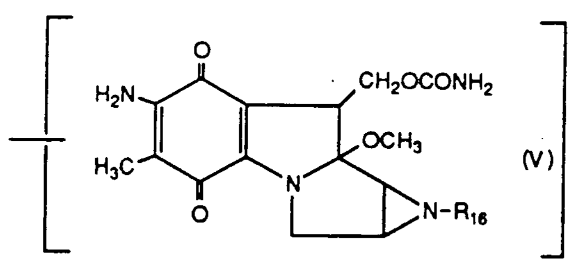

(V)

wherein $R_{16}$ is hydrogen or methyl;

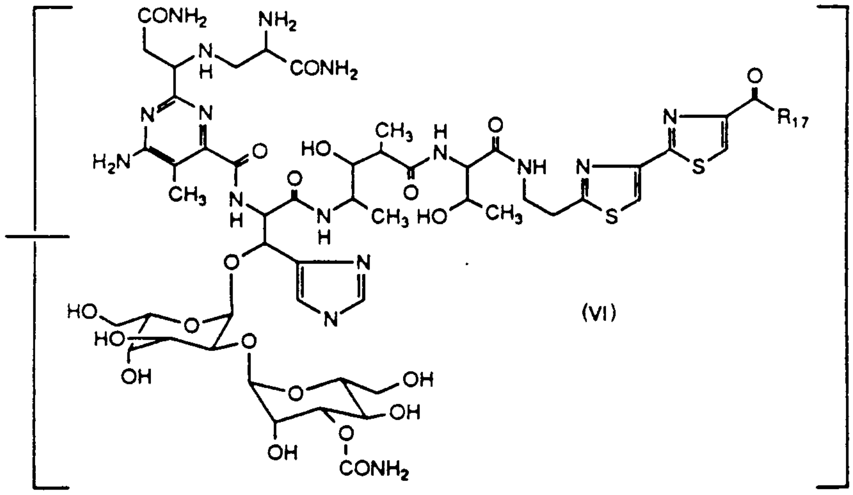

(VI)

wherein $R_{17}$ is amino, $C_1$-$C_3$ alkylamino, di-($C_1$-$C_3$-alkyl)amino, or $C_4$-$C_6$ polymethylene amino;

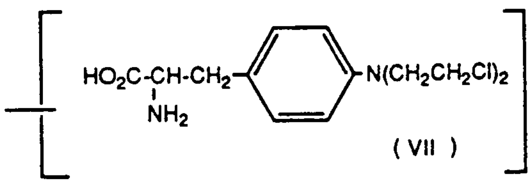

(VII)

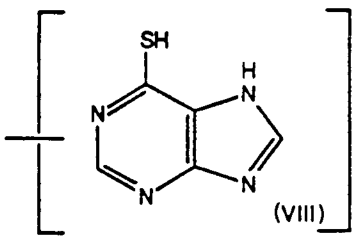

(VIII)

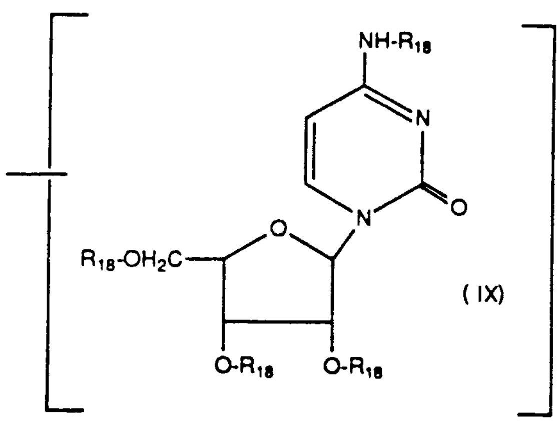

(IX)

wherein one of the $R_{18}$ moieties is a bond and the others are hydrogen;

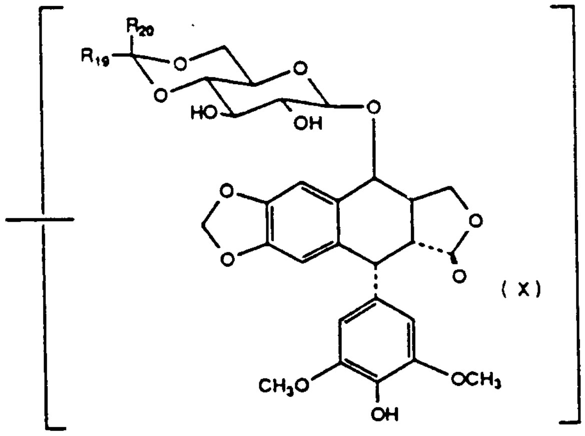

( X )

wherein $R_{19}$ is hydrogen or methyl;
$R_{20}$ is methyl or thienyl;

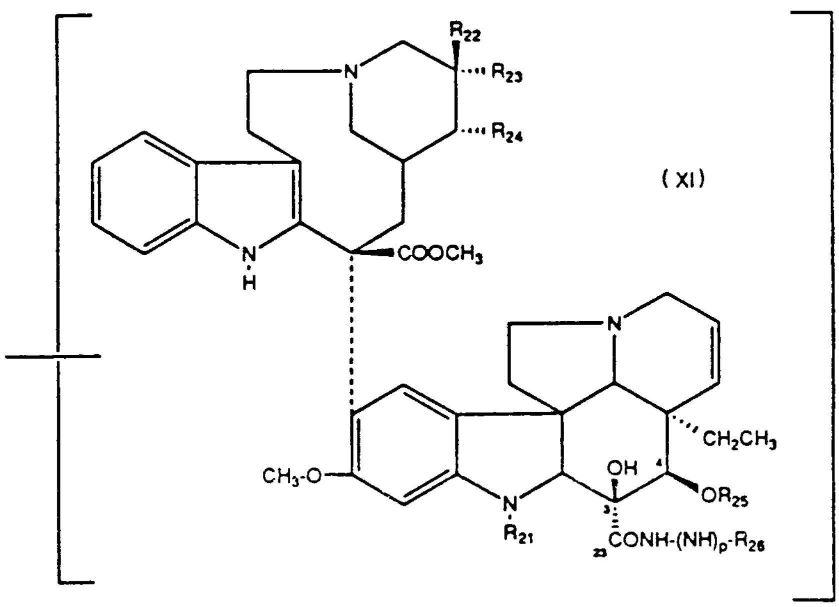

( XI )

wherein $R_{21}$ is H, $CH_3$ or CHO; when $R_{23}$ and $R_{24}$ are taken singly, $R_{24}$ is H, and one of $R_{22}$ and $R_{23}$ is ethyl and the other is H or OH; when $R_{23}$ and $R_{24}$ are taken together with the carbons to which they are attached, they form an oxirane ring in which case $R_{22}$ is ethyl; $R_{25}$ is hydrogen, ($C_1$-$C_3$ alkyl)-CO-, or chloro-substituted ($C_1$-$C_3$ alkyl)-CO;

p is 0 or 1;

$R_{26}$ is a bond; -($C_2$-$C_4$ alkyl)-X, or a group that requires that p is 1 and which is in turn bonded to a carbonyloxy group;

X is -O-, -S-, or -NH-;

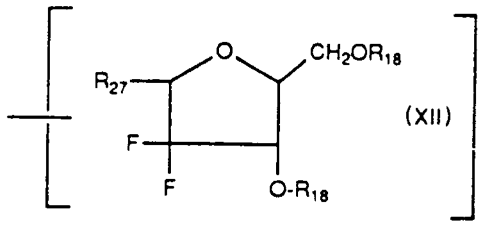

(XII)

wherein $R_{27}$ is a base of one of the formulae:

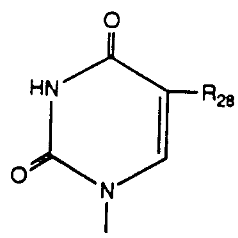

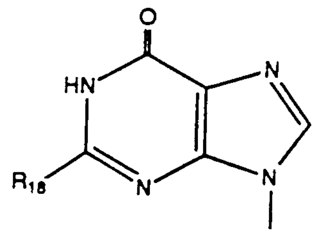

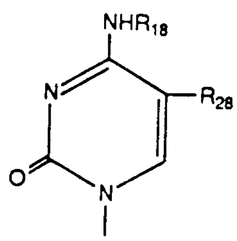

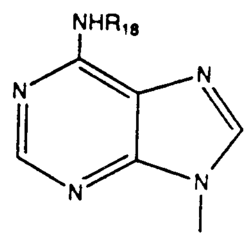

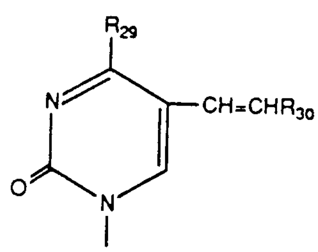

wherein $R_{28}$ is hydrogen, methyl, bromo, fluoro, chloro, or iodo;
$R_{29}$ is $-OR_{18}$ or $-NHR_{18}$;
$R_{30}$ is hydrogen, bromo, chloro, or iodo;

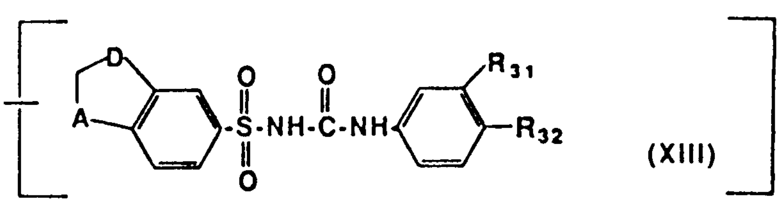

(XIII)

wherein A is -O-, $-NCH_3-$, $-CH_2-$, $-CH_2CH_2-$, or $-CH_2O-$;
D is $-CH_2-$ or -O-;
$R_{31}$ is hydrogen or halo;
$R_{32}$ is halo or trifluoromethyl;

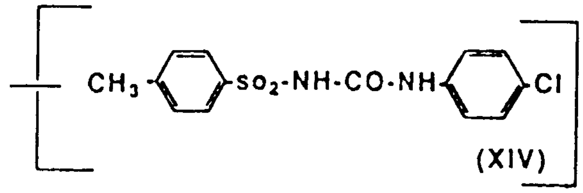

(XIV)

or, 5-uracil.

**5.** A compound of Claim 4, wherein $R_1$ is acetyl, propionyl, butanoyl, isopropionyl, pivaloyl, methoxymethyl, phenylacetyl, phenoxyacetyl, 2-(aminomethyl)-phenylacetyl, 2-phenyl-2-hydroxyacetyl, 2-phenyl-2-(sodium sulfonato)-acetyl, 2-phenyl-2-2-carboxyacetyl, 2-(4-hydroxyphenyl)-2-carboxyacetyl, 2-phenyl-2-aminoacetyl, 2-(4-hydroxyphenyl)-2-aminoacetal, 2-(3-(N-(methylsulfonylamino))phenyl)-2-aminoacetyl, 2-phenyl-2-(5-indanyl carboxylate)acetyl, 2-phenyl-2-(phenyl carboxylate)acetyl, 2-phenyl-2-azidoacetyl, 2-phenoxypropionyl, 2,5-dimethoxybenzoyl, 2-(formyloxy)-2-phenylacetyl, 2-(2-ethoxynaphth-1-yl)acetyl, 2-(naphth-1-yl)-2-aminoacetyl, 2-(naphth-2-yl)-2-aminoacetyl, 2-(2,5-dichlorophenylthio)acetyl, 2-(3,4-dichlorophenylthio)acetyl, 2-(1-tetrazolyl)acetyl, 2-(N-(3,5-dichloropyrid-4-oxyl))acetyl, 2-(2-aminothiazol-4-yl)-acetyl, 2-(2-thienyl)acetyl, 2-(4-pyridylthio)acetyl, 2-(N-methyl-4-pyridiniumthio)-acetyl, 2-(2-amino-4-phenylthiazol-5-yl)acetyl, 2-(3-hydroxy-4-carboxyisothiazol-5-ylthio)acetyl, 3-phenyl-5-methylisoxazolyl-3-formyl, 3-(2-chlorophenyl)-5-methylisoxazolyl-3-formyl, 3-(2,5-dichlorophenyl)-5-methylisoxazolyl-3-formyl, 3-(2-fluoro-5-chlorophenyl)-5-methylisoxazolyl-3-formyl, 2-(2-thienyl)-2-aminoacetyl, 2-(2-thienyl)-2-(sodium carboxylate)acetyl, 2-(N-(4-pyridinium))acetyl, 2-(2-benzothienyl)-acetyl, 2-(3-benzothienyl)acetyl, 2-(2-benzofuryl)acetyl, and 2-(3-benzofuryl)acetyl.

**6.** A compound as claimed in Claim 5, wherein the Cytotoxic Agent is methotrexate, 5-fluorouracil, desacetylvinblastine hydrazide, desacetylvinblastine aminoethanethiol, a desacetylvinblastine hydrazodecarboxy moiety, a 7-(carboxyamino)desacetylcolchicine moiety, N-(p-tosyl)-N'-(p-chlorophenyl)urea, or N-(((4-chloro-phenyl)amino)-carbonyl)-2,3-dihydro-1H-indene-5-sulfonamide.

**7.** A compound as claimed in Claim 6, wherein $R_1$ is 2-(thien-2-yl)acetyl and ZZ is 0.

**8.** A compound as claimed in Claim 7, wherein the Cytotoxic Agent is methotrexate.

**9.** A compound as claimed in Claim 8, wherein the methotrexate is bonded through its carboxy group to the cephalosporin Substrate.

**10.** A compound as claimed in Claim 7, wherein the Cytotoxic Agent is 5-fluorouracil.

**11.** A compound as claimed in Claim 10, wherein the 5-fluorouracil is bonded through its $N_1$ nitrogen to the cephalosporin Substrate.

**12.** A compound as claimed in Claim 7, wherein the Cytotoxic Agent is desacetylvinblastine hydrazide.

**13.** A compound of Claim 12, wherein the desacetylvinblastine hydrazide is bonded through the otherwise unbound nitrogen of its hydrazido group to the cephalosporin Substrate.

**14.** A compound as claimed in Claim 7, wherein the Cytotoxic Agent is desacetylvinblastine aminoethanethiol.

**15.** A compound as claimed in Claim 14, wherein the desacetylvinblastine aminoethanethiol is bonded through the thiol group of its aminoethanethiol moiety to the cephalosporin Substrate.

**16.** A compound as claimed in Claim 7, wherein the Cytotoxic Agent is a desacetylvinblastine hydrazidocarboxy moiety.

**17.** A compound as claimed in Claim 16, wherein the desacetylvinblastine moiety is bonded through an oxygen atom of the hydrazidocarboxy group to the cephalosporin Substrate.

**18.** A compound as claimed in Claim 7, wherein the Cytotoxic Agent is N-(p-tosyl)-N'-(p-chlorophenyl)urea.

**19.** A compound as claimed in Claim 18, wherein N-(p-tosyl)-N'-(p-chlorophenyl)urea is bonded through the p-tosyl nitrogen to the cephalosporin Substrate.

**20.** A compound as claimed in Claim 6, wherein $R_1$ is 2-(thien-2-yl)acetyl and ZZ is 1.

**21.** A compound as claimed in Claim 20, wherein the Cytotoxic Agent is a 7-(carboxyamino)-desacetylcolchicine moiety.

**22.** A compound as claimed in Claim 21, wherein the 7-(carboxyamino)desacetylcolchicine moiety is bonded through an oxygen atom of the 7-(carboxyamino) group to the cephalosporin Substrate.

**23.** A compound as claimed in Claim 20, wherein the Cytotoxic Agent is desacetylvinblastine aminoethanethiol.

**24.** A compound as claimed in Claim 23, wherein desacetylvinblastine aminoethanethiol is bonded through the thiol group of the aminoethanethiol group to the cephalosporin Substrate.

**25.** A compound as claimed in Claim 20, wherein the Cytotoxic Agent is the desacetylvinblastine hydrazidocarboxy moiety.

**26.** A compound as claimed in Claim 25, wherein the desacetylvinblastine hydrazidocarboxy moiety is bonded through an oxygen atom of the hydrazidocarboxy group to the cephalosporin moiety.

**27.** A compound as claimed in Claim 6, wherein $R_1$ is phenoxyacetyl and ZZ is 1.

**28.** A compound as claimed in Claim 27, wherein the Cytotoxic Agent is the desacetylvinblastine hydrazidocarboxy moiety.

**29.** A compound as claimed in Claim 28, wherein the desacetylvinblastine hydrazidocarboxy moiety is bonded through an oxygen atom of the hydrazidocarboxy group to the cephalosporin Substrate.

**30.** An antibody-enzyme conjugate compound of Formula (I):

Antibody-Enzyme (I)

wherein:

the Enzyme reacts with a compound of any one of Claims 1 to 29, and causes the separation of the Cytotoxic Agent from the Substrate; and

the Antibody has specificity for an antigen expressed by the target tissue, and

wherein the Enzyme is beta-lactamase.

**31.** A compound as claimed in Claim 30, wherein the Antibody complexes with antigens expressed solely, or in supernatural abundance, by malignant tumor cells.

**32.** A compound as claimed in Claim 31, wherein the Antibody has specificity for carcinoembryonic antigen.

**33.** A compound as claimed in Claim 32, wherein the Antibody is ZCE 025.

**34.** A compound as claimed in Claim 32, wherein the Antibody is CEM 231.6.7.

**35.** A compound as claimed in Claim 31, wherein the Antibody is T101.

**36.** A compound as claimed in Claim 31, wherein the Antibody has specificity for the KS1/4 antigen.

**37.** A compound as claimed in any one of Claims 30 to 36 wherein the Antibody is an F(ab') fragment.

**38.** A pharmaceutical formulation which comprises as an ingredient, a compound of any one of Claims 1 to 29, associated with a pharmaceutically acceptable carrier, diluent or excipient therefor.

**39.** A pharmaceutical formulation which comprises as an ingredient, a compound of any one of Claims 30 to 37, associated with a pharmaceutically acceptable carrier, diluent or excipient therefor.

**40.** A kit adapted for the treatment of a neoplastic disease which comprises an Antibody-Enzyme Conjugate of Formula (I), as defined in any one of Claims 30 to 37 and a Substrate-Cytotoxic Agent compound of Formula (II), as defined in any one of Claims 1 to 29.

**41.** A compound of Formula (I), as defined in any one of Claims 30 to 37, for use in the treatment of neoplastic disease.

**42.** A compound of Formula (II), as defined in any one of Claims 1 to 29, for use in the treatment of neoplastic disease.

**Claims for the following Contracting State : ES**

**1.** A process for preparing a compound of formula (II):

Substrate-Cytotoxic Agent (II)

wherein the Substrate denotes a molecule that is a substrate for an enzyme, wherein said enzyme cleaves, and thus separates, the substrate from the Cytotoxic Agent, comprising the steps of;

a) protecting any carboxy, amino, thiol or hydroxy groups on the Substrate or Cytotoxic Agent.

b) displacing a leaving group on either the Substrate or the Cytotoxic Agent with a nucleophilic group of the other of the Substrate or the Cytotoxic Agent under $S_n1$ or $S_n2$ conditions, and

c) deprotecting the protected groups,

wherein the Substrate is a molecule that is a substrate for a beta-lactamase.

**2.** A process as claimed in Claim 1, wherein the Substrate is:

a cephalosporin of the formula:

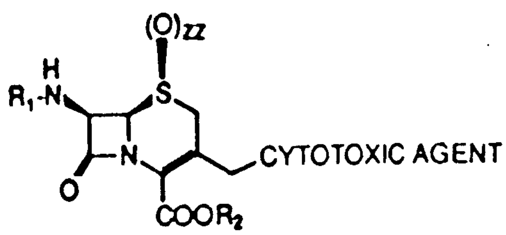

wherein ZZ is 0 or 1, $R_1$ is an acyl group derived from a $C_1$ to $C_{30}$ alkyl group, and $R_2$ is hydrogen, an organic or inorganic cation, a carboxy-protecting group, or a non-toxic, metabolically-labile ester-forming group,

**3.** A process of Claim 2, wherein $R_1$ is an acyl group of the formula $-COR_3$, wherein $R_3$ is:

i) $C_1$ to $C_6$ alkyl, $C_1$ to $C_6$ substituted alkyl, $C_1$ to $C_4$ alkoxy, $C_1$ to $C_4$ alkylthio, or a group of the formulae:

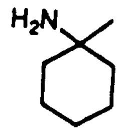 ;

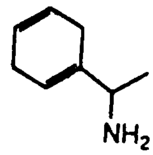 ;

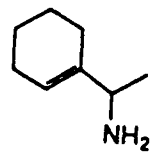 ;

$F_3C-S-CH_2-$ ;

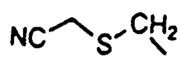 ;

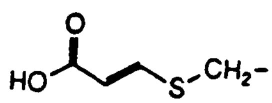 ; or

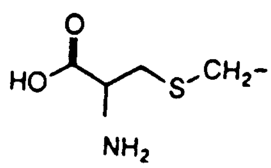 ;

or a protected amino and/or protected carboxy derivative thereof;

ii) a group of the formulae:

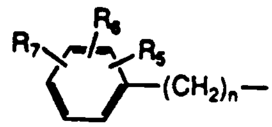
;

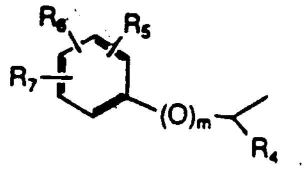
;

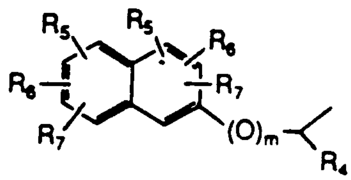
;

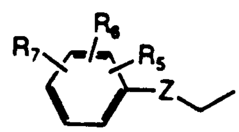
;

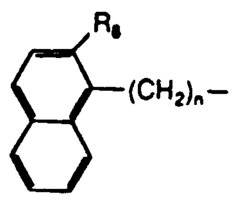
;

wherein each of $R_5$, $R_6$ and $R_7$ is, independently, hydrogen, halo, hydroxy, protected hydroxy, nitro, amino, protected amino, an amine salt, cyano, trifluoromethyl, amino-methyl, protected aminomethyl, N-(methyl- or ethyl-sulfonyl)amino, $C_1$ to $C_6$ alkyl or $C_1$ to $C_4$ alkoxy, $R_4$ is hydroxy, protected hydroxy, formyloxy, amino, protected amino, an amine salt, carboxy, a carboxylate salt, protected carboxy, phenyl carboxylate, (5-indanyl)-carboxylate, sulfonic acid, a sulfonate salt, azido, halo or $C_1$ to $C_6$ alkyl; $R_8$ is $C_1$ to $C_4$ alkoxy; Z is oxygen or sulfur; n is 0, 1, 2 or 3; and m is 0 or 1;
iii) a group of the formula

$R_9-(CH_2)_n-$ ;

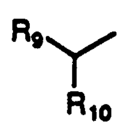
;

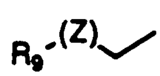
or

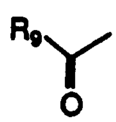

wherein $R_9$ is a heterocyclic ring; $R_{10}$ is hydroxy, protected hydroxy, formyloxy, amino, protected amino, an amine salt, carboxy, a carboxylate salt, protected carboxy, phenyl carboxylate, (5-indanyl)carboxylate, sulfonic acid, a sulfonate salt, azido, halo or $C_1$ to $C_6$ alkyl; n is 0, 1, 2, or 3; and Z is oxygen or sulphur.

**4.** A process of claim 3, wherein the Cytotoxic Agent is:

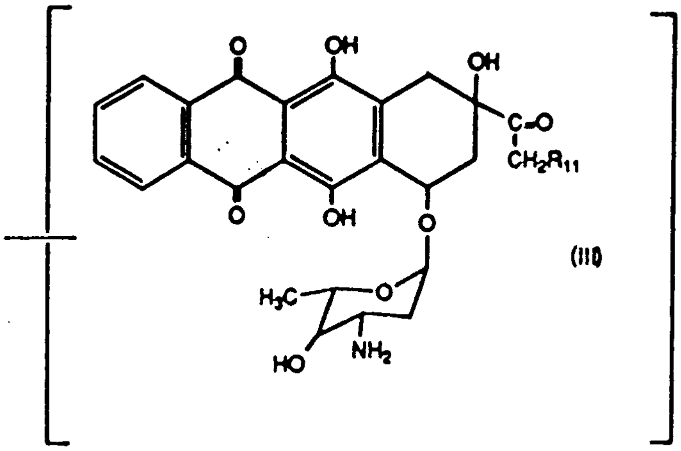

(III)

wherein $R_{11}$ is hydrogen or hydroxy;

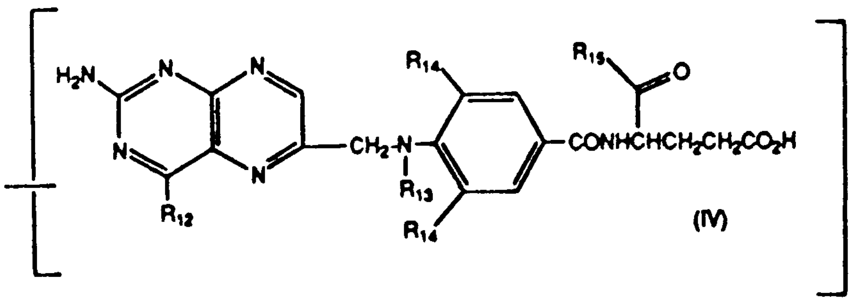

(IV)

wherein $R_{12}$ is amino or hydroxy;
$R_{13}$ is hydrogen or methyl;
$R_{14}$ is hydrogen, fluoro, chloro, bromo, iodo;
$R_{15}$ is hydroxy or a moiety which completes a salt of the carboxylic acid;

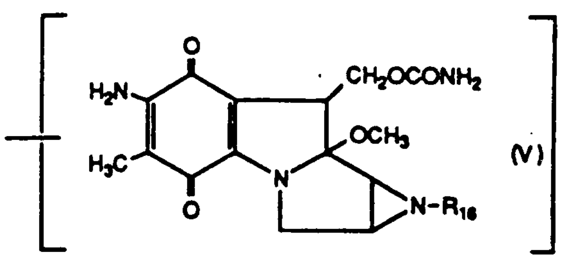

(V)

wherein $R_{16}$ is hydrogen or methyl;

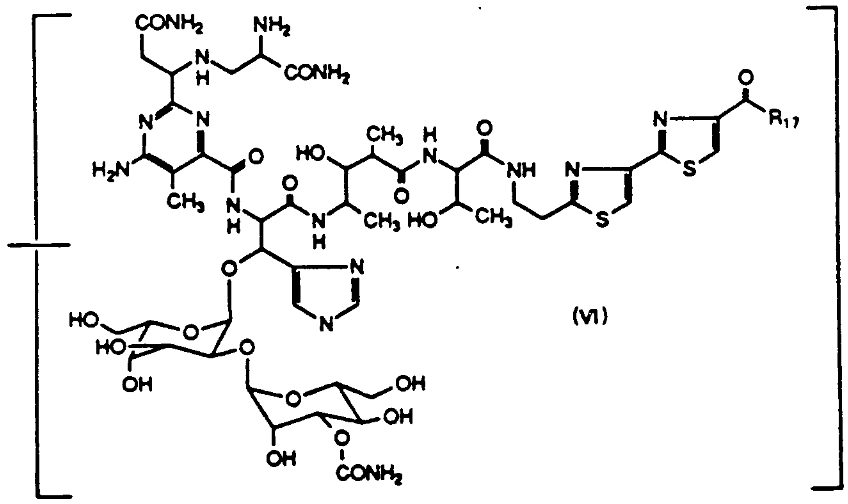

wherein $R_{17}$ is amino, $C_1$-$C_3$ alkylamino, di-($C_1$-$C_3$-alkyl)amino, or $C_4$-$C_6$ polymethylene amino;

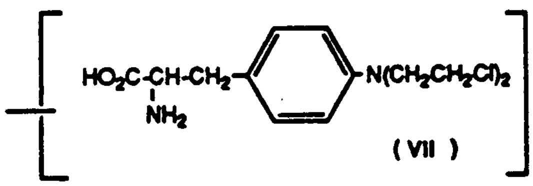

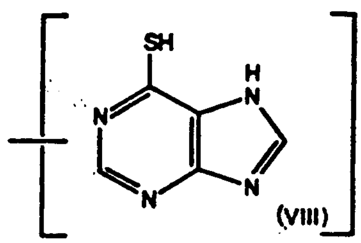

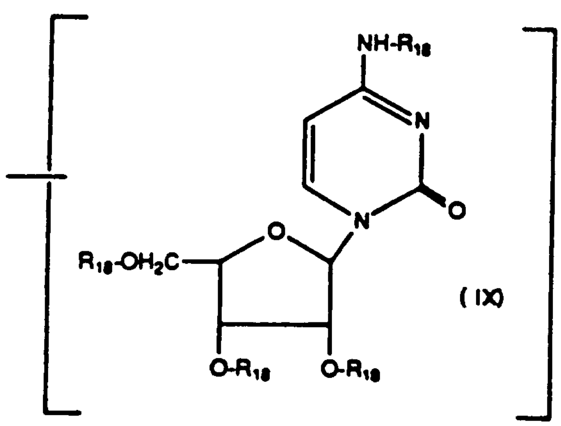

wherein one of the $R_{18}$ moieties is a bond and the others are hydrogen;

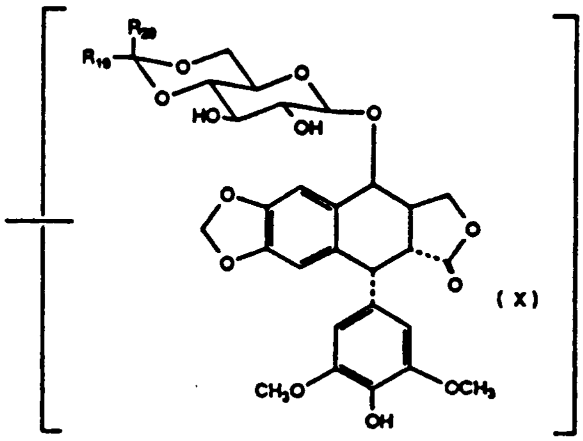

(X)

wherein $R_{19}$ is hydrogen or methyl;
$R_{20}$ is methyl or thienyl;

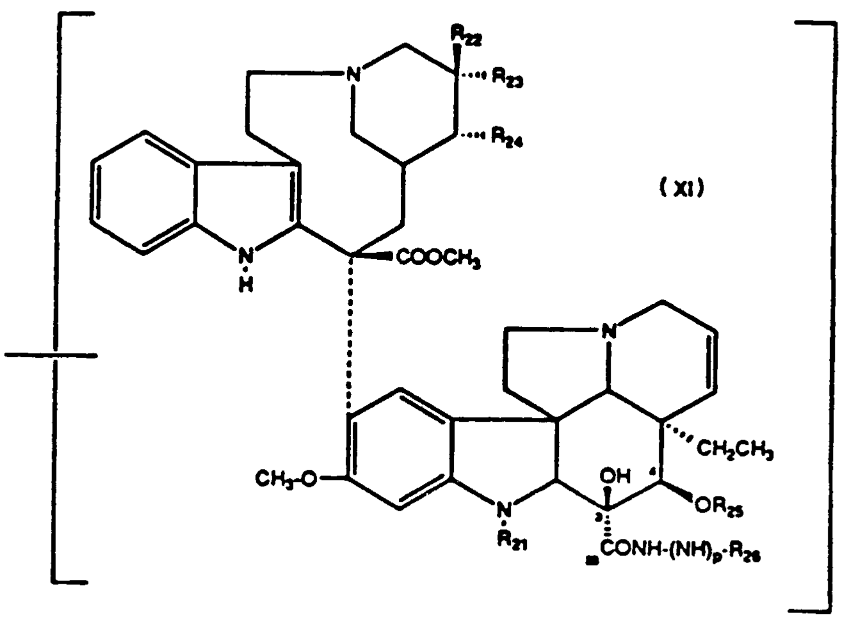

(XI)

wherein $R_{21}$ is H, $CH_3$ or CHO; when $R_{23}$ and $R_{24}$ are taken singly, $R_{24}$ is H, and one of $R_{22}$ and $R_{23}$ is ethyl and the other is H or OH; when $R_{23}$ and $R_{24}$ are taken together with the carbons to which they are attached, they form an oxirane ring in which case $R_{22}$ is ethyl; $R_{25}$ is hydrogen, ($C_1$-$C_3$ alkyl)-CO-, or chloro-substituted ($C_1$-$C_3$ alkyl)-CO;

p is 0 or 1;

$R_{26}$ is a bond, -($C_2$-$C_4$ alkyl)-X, or a group that requires that p is 1 and which is in turn bonded to a carbonyloxy group;

X is -O-, -S-, or -NH-;

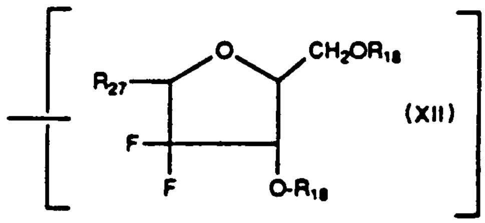

(XII)

wherein $R_{27}$ is a base of one of the formulae:

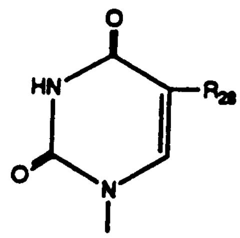

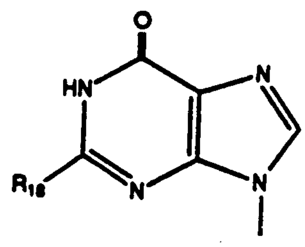

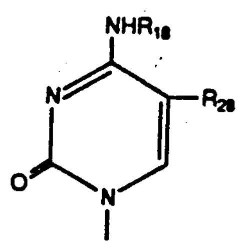

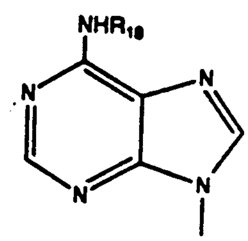

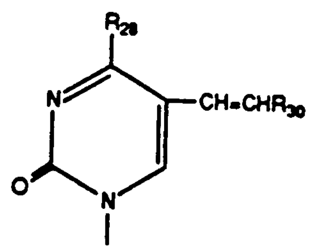

wherein $R_{28}$ is hydrogen, methyl, bromo, fluoro, chloro, or iodo;
$R_{29}$ is $-OR_{18}$ or $-NHR_{18}$;
$R_{30}$ is hydrogen, bromo, chloro, or iodo;

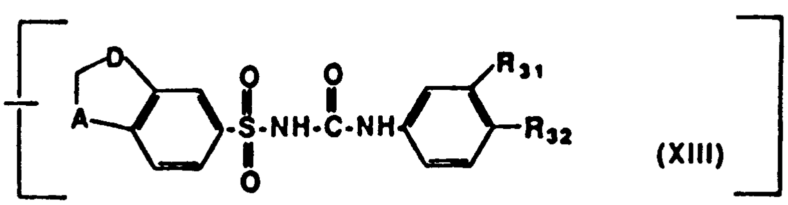

(XIII)

wherein A is -O-, $-NCH_3-$, $-CH_2-$, $-CH_2CH_2-$, or $-CH_2O-$;
D is $-CH_2-$ or -O-;
$R_{31}$ is hydrogen or halo;
$R_{32}$ is halo or trifluoromethyl;

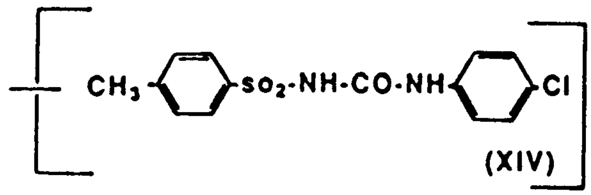

(XIV)

or, 5-uracil.

5. A process of claim 4, wherein $R_1$ is acetyl, propionyl, butanoyl, isopropionyl, pivaloyl, methoxymethyl, phenylacetyl, phenoxyacetyl, 2-(aminomethyl)-phenylacetyl, 2-phenyl-2-hydroxyacetyl, 2-phenyl-2-(sodium sulfonato)-acetyl, 2-phenyl-2-2-carboxyacetyl, 2-(4-hydroxyphenyl)-2-carboxyacetyl, 2-phenyl-2-aminoacetyl, 2-(4-hydroxyphenyl)-2-aminoacetal, 2-(3-(N-(methylsulfonylamino))phenyl)-2-aminoacetyl, 2-phenyl-2-(5-indanyl carboxylate)acetyl, 2-phenyl-2-(phenyl carboxylate)acetyl, 2-phenyl-2-azidoacetyl, 2-phenoxypropionyl, 2,5-dimethoxybenzoyl, 2-(formyloxy)-2-phenylacetyl, 2-(2-ethoxynaphth-1-yl)acetyl, 2-(naphth-1-yl)-2-aminoacetyl, 2-(naphth-2-yl)-2-aminoacetyl, 2-(2,5-dichlorophenylthio)acetyl, 2-(3,4-dichlorophenylthio)acetyl, 2-(1-tetrazolyl)acetyl, 2-(N-(3,5-dichloropyrid-4-oxyl))acetyl, 2-(2-aminothiazol-4-yl)-acetyl, 2-(2-thienyl)acetyl, 2-(4-pyridylthio)acetyl, 2-(N-methyl-4-pyridiniumthio)acetyl, 2-(2-amino-4-phenylthiazol-5-yl)acetyl, 2-(3-hydroxy-4-carboxyisothiazol-5-ylthio)acetyl, 3-phenyl-5-methylisoxazolyl-3-formyl, 3-(2-chlorophenyl)-5-methylisoxazolyl-3-formyl, 3-(2,5-dichlorophenyl)-5-methylisoxazolyl-3-formyl, 3-(2-fluoro-5-chlorophenyl)-5-methylisoxazolyl-3-formyl, 2-(2-thienyl)-2-aminoacetyl, 2-(2-thienyl)-2-(sodium carboxylate)acetyl, 2-(N-(4-pyridinium))acetyl, 2-(2-benzothienyl)acetyl, 2-(3-benzothienyl)acetyl, 2-(2-benzofuryl) acetyl, and 2-(3-benzofuryl)acetyl.

6. A process as claimed in Claim 5, wherein the Cytotoxic Agent is methotrexate, 5-fluorouracil, desacetylvinblastine hydrazide, desacetylvinblastine aminoethanethiol, a desacetylvinblastine hydrazodecarboxy moiety, a 7-(carboxyamino)desacetylcolchicine moiety, N-(p-tosyl)-N'-(p-chlorophenyl)urea, or N-(((4-chloro-phenyl)amino)-carbonyl)-2,3-dihydro-1H-indene-5-sulfonamide.

7. A process as claimed in Claim 6, wherein $R_1$ is 2-(thien-2-yl)acetyl and ZZ is 0.

8. A process as claimed in Claim 7, wherein the Cytotoxic Agent is methotrexate.

9. A process as claimed in Claim 8, wherein the methotrexate is bonded through its carboxy group to the cephalosporin Substrate.

10. A process as claimed in Claim 7, wherein the Cytotoxic Agent is 5-fluorouracil.

11. A process as claimed in Claim 10, wherein the 5-fluorouracil is bonded through its $N_1$ nitrogen to the cephalosporin Substrate.

12. A process as claimed in Claim 7, wherein the Cytotoxic Agent is desacetylvinblastine hydrazide.

13. A process of Claim 12, wherein the desacetylvinblastine hydrazide is bonded through the otherwise unbound nitrogen of its hydrazido group to the cephalosporin Substrate.

14. A process as claimed in Claim 7, wherein the Cytotoxic Agent is desacetylvinblastine aminoethanethiol.

15. A process as claimed in Claim 14, wherein the desacetylvinblastine aminoethanethiol is bonded through the thiol group of its aminoethanethiol moiety to the cephalosporin Substrate.

16. A process as claimed in Claim 7, wherein the Cytotoxic Agent is a desacetylvinblastine hydrazidocarboxy moiety.

17. A process as claimed in Claim 16, wherein the desacetylvinblastine moiety is bonded through an oxygen atom of the hydrazidocarboxy group to the cephalosporin Substrate.

18. A process as claimed in Claim 7, wherein the Cytotoxic Agent is N-(p-tosyl)-N'-(p-chlorophenyl)urea.

19. A process as claimed in Claim 18, wherein N-(p-tosyl)-N'-(p-chlorophenyl)urea is bonded through the p-tosyl nitrogen to the cephalosporin Substrate.

20. A process as claimed in claim 6, wherein $R_1$ is 2-(thien-2-yl)acetyl and ZZ is 1.

21. A process as claimed in Claim 20, wherein the Cytotoxic Agent is a 7-(carboxyamino)-desacetylcolchicine moiety.

22. A process as claimed in Claim 21, wherein the 7-(carboxyamlno)desacetylcolchicine moiety is bonded through an oxygen atom of the 7-(carboxyamino) group to the cephalosporin Substrate.

23. A process as claimed in Claim 20, wherein the Cytotoxic Agent is desacetylvinblastine aminoethanethiol.

24. A process as claimed in claim 23, wherein desacetylvinblastine aminoethanethiol is bonded through the thiol group of the aminoethanethiol group to the cephalosporin Substrate.

25. A process as claimed in Claim 20, wherein the Cytotoxic Agent is the desacetylvinblastine hydrazidocarboxy moiety.

26. A process as claimed in Claim 25, wherein the desacetylvinblastine hydrazidocarboxy moiety is bonded through an oxygen atom of the hydrazidocarboxy group to the cephalosporin moiety.

27. A process as claimed in Claim 6, wherein $R_1$ is phenoxyacetyl and ZZ is 1.

28. A process as claimed in Claim 27, wherein the Cytotoxic Agent is the desacetylvinblastine hydrazidocarboxy moiety.

29. A process as claimed in Claim 28, wherein the desacetylvinblastine hydrazidocarboxy moiety is bonded though an oxygen atom of the hydrazidocarboxy group to the cephalosporin Substrate.

30. A process for preparing an antibody-enzyme conjugate compound of Formula (I):

Antibody-Enzyme (I)

wherein:

the Enzyme reacts with a compound prepared by a process of any one of Claims 1 to 31, and causes the separation of the Cytotoxic Agent from the Substrate; and

the Antibody has specificity for an antigen expressed by the target tissue which comprises conjugating an Enzyme and an Antibody using a protein-protein coupling reagent,

wherein the Enzyme is beta-lactamase.

31. A process as claimed in Claim 30, wherein the Antibody complexes with antigens expressed solely, or in supernatural abundance, by malignant tumor cells.

32. A process as claimed in Claim 31, wherein the Antibody has specificity for carcinoembryonic antigen.

33. A process as claimed in Claim 32, wherein the Antibody is ZCE 025.

34. A process as claimed in Claim 32, wherein the Antibody is CEM 231.6.7.

35. A process as claimed in Claim 31, wherein the Antibody is T101.

36. A process as claimed in Claim 31, wherein the Antibody has specificity for the KSI/4 antigen.

37. A process as claimed in any one of Claims 30 to 36 wherein the Antibody is an F(ab') fragment.

**38.** A process for preparing a composition containing an Antibody-Enzyme compound of formula (I) as defined in any one of claims 30-37 which comprises combining the compound with a pharmaceutically-acceptable carrier to provide a solid or liquid formulation for administration orally, topically or by injection.

**39.** A process for preparing a composition containing a Substrate-Cytotoxic Agent compound of formula (II) as defined in any one of claims 1-29 which comprises combining the compound with a pharmaceutically-acceptable carrier to provide a solid or liquid formulation for administration orally, topically or by injection.

**40.** A kit of parts adapted for the treatment of a neoplastic disease comprising a composition prepared by the process of claim 38 or claim 39.

**Patentansprüche**
**Patentansprüche für folgende Vertragsstaaten : AT, BE, CH, DE, FR, GB, IT, LI, NL, SE**

**1.** Verbindung der Formel (II):

Substrat-cytotoxisches Mittel (II)

worin Substrat für ein Molekül steht, das ein Substrat für ein $\beta$-Lactamaseenzym ist, wobei dieses Enzym das Substrat vom cytotoxischen Mittel abspaltet und somit trennt.

**2.** Verbindung nach Anspruch 1, in der das Substrat ein Cephalosporin folgender Formel ist

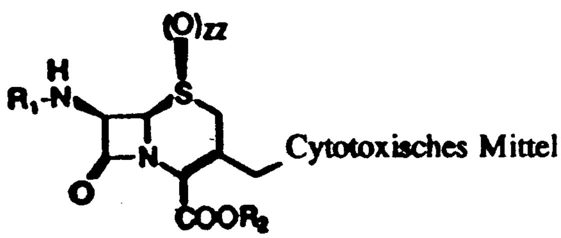

worin ZZ für 0 oder 1 steht, $R_1$ für eine von einer $C_1$ bis $C_{30}$ Alkylgruppe stammenden Acylgruppe steht, und $R_2$ für Wasserstoff, ein organisches oder anorganisches Kation, eine Carboxy-Schutzgruppe oder eine nicht-toxische, metabolisch labile esterbildende Gruppe steht.

**3.** Verbindung nach Anspruch 1, in der $R_1$ für eine Acylgruppe der Formel $-COR_3$ steht, worin $R_3$ steht für

i) $C_1$ bis $C_6$ Alkyl, $C_1$ bis $C_6$ substituiertes Alkyl, $C_1$ bis $C_4$ Alkoxy, $C_1$ bis $C_4$ Alkylthio oder eine Gruppe folgender Formeln

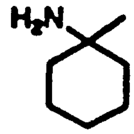 ;

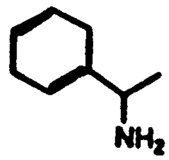 ;

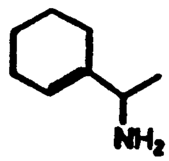 ;

$F_3C-S-CH_2-$ ;

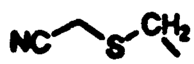 ;

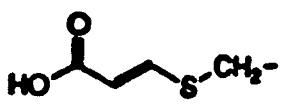 ; oder

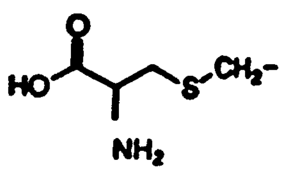 ;

oder ein geschütztes Amino- und/oder Carboxyderivat hiervon,

ii) eine Gruppe der Formeln

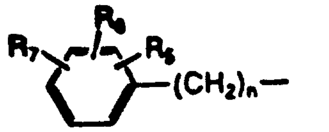 ;

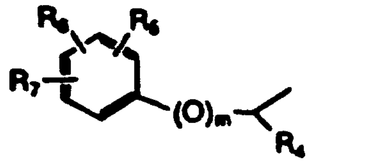 ;

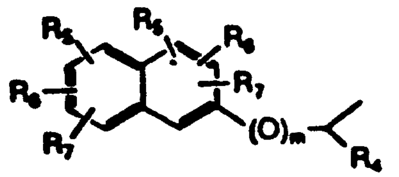 ;

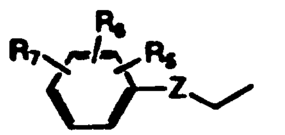 ;

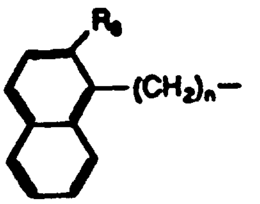 ;

worin jeweils $R_5$, $R_6$ und $R_7$ unabhängig voneinander für Wasserstoff, Halogen, Hydroxy, geschütztes Hydroxy, Nitro, Amino, geschütztes Amino, ein Aminsalz, Cyano, Trifluormethyl, Aminomethyl, geschütztes Aminomethyl, N-(Methyl- oder Ethylsulfonyl)amino, $C_1$ bis $C_6$ Alkyl oder $C_1$ bis $C_4$ Alkoxy steht, $R_4$ für Hydroxy, geschütztes Hydroxy, Formyloxy, Amino, geschütztes Amino, ein Aminsalz, Carboxy, ein Carboxylatsalz, geschütztes Carboxy, Phenylcarboxylat, (5-Indanyl)-carboxylat, Sulfonsäure, ein Sulfonatsalz, Azid, Halogen oder $C_1$ bis $C_6$ Alkyl steht, $R_8$ für $C_1$ bis $C_4$ Alkoxy steht, Z für Sauerstoff oder Schwefel steht, n für 0, 1, 2 oder 3 steht und m für 0 oder 1 steht,

iii) eine Gruppe der Formel

$R_9-(CH_2)_n-$ ;

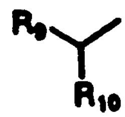 ;

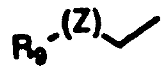 oder

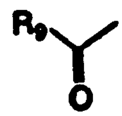

worin $R_9$ für einen heterocyclischen Ring steht, $R_{10}$ für Hydroxy, geschütztes Hydroxy, Formyloxy, Amino, geschütztes Amino, ein Aminsalz, Carboxy, ein Carboxylatsalz, geschütztes Carboxy, Phenylcarboxylat, (5-Indanyl)-carboxylat, Sulfonsäure, ein Sulfonatsalz, Azid, Halogen oder $C_1$ bis $C_6$ Alkyl steht, n für 0, 1, 2 oder 3 steht und Z für Sauerstoff oder Schwefel steht.

4. Verbindung nach Anspruch 3, in der das cytotoxische Mittel folgendes ist

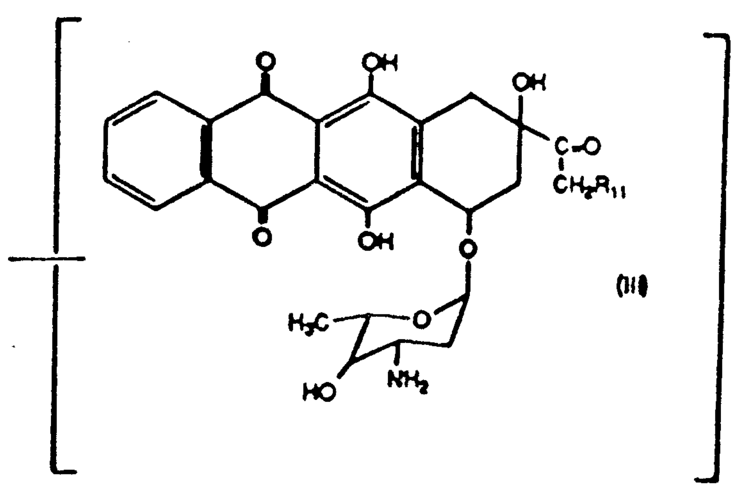

(III)

worin $R_{11}$ für Wasserstoff oder Hydroxy steht,

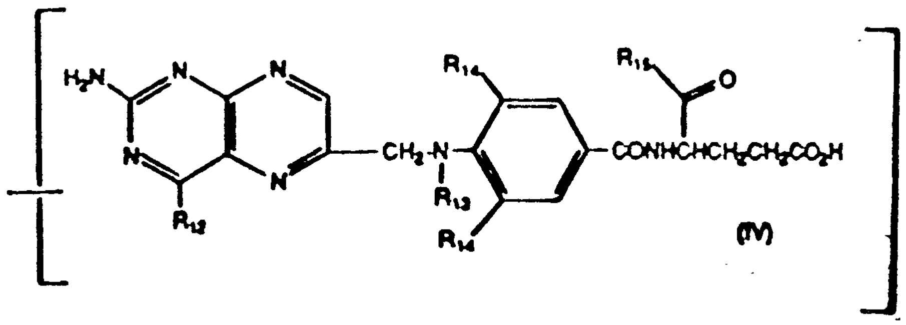

(IV)

worin $R_{12}$ für Amino oder Hydroxy steht,
$R_{13}$ für Wasserstoff oder Methyl steht,
$R_{14}$ für Wasserstoff, Fluor, Chlor, Brom, Iod steht,
$R_{15}$ für Hydroxy oder einen Rest steht, der ein Salz der Carbonsäure vervollständigt,

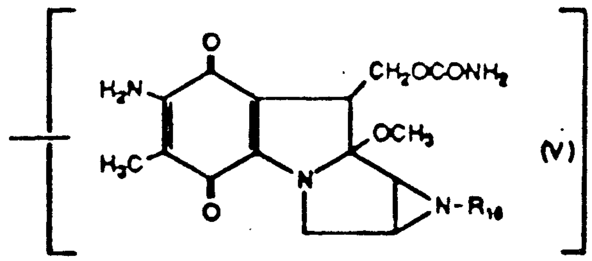

(V)

worin $R_{16}$ für Wasserstoff oder Methyl steht,

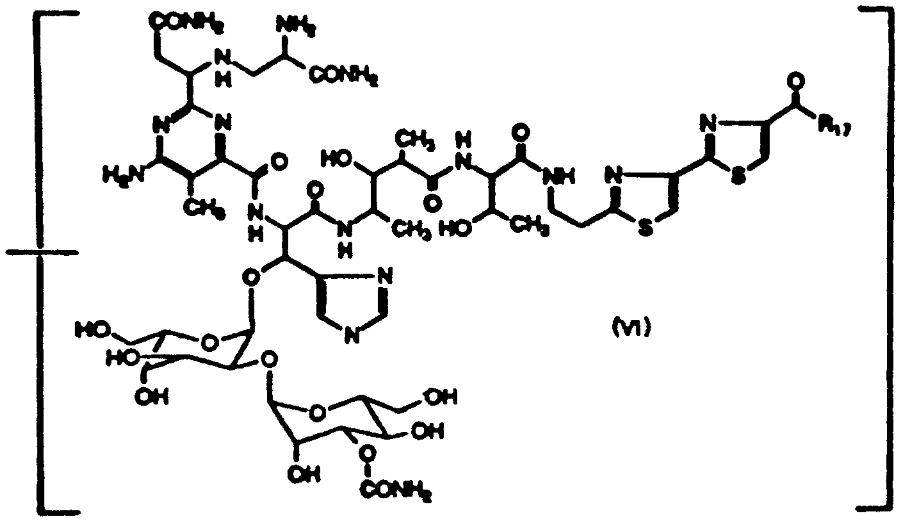

(VI)

worin $R_{17}$ für Amino, $C_1$-$C_3$ Alkylamino, Di-($C_1$-$C_3$ alkyl)amino oder $C_4$-$C_6$ Polmethylenamino steht,

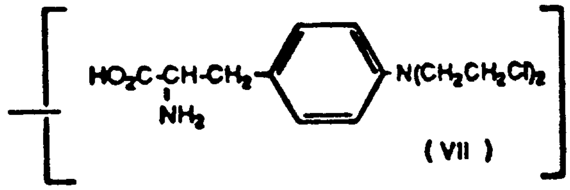

(VII)

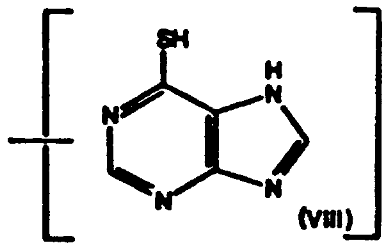

(VIII)

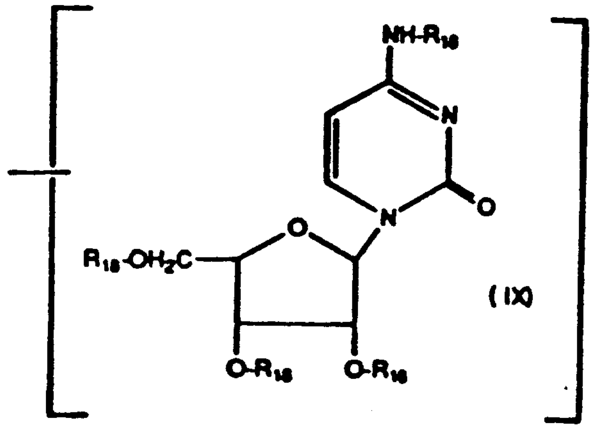

(IX)

worin einer der $R_{18}$ Reste für eine Bindung steht und die anderen für Wasserstoff stehen,

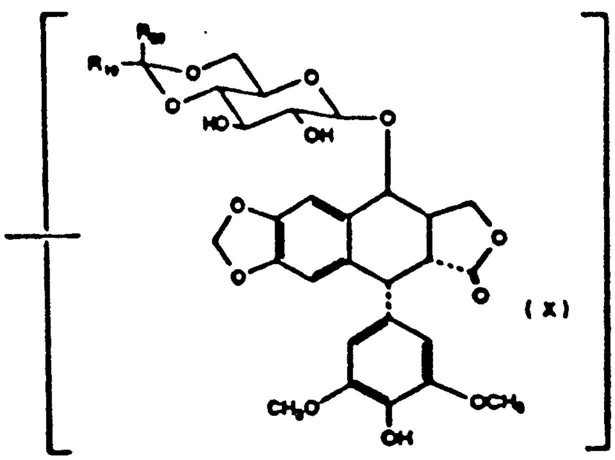

(X)

worin $R_{19}$ für Wasserstoff oder Methyl steht,
$R_{20}$ für Methyl oder Thienyl steht,

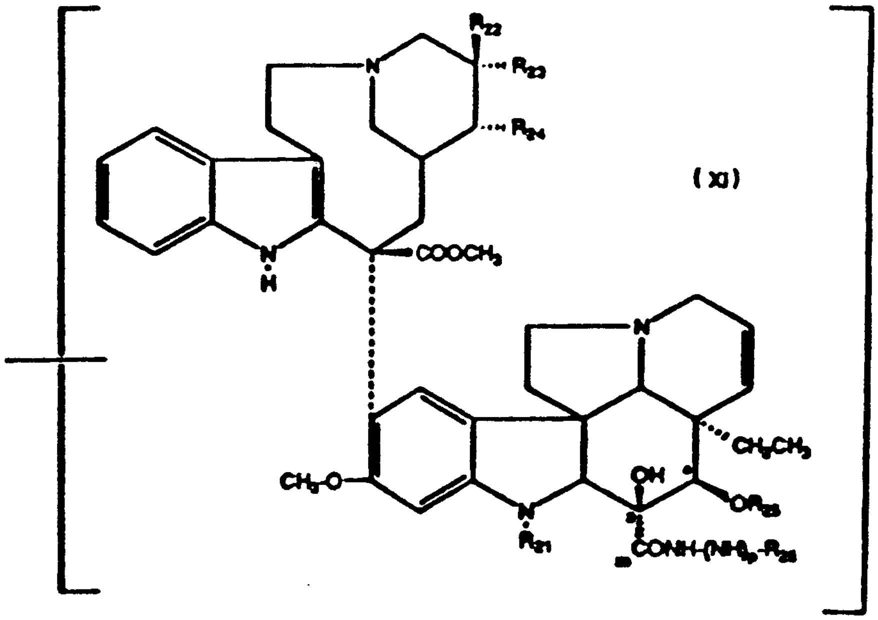

worin $R_{21}$ für H, $CH_3$ oder CHO steht, wenn $R_{23}$ und $R_{24}$ einzeln betrachtet werden, steht $R_{24}$ für H und eines von $R_{22}$ und $R_{23}$ für Ethyl und das andere für H oder OH, wenn $R_{23}$ und $R_{24}$ mit den Kohlenstoffatomen zusammengenommen werden, an die sie gebunden sind, bilden sie einen Oxiranring, wobei dann $R_{22}$ für Ethyl steht, $R_{25}$ für Wasserstoff, ($C_1$-$C_3$-Alkyl)-CO- oder chlorsubstituiertes ($C_1$-$C_3$-Alkyl)-CO- steht,

p für 0 oder 1 steht,

$R_{26}$ für eine Bindung, -($C_2$-$C_4$-Alkyl)-X oder eine Gruppe steht, was erfordert, daß p für 1 steht und die wiederum an eine Carbonyloxygruppe gebunden ist,

X für -O-, -S- oder -NH- steht,

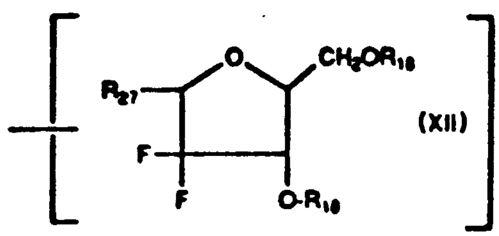

worin $R_{27}$ für eine Base einer der folgenden Formeln steht:

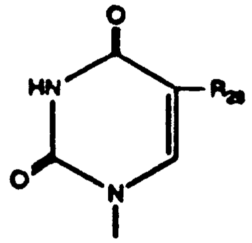

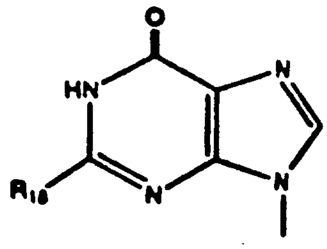

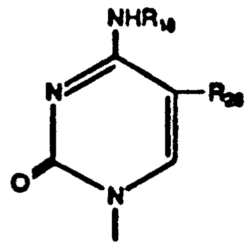

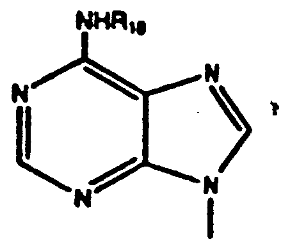

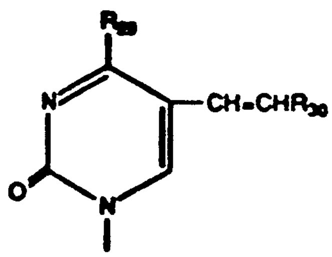

worin $R_{28}$ für Wasserstoff, Methyl, Brom, Fluor, Chlor oder Iod steht,
$R_{29}$ für $-OR_{18}$ oder $-NHR_{18}$ steht,
$R_{30}$ für Wasserstoff, Brom, Chlor oder Iod steht,

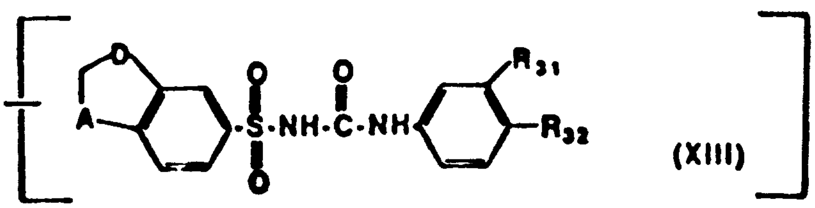

(XIII)

worin A für -O-, $-NCH_3-$, $-CH_2-$, $-CH_2CH_2-$ oder $-CH_2O-$ steht,
D für $-CH_2-$ oder -O- steht,
$R_{31}$ für Wasserstoff oder Halogen steht,
$R_{32}$ für Halogen oder Trifluormethyl steht,

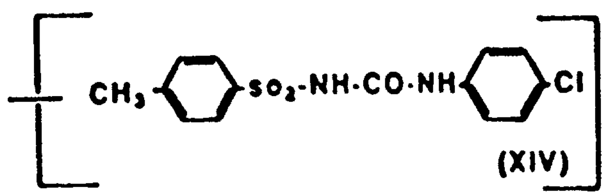

(XIV)

oder 5-Uracil.

5. Verbindung nach Anspruch 4, in der $R_1$ steht für Acetyl, Propionyl, Butanoyl, Isopropionyl, Pivaloyl, Methoxymethyl, Phenylacetyl, Phenoxyacetyl, 2-(Aminomethyl)-phenylacetyl, 2-Phenyl-2-hydroxyacetyl, 2-Phenyl-2-(natriumsulfonat)-acetyl, 2-Phenyl-2-2-carboxyacetyl, 2-(4-Hydroxyphenyl)-2-carboxyacetyl, 2-Phenyl-2-aminoacetyl, 2-(4-Hydroxyphenyl)-2-aminoacetal, 2-(3-(n-(Methylsulfonylamino))phenyl)-2-aminoacetyl, 2-Phenyl-2-(5-indanylcarboxylat)acetyl, 2-Phenyl-2-(phenylcarboxylat)acetyl, 2-Phenyl-2-azidoacetyl, 2-Phenoxypropionyl, 2,5-Dimethoxybenzoyl, 2-(Formyloxy)-2-phenylacetyl, 2-(2-Ethoxynaphth-1-yl)acetyl, 2-(Naphth-1-yl)-2-aminoacetyl, 2-(Naphth-2-yl)-2-aminoacetyl, 2-(2,5-Dichlorphenylthio)acetyl, 2-(3,4-Dichlorphenylthio)acetyl, 2-(1-Tetrazolyl)acetyl, 2-(N-(3,5-Dichlorpyrid-4-oxyl))acetyl, 2-(2-Aminothiazol-4-yl)acetyl, 2-(2-Thienyl)acetyl, 2-(4-Pyridylthio)acetyl, 2-(N-Methyl-4-pyridiniumthio)-acetyl, 2-(2-Amino-4-phenylthiazol-5-yl)acetyl, 2-(3-Hydroxy-4-carboxyisothiazol-5-ylthio)acetyl, 3-Phenyl-5-methylisoxazolyl-3-formyl, 3-(2-Chlorphenyl)-5-methylisoxazolyl-3-formyl, 3-(2,5-Dichlorphenyl)-5-methylisoxazolyl-3-formyl, 3-(2-Fluor-5-chlorphenyl)-5-methylisoxazolyl-3-formyl, 2-(2-Thienyl)-2-aminoacetyl, 2-(2-Thienyl)-2-(Natriumcarboxylat)acetyl, 2-(N-(4-Pyridinium))acetyl, 2-(2-Benzothienyl)acetyl, 2-(3-Benzothienyl)acetyl, 2-(2-Benzofuryl)acetyl und 2-(3-Benzofuryl)acetyl.

6. Verbindung nach Anspruch 5, in der das cytotoxische Mittel ist Methotrexat, 5-Fluoruracil, Desacetylvinblastinhydrazid, Desacetylvinblastinaminoethanthiol, ein Desacetylvinblastinhydrazodecarboxyrest, ein 7-(Carboxyamino)desacetylcolchizinrest, N-(p-Tosyl)-N'-(p-chlorphenyl)harnstoff oder N-(((4-Chlorphenyl)amino)carbonyl)-2,3-dihydro-1H-indol-5-sulfonamid.

7. Verbindung nach Anspruch 6, in der $R_1$ für 2-(Thien-2-yl)acetyl steht und ZZ für 0 steht.

8. Verbindung nach Anspruch 7, in der das cytotoxische Mittel Methotrexat ist.

9. Verbindung nach Anspruch 8, in der das Methotrexat durch dessen Carboxygruppe an das Cephalosporinsubstrat gebunden ist.

10. Verbindung nach Anspruch 7, in der das cytotoxische Mittel 5-Fluoruracil ist.

11. Verbindung nach Anspruch 10, in der das 5-Fluoruracil durch dessen $N_1$ Stickstoff an das Cephalosporinsubstrat gebunden ist.

12. Verbindung nach Anspruch 7, in der das cytotoxische Mittel Desacetylvinblastinhydrazid ist.

13. Verbindung nach Anspruch 12, in der das Desacetylvinblastinhydrazid durch den andererseits ungebundenen Stickstoff der Hydrazidgruppe an das Cephalosporinsubstrat gebunden ist.

14. Verbindung nach Anspruch 7, in der das cytotoxische Mittel Desacetylvinblastinaminoethanthiol ist.

15. Verbindung nach Anspruch 14, in der das Desacetylvinblastinaminoethanthiol durch die Thiolgruppe des Aminoethanthiolrests an das Cephalosporinsubstrat gebunden ist.

16. Verbindung nach Anspruch 7, in der das cytotoxische Mittel ein Desacetylvinblastinhydrazidcarboxyrest ist.

17. Verbindung nach Anspruch 16, in der der Desacetylvinblastinrest durch ein Sauerstoffatom der Hydrazidcarboxygruppe an das Cephalosporinsubstrat gebunden ist.

18. Verbindung nach Anspruch 7, in der das cytotoxische Mittel N-(p-Tosyl)-N'-(p-chlorphenyl)harnstoff ist.

19. Verbindung nach Anspruch 18, in der N-(p-Tosyl)-N'-(p-chlorphenyl)harnstoff durch den p-Tosylstickstoff an das Cephalosporinsubstrat gebunden ist.

20. Verbindung nach Anspruch 6, in der $R_1$ für 2-(Thien-2-yl)acetyl steht und ZZ für 1 steht.

21. Verbindung nach Anspruch 20, in der das cytotoxische Mittel ein 7-(Carboxyamino)-desacetylcolchizinrest ist.

**22.** Verbindung nach Anspruch 21, in der der 7-(Carboxyamino)desacetylcolchizinrest durch ein Sauerstoffatom der 7-(Carboxyamino)gruppe an das Cephalosporinsubstrat gebunden ist.

**23.** Verbindung nach Anspruch 20, in der das cytotoxische Mittel Desacetylvinblastinaminoethanthiol ist.

**24.** Verbindung nach Anspruch 23, in der Desacetylvinblastinaminoethanthiol durch die Thiolgruppe der Aminoethanthiolgruppe an das Cephalosporinsubstrat gebunden ist.

**25.** Verbindung nach Anspruch 20, in der das cytotoxische Mittel der Desacetylvinblastinhydrazidcarboxyrest ist.

**26.** Verbindung nach Anspruch 25, in der der Desacetylvinblastinhydrazidcarboxyrest durch ein Sauerstoffatom der Hydrazidcarboxygruppe an den Cephalosporinrest gebunden ist.

**27.** Verbindung nach Anspruch 6, in der $R_1$ für Phenoxyacetyl steht und ZZ für 1 steht.

**28.** Verbindung nach Anspruch 27, in der das cytotoxische Mittel der Desacetylvinblastinhydrazidcarboxyrest ist.

**29.** Verbindung nach Anspruch 28, in der der Desacetylvinblastinhydrazidcarboxyrest durch ein Sauerstoffatom der Hydrazidcarboxygruppe an das Cephalosporinsubstrat gebunden ist.

**30.** Antikörper-Ensym-Konjugatverbindung der Formel (I)

Antikörper-Enzym (I)

worin

das Enzym mit einer Verbindung nach einem der Ansprüche 1 bis 29 reagiert und die Trennung des cytotoxischen Mittels vom Substrat verursacht, und

der Antikörper eine Spezifität für ein Antigen aufweist, das vom Zielgewebe exprimiert wird, und worin das Enzym beta-Lactamase ist.

**31.** Verbindung nach Anspruch 30, in der der Antikörper mit Antigenen Komplexe bildet, die ausschließlich oder in unnatürlichem Ausmaß von malignen Tumorzellen exprimiert werden.

**32.** Verbindung nach Anspruch 31, in der der Antikörper eine Spezifität für das carcinoembryonale Antigen aufweist.

**33.** Verbindung nach Anspruch 32, in der der Antikörper ZCE 025 ist.

**34.** Verbindung nach Anspruch 32, in der der Antikörper CEM 231.6.7 ist.

**35.** Verbindung nach Anspruch 31, in der der Antikörper T101 ist.

**36.** Verbindung nach Anspruch 31, in der der Antikörper eine Spezifität für das KS1/4 Antigen aufweist.

**37.** Verbindung nach einem der Ansprüche 30 bis 36, in der der Antikörper ein F(ab)' Fragment ist.

**38.** Pharmazeutische Formulierung, die als einen Bestandteil eine Verbindung nach einem der Ansprüche 1 bis 29 zusammen mit einem pharmazeutisch annehmbaren Träger, Verdünnungsmittel oder Hilfsstoff hierfür enthält.

**39.** Pharmazeutische Formulierung, die als einen Bestandteil eine Verbindung nach einem der Ansprüche 30 bis 37 zusammen mit einem pharmazeutisch annehmbaren Träger, Verdünnungsmittel oder Hilfsstoff hierfür enthält.

**40.** Zur Behandlung einer neoplastischen Krankheit angepaßter Kit, der ein Antikörper-Enzym-Konjugat der Formel (I) nach einem der Ansprüche 30 bis 37 und eine Verbindung Substratcyctotoxisches Mittel der Formel (II) nach einem der Ansprüche 1 bis 29 enthält.

**41.** Verbindung der Formel (I) nach einem der Ansprüche 30 bis 37 zur Verwendung bei der Behandlung einer neoplastischen Krankheit.

**42.** Verbindung der Formel (II) nach einem der Ansprüche 1 bis 29 zur Verwendung bei der Behandlung einer neoplastischen Krankheit.

**Patentansprüche für folgenden Vertragsstaat : ES**

**1.** Verfahren zur Herstellung einer Verbindung der Formel (II)

Substrat-cytotoxisches Mittel (II)

worin Substrat für ein Molekül steht, das ein Substrat für ein Enzym ist, wobei dieses Enzym das Substrat vom cytotoxischen Mittel abspaltet und somit trennt,

gekennzeichnet durch folgende Schritte

a) Schützen aller Carboxy- Amino-, Thiol- oder Hydroxygruppen am Substrat oder am cytotoxischen Mittel,

b) Ersetzen einer Abgangsgruppe entweder am Substrat oder am cytotoxischen Mittel durch eine nukleophile Gruppe des jeweils anderen von Substrat und cytotoxischem Mittel unter $S_n1$ oder $S_n2$ Bedingungen, und

c) Abspalten der Schutzgruppen,

worin das Substrat ein Molekül darstellt, das ein Substrat für eine beta-Lactamase ist.

**2.** Verfahren nach Anspruch 1, in dem das Substrat ein Cephalosporin folgender Formel ist

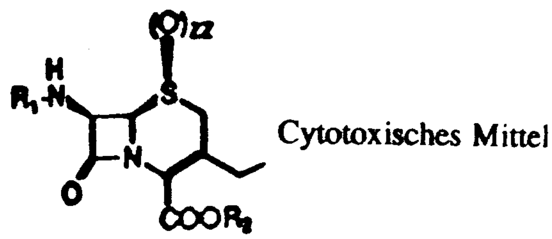

worin ZZ für 0 oder 1 steht, $R_1$ für eine von einer $C_1$ bis $C_{30}$ Alkylgruppe stammenden Acylgruppe steht, und $R_2$ für Wasserstoff, ein organisches oder anorganisches Kation, eine Carboxy-Schutzgruppe oder eine nicht-toxische, metabolisch labile esterbildende Gruppe steht.

**3.** Verfahren nach Anspruch 2, in dem $R_1$ für eine Acylgruppe der Formel $-COR_3$ steht, worin $R_3$ steht für

i) $C_1$ bis $C_6$ Alkyl, $C_1$ bis $C_6$ substituiertes Alkyl, $C_1$ bis $C_4$ Alkoxy, $C_1$ bis $C_4$ Alkylthio oder eine Gruppe folgender Formeln

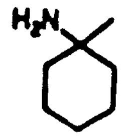 ;

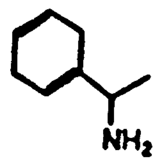 ;

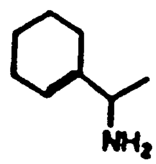 ;

$F_3C-S-CH_2-$ ;

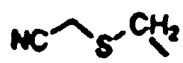 ;

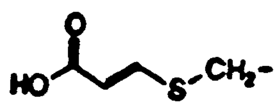 ; oder

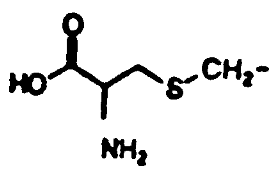 ;

oder ein geschütztes Amino- und/oder Carboxyderivat hiervon,

ii) eine Gruppe der Formeln

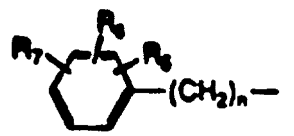 ;

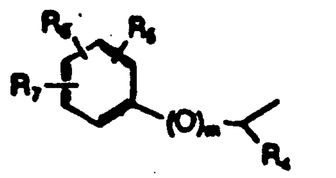 ;

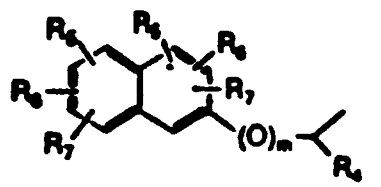 ;

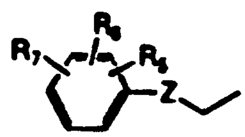 ;

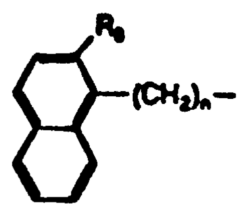 ;

worin jeweils $R_5$, $R_6$ und $R_7$ unabhängig voneinander für Wasserstoff, Halogen, Hydroxy, geschütztes Hydroxy, Nitro, Amino, geschütztes Amino, ein Aminsalz, Cyano, Trifluormethyl, Aminomethyl, geschütztes Aminomethyl, N-(Methyl- oder Ethylsulfonyl)amino, $C_1$ bis $C_6$ Alkyl oder $C_1$ bis $C_4$ Alkoxy steht, $R_4$ für Hydroxy, geschütztes Hydroxy, Formyloxy, Amino, geschütztes Amino, ein Aminsalz, Carboxy, ein Carboxylatsalz, geschütztes Carboxy, Phenylcarboxylat, (5-Indanyl)-carboxylat, Sulfonsäure, ein Sulfonatsalz, Azid, Halogen oder $C_1$ bis $C_6$ Alkyl steht, $R_8$ für $C_1$ bis $C_4$ Alkoxy steht, Z für Sauerstoff oder Schwefel steht, n für 0, 1, 2 oder 3 steht und m für 0 oder 1 steht,

iii) eine Gruppe der Formel

$R_9-(CH_2)_n-$ ;

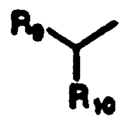

;

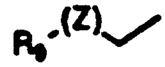

oder

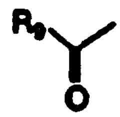

worin $R_9$ für einen heterocyclischen Ring steht, $R_{10}$ für Hydroxy, geschütztes Hydroxy, Formyloxy, Amino, geschütztes Amino, ein Aminsalz, Carboxy, ein Carboxylatsalz, geschütztes Carboxy, Phenylcarboxylat, (5-Indanyl)-carboxylat, Sulfonsäure, ein Sulfonatsalz, Azid, Halogen oder $C_1$ bis $C_6$ Alkyl steht, n für 0, 1, 2 oder 3 steht und Z für Sauerstoff oder Schwefel steht.

**4.** Verfahren nach Anspruch 3, in dem das cytotoxische Mittel folgendes ist

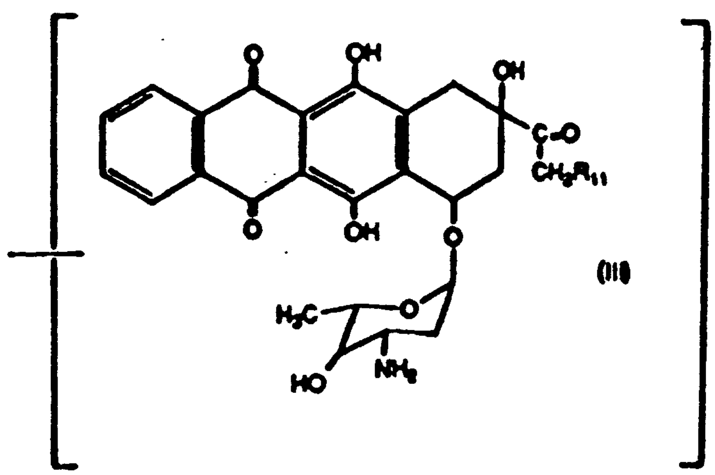

(III)

worin $R_{11}$ für Wasserstoff oder Hydroxy steht,

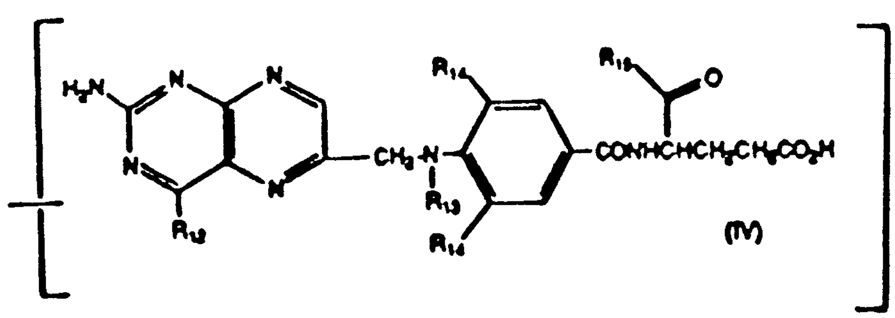

(IV)

worin $R_{12}$ für Amino oder Hydroxy steht,
$R_{13}$ für Wasserstoff oder Methyl steht,
$R_{14}$ für Wasserstoff, Fluor, Chlor, Brom, Iod steht,
$R_{15}$ für Hydroxy oder einen Rest steht, der ein Salz der Carbonsäure vervollständigt,

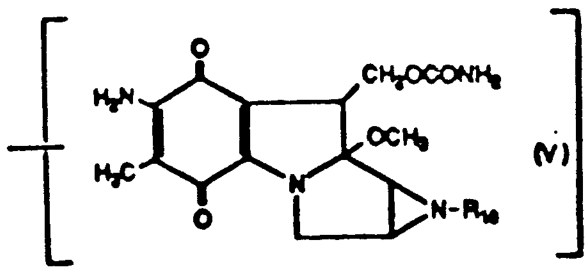

(V)

worin $R_{16}$ für Wasserstoff oder Methyl steht,

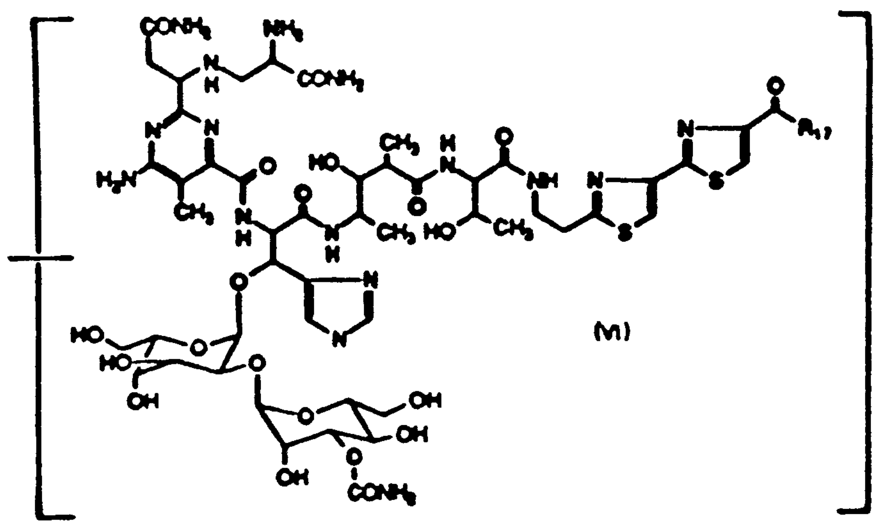

(VI)

worin $R_{17}$ für Amino, $C_1$-$C_3$ Alkylamino, Di-($C_1$-$C_3$ alkyl)amino oder $C_4$-$C_6$ Polymethylenamino steht,

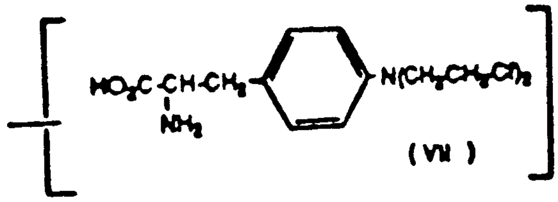

(VII)

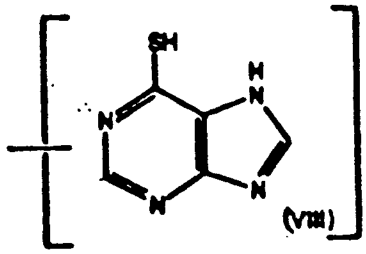

(VIII)

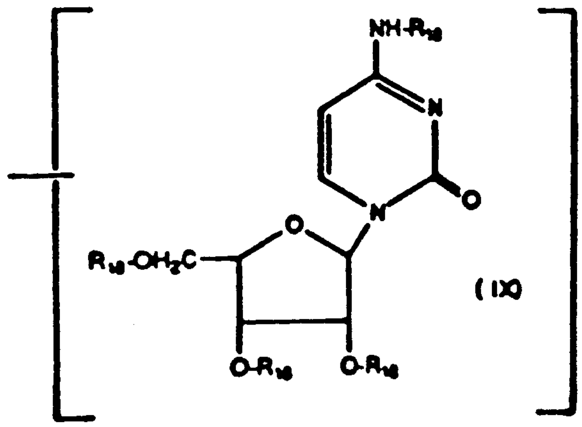

(IX)

worin einer der $R_{18}$ Reste für eine Bindung steht und die anderen für Wasserstoff stehen,

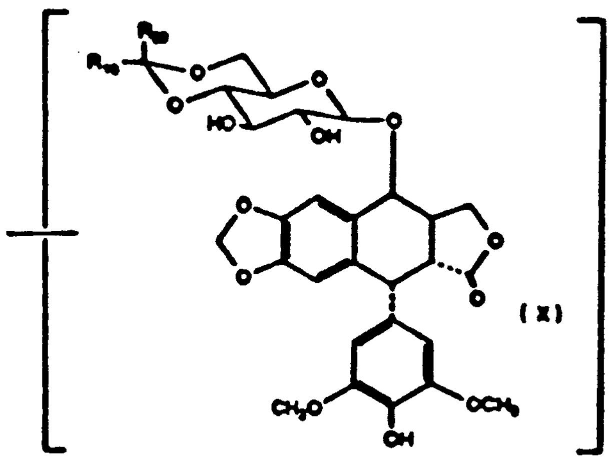

(X)

worin $R_{19}$ für Wasserstoff oder Methyl steht,
$R_{20}$ für Methyl oder Thienyl steht,

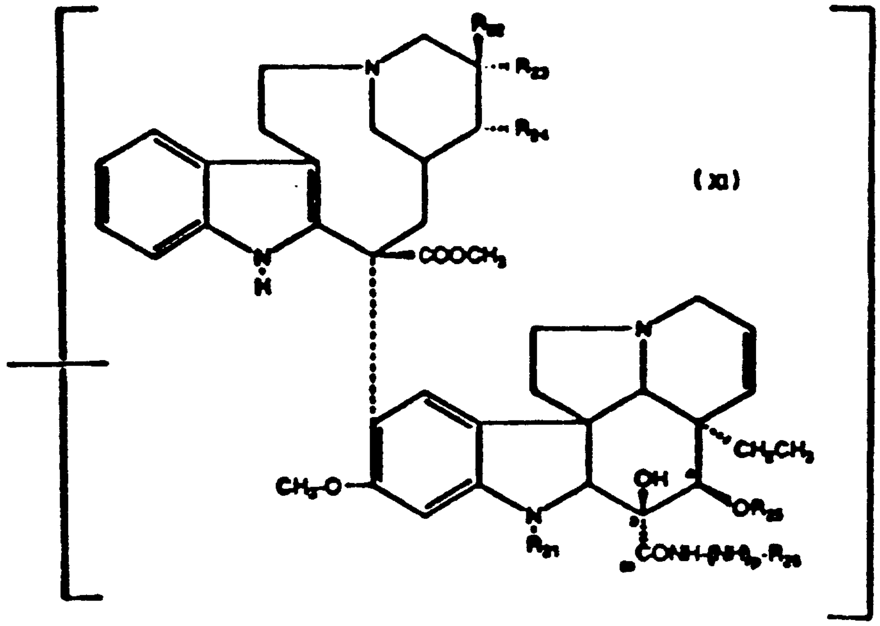

(XI)

worin $R_{21}$ für H, $CH_3$ oder CHO steht, wenn $R_{23}$ und $R_{24}$ einzeln betrachtet werden, steht $R_{24}$ für H und eines von $R_{22}$ und $R_{23}$ für Ethyl und das andere für H oder OH, wenn $R_{23}$ und $R_{24}$ mit den Kohlenstoffatomen zusammengenommen werden, an die sie gebunden sind, bilden sie einen Oxiranring, wobei dann $R_{22}$ für Ethyl steht, $R_{25}$ für Wasserstoff, ($C_1$-$C_3$-Alkyl)-CO- oder chlorsubstituiertes ($C_1$-$C_3$-Alkyl)-CO- steht,

p für 0 oder 1 steht,

$R_{26}$ für eine Bindung, -($C_2$-$C_4$-Alkyl)-X oder eine Gruppe steht, was erfordert, daß p für 1 steht und die wiederum an eine Carbonyloxygruppe gebunden ist,

X für -O-, -S- oder -NH- steht,

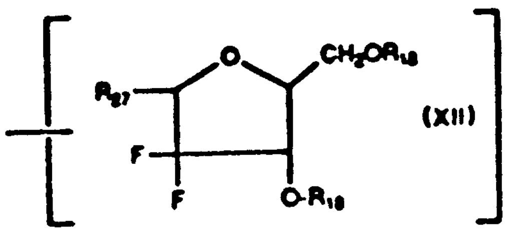

(XII)

worin $R_{27}$ für eine Base einer der folgenden Formeln steht:

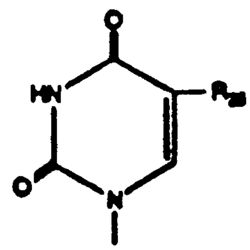
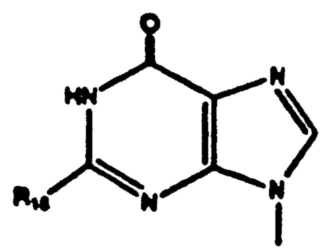

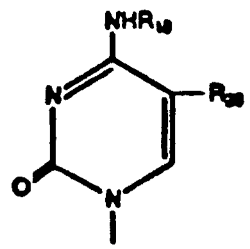
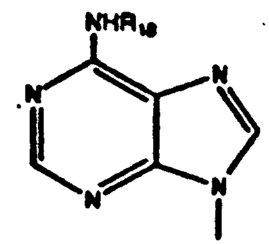

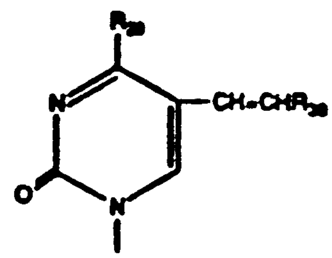

worin $R_{28}$ für Wasserstoff, Methyl, Brom, Fluor, Chlor oder Iod steht,
$R_{29}$ für $-OR_{18}$ oder $-NHR_{18}$ steht,
$R_{30}$ für Wasserstoff, Brom, Chlor oder Iod steht,

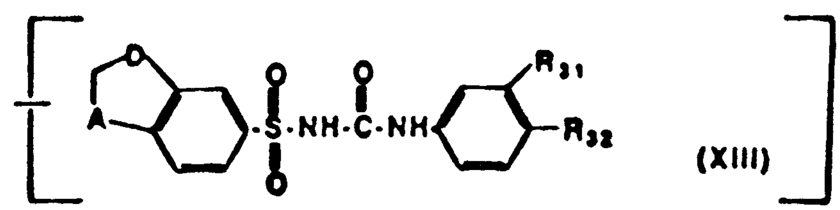
(XIII)

worin A für -O-, $-NCH_3-$, $-CH_2-$, $-CH_2CH_2-$ oder $-CH_2O-$ steht,
D für $-CH_2-$ oder -O- steht,
$R_{31}$ für Wasserstoff oder Halogen steht,
$R_{32}$ für Halogen oder Trifluormethyl steht,

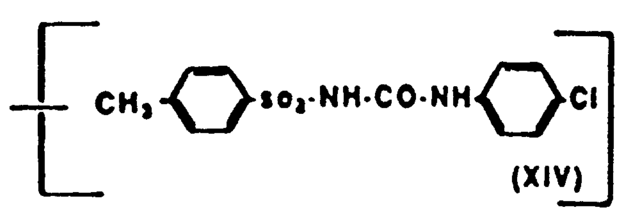
(XIV)

oder 5-Uracil.

**5.** Verfahren nach Anspruch 4, in dem $R_1$ steht für Acetyl, Propionyl, Butanoyl, Isopropionyl, Pivaloyl, Methoxymethyl, Phenylacetyl, Phenoxyacetyl, 2-(Aminomethyl)-phenylacetyl, 2-Phenyl-2-hydroxyacetyl, 2-Phenyl-2-(natriumsulfonat)-acetyl, 2-Phenyl-2-2-carboxyacetyl, 2-(4-Hydroxyphenyl)-2-carboxyacetyl, 2-Phenyl-2-aminoacetyl, 2-(4-Hydroxyphenyl)-2-aminoacetal, 2-(3-(n-(Methylsulfonylamino))phenyl)-2-aminoacetyl, 2-Phenyl-2-(5-indanylcarboxylat)acetyl, 2-Phenyl-2-(phenylcarboxylat)acetyl, 2-Phenyl-2-azidoacetyl, 2-Phenoxypropionyl, 2,5-Dimethoxybenzoyl, 2-(Formyloxy)-2-phenylacetyl, 2-(2-Ethoxynaphth-1-yl)acetyl, 2-(Naphth-1-yl)-2-aminoacetyl, 2-(Naphth-2-yl)-2-aminoacetyl, 2-(2,5-Dichlorphenylthio)acetyl, 2-(3,4-Dichlorphenylthio)acetyl, 2-(1-Tetrazolyl)acetyl, 2-(N-(3,5-Dichlorpyrid-4-oxyl))acetyl, 2-(2-Aminothiazol-4-yl)acetyl, 2-(2-Thienyl)acetyl, 2-(4-Pyridylthio)acetyl, 2-(N-Methyl-4-pyridiniumthio)-acetyl, 2-(2-Amino-4-phenylthiazol-5-yl)acetyl, 2-(3-Hydroxy-4-carboxyisothiazol-5-ylthio)acetyl, 3-Phenyl-5-methylisoxazolyl-3-formyl, 3-(2-Chlorphenyl)-5-methylisoxazolyl-3-formyl, 3-(2,5-Dichlorphenyl)-5-methylisoxazolyl-3-formyl, 3-(2-Fluor-5-chlorphenyl)-5-methylisoxazolyl-3-formyl, 2-(2-Thienyl)-2-aminoacetyl, 2-(2-Thienyl)-2-(Natriumcarboxylat)acetyl, 2-(N-(4-Pyridinium))acetyl, 2-(2-Benzothienyl)acetyl, 2-(3-Benzothienyl)acetyl, 2-(2-Benzofuryl)acetyl und 2-(3-Benzofuryl)acetyl.

**6.** Verfahren nach Anspruch 5, in dem das cytotoxische Mittel ist Methotrexat, 5-Fluoruracil, Desacetylvinblastinhydrazid, Desacetylvinblastinaminoethanthiol, ein Desacetylvinblastinhydrazodecarboxyrest, ein 7-(Carboxyamino)desacetylcolchizinrest, N-(p-Tosyl)-N'-(p-chlorphenyl)harnstoff oder N-(((4-Chlorphenyl)amino)carbonyl)-2,3-dihydro-1H-indol-5-sulfonamid.

**7.** Verfahren nach Anspruch 6, in dem $R_1$ für 2-(Thien-2-yl)acetyl steht und ZZ für 0 steht.

**8.** Verfahren nach Anspruch 7, in dem das cytotoxische Mittel Methotrexat ist.

**9.** Verfahren nach Anspruch 8, in dem das Methotrexat durch dessen Carboxygruppe an das Cephalosporinsubstrat gebunden ist.

**10.** Verfahren nach Anspruch 7, in dem das cytotoxische Mittel 5-Fluoruracil ist.

**11.** Verfahren nach Anspruch 10, in dem das 5-Fluoruracil durch dessen $N_1$ Stickstoff an das Cephalosporinsubstrat gebunden ist.

**12.** Verfahren nach Anspruch 7, in dem das cytotoxische Mittel Desacetylvinblastinhydrazidist.

**13.** Verfahren nach Anspruch 12, in dem das Desacetylvinblastinhydrazid durch den andererseits ungebundenen Stickstoff der Hydrazidgruppe an das Cephalosporinsubstrat gebunden ist.

**14.** Verfahren nach Anspruch 7, in dem das cytotoxische Mittel Desacetylvinblastinaminoethanthiol ist.

**15.** Verfahren nach Anspruch 14, in dem das Desacetylvinblastinaminoethanthiol durch die Thiolgruppe des Aminoethanthiolrests an das Cephalosporinsubstrat gebunden ist.

**16.** Verfahren nach Anspruch 7, in dem das cytotoxische Mittel ein Desacetylvinblastinhydrazidcarboxyrest ist.

**17.** Verfahren nach Anspruch 16, in dem der Desacetylvinblastinrest durch ein Sauerstoffatom der Hydrazidcarboxygruppe an das Cephalosporinsubstrat gebunden ist.

**18.** Verfahren nach Anspruch 7, in dem das cytotoxische Mittel N-(p-Tosyl)-N'-(p-chlorphenyl)harnstoff ist.

**19.** Verfahren nach Anspruch 18, in dem N-(p-Tosyl)-N'-(p-chlorphenyl)harnstoff durch den p-Tosylstickstoff an das Cephalosporinsubstrat gebunden ist.

**20.** Verfahren nach Anspruch 6, in dem $R_1$ für 2-(Thien-2-yl)acetyl steht und ZZ für 1 steht.

21. Verfahren nach Anspruch 20, in dem das cytotoxische Mittel ein 7-(Carboxyamino)-desacetylcolchizinrest ist.

22. Verfahren nach Anspruch 21, in dem der 7-(Carboxyamino)desacetylcolchizinrest durch ein Sauerstoffatom der 7-(Carboxyamino)gruppe an das Cephalosporinsubstrat gebunden ist.

23. Verfahren nach Anspruch 20, in dem das cytotoxische Mittel Desacetylvinblastinaminoethanthiol ist.

24. Verfahren nach Anspruch 23, in dem Desacetylvinblastinaminoethanthiol durch die Thiolgruppe der Aminoethanthiolgruppe an das Cephalosporinsubstrat gebunden ist.

25. Verfahren nach Anspruch 20, in dem das cytotoxische Mittel der Desacetylvinblastinhydrazidcarboxyrest ist.

26. Verfahren nach Anspruch 25, in dem der Desacetylvinblastinhydrazidcarboxyrest durch ein Sauerstoffatom der Hydrazidcarboxygruppe an den Cephalosporinrest gebunden ist.

27. Verfahren nach Anspruch 6, in dem $R_1$ für Phenoxyacetyl steht und ZZ für 1 steht.

28. Verfahren nach Anspruch 27, in dem das cytotoxische Mittel der Desacetylvinblastinhydrazidcarboxyrest ist.

29. Verfahren nach Anspruch 28, in dem der Desacetylvinblastinhydrazidcarboxyrest durch ein Sauerstoffatom der Hydrazidcarboxygruppe an das Cephalosporinsubstrat gebunden ist.

30. Verfahren zur Herstellung einer Antikörper-Enzym-Konjugatverbindung der Formel (I)
Antikörper-Enzym (I)
worin
das Enzym mit einer Verbindung reagiert, die durch ein Verfahren nach einem der Ansprüche 1 bis 31 hergestellt wird und die Trennung des cytotoxischen Mittels vom Substrat verursacht, und
der Antikörper eine Spezifität für ein Antigen aufweist, das vom Zielgewebe exprimiert wird,
gekennzeichnet durch die Konjugation eines Enzyms und eines Antikörpers mittels eines Protein-Protein-Kupplungsmittels.

31. Verfahren nach Anspruch 30, in dem der Antikörper mit Antigenen Komplexe bildet, die ausschließlich oder in unnatürlichem Ausmaß von malignen Tumorzellen exprimiert werden.

32. Verfahren nach Anspruch 31, in dem der Antikörper eine Spezifität für das carcinoembryonale Antigen aufweist.

33. Verfahren nach Anspruch 32, in dem der Antikörper ZCE 025 ist.

34. Verfahren nach Anspruch 32, in dem der Antikörper CEM 231.6.7 ist.

35. Verfahren nach Anspruch 31, in dem der Antikörper T101 ist.

36. Verfahren nach Anspruch 31, in dem der Antikörper eine Spezifität für das KS1/4 Antigen aufweist.

37. Verfahren nach einem der Ansprüche 30 bis 36, in dem der Antikörper ein F(ab)' Fragment ist.

38. Verfahren zur Herstellung einer Zusammensetzung, die eine Antikörper-Enzym-Verbindung der Formel (I) nach einem der Ansprüche 30-37 enthält, gekennzeichnet durch Kombinieren der Verbindung mit einem pharmazeutisch annehmbaren Träger unter Bereitstellung einer festen oder flüssigen Formulierung, die oral, topisch oder durch Injektion verabreicht werden kann.

39. Verfahren zur Herstellung einer Zusammensetzung, die eine Verbindung Substrat-cytotoxisches Mittel der Formel (II) nach einem der Ansprüche 1-29 enthält, gekennzeichnet durch Kombinieren der Verbindung mit einem pharmazeutisch annehmbaren Träger unter Bereitstellung einer festen oder

flüssigen Formulierung, die oral, topisch oder durch Injektion verabreicht werden kann.

**40.** Zur Behandlung einer neoplastischen Krankheit angepaßter Kit, der eine durch das Verfahren nach Anspruch 38 oder Anspruch 39 hergestellte Zusammensetzung enthält.

**Revendications**

**Revendications pour les Etats contractants suivants : AT, BE, CH, DE, FR, GB, IT, LI, NL, SE**

**1.** Composé répondant à la Formule (II) :

Substrat-Agent Cytotoxique (II)

où le Substrat représente une molécule qui est un substrat pour une enzyme bêta-lactamase, où ladite enzyme clive, et par conséquent sépare, le substrat de l'Agent Cytotoxique.

**2.** Composé, tel que revendiqué à la Revendication 1, où le Substrat est une céphalosporine répondant à la formule :

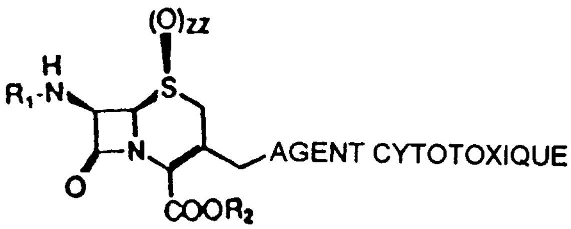

où ZZ vaut 0 ou 1, $R_1$ représente un groupe acyle dérivé d'un groupe alkyle en $C_1$ à $C_{30}$, et $R_2$ représente un atome d'hydrogène, un cation organique ou inorganique, un groupe carboxy-protecteur ou un groupe formant un ester, non-toxique et métaboliquement instable.

**3.** Composé selon la Revendication 1, où $R_1$ représente un groupe acyle répondant à la formule $-COR_3$ où $R_3$ représente :

i) un groupe alkyle en $C_1$ à $C_6$, un groupe alkyle en $C_1$ à $C_6$ substitué, un groupe alcoxy en $C_1$ à $C_4$, un groupe alkylthio en $C_1$ à $C_4$ ou un groupe répondant aux formules :

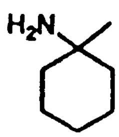

;

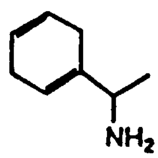

;

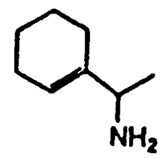 ;

$F_3C-S-CH_2-$ ;

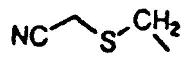 ;

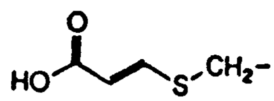 ; ou

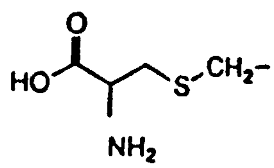 ;

ou un groupe amino protégé et/ou un dérivé carboxyle protégé de ceux-ci;

ii) un groupe répondant aux formules :

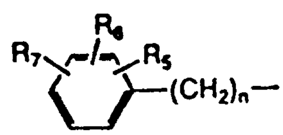 ;

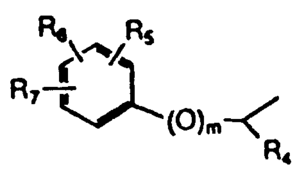 ;

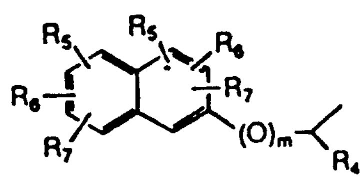 ;

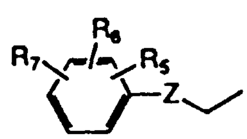 ;

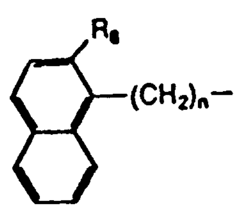 ;

où chacun des groupes $R_5$, $R_6$ et $R_7$ représente, indépendamment l'un de l'autre, un atome d'hydrogène, un atome d'halogène, un groupe hydroxyle, un groupe hydroxyle protégé, un groupe nitro, un groupe amino, un groupe amino protégé, un sel d'amine, un groupe cyano, un groupe trifluorométhyle, un groupe aminométhyle, un groupe aminométhyle protégé, un groupe N-(méthyl- ou éthyl-sulfonyl)amino, un groupe alkyle en $C_1$ à $C_6$ ou un groupe alcoxy en $C_1$ à $C_4$, $R_4$ représente un groupe hydroxyle, un groupe hydroxyle protégé, un groupe formyloxy, un groupe amino, un groupe amino protégé, un sel d'amine, un groupe carboxyle, un sel carboxylate, un groupe carboxyle protégé, un groupe phénylcarboxylate, un groupe (5-indanyl)-carboxylate, un acide sulfonique, un sel sulfonate, un groupe azido, un atome d'halogène ou un groupe alkyle en $C_1$ à $C_6$; $R_8$ représente un groupe alcoxy en $C_1$ à $C_4$; Z représente un atome d'oxygène ou de soufre; n vaut 0, 1, 2 ou 3; et m vaut 0 au 1;

iii) un groupe répondant à la formule :

$$R_9-(CH_2)_n- \quad ;$$

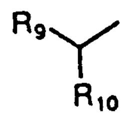

;

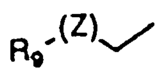

ou

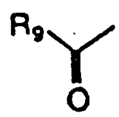

où $R_9$ représente un noyau hétérocyclique; $R_{10}$ représente un groupe hydroxyle, un groupe hydroxyle protégé, un groupe formyloxy, un groupe amino, un groupe amino protégé, un sel d'amine, un groupe carboxyle, un sel carboxylate, un groupe carboxyle protégé, un groupe phénylcarboxylate, un groupe (5-indanyl)carboxylate, un acide sulfonique, un sel sulfonate, un groupe azido, un atome d'halogène ou un groupe alkyle en $C_1$ à $C_6$; n vaut 0, 1, 2 ou 3; et Z représente un atome d'oxygène ou de soufre.

**4.** Composé selon la Revendication 3, où l'Agent Cytotoxique est :

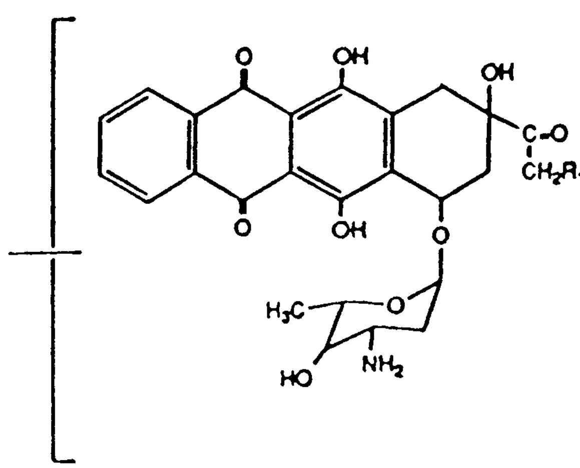

(III)

où $R_{11}$ représente un atome d'hydrogène ou un groupe hydroxyle;

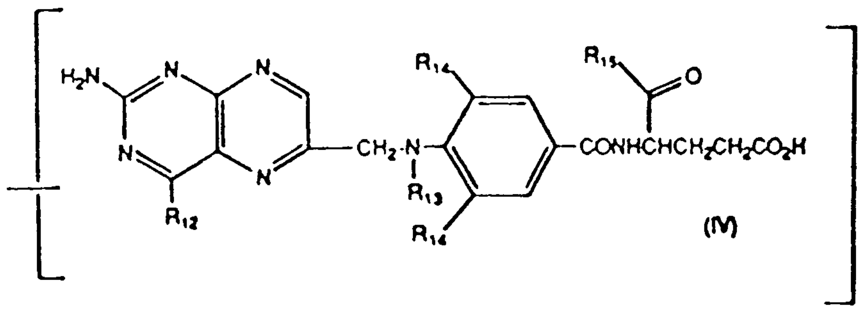

(IV)

où $R_{12}$ représente un groupe amino ou un groupe hydroxyle; $R_{13}$ représente un atome d'hydrogène ou un groupe méthyle; $R_{14}$ représente un atome d'hydrogène, un atome de fluor, un atome de chlore, un atome de brome, un atome d'iode; $R_{15}$ représente un groupe hydroxyle ou une fraction qui complète un sel de l'acide carboxylique;

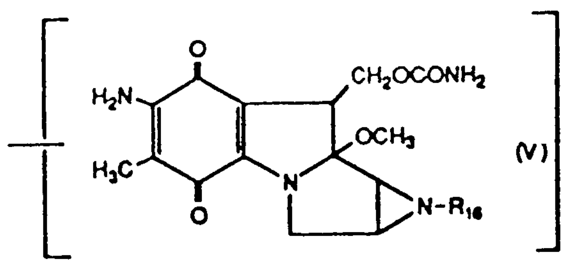

(V)

où $R_{16}$ représente un atome d'hydrogène ou un groupe méthyle;

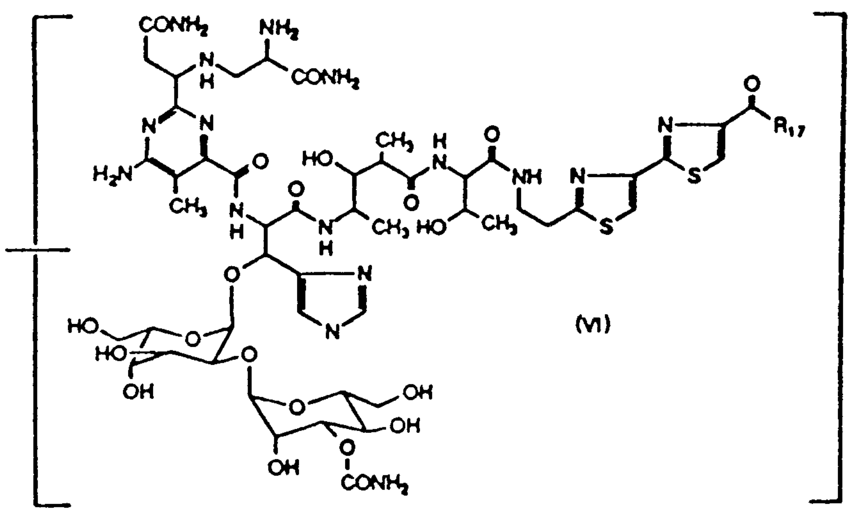

(VI)

où $R_{17}$ représente un groupe amino, un groupe alkylamino en $C_1$ à $C_3$, un groupe di-(alkyle en $C_1$ à $C_3$)amino ou un groupe (polyméthyléne en $C_4$ à $C_6$)amino;

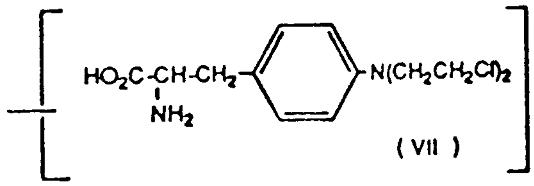

(VII)

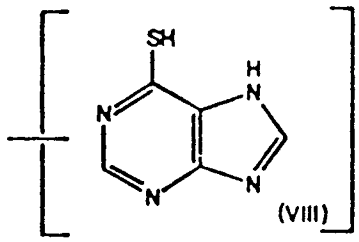

(VIII)

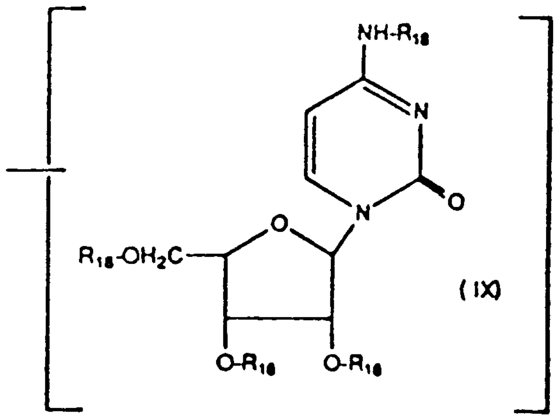

(IX)

où une des fractions $R_{18}$ représente une liaison et les autres sont des atomes d'hydrogène;

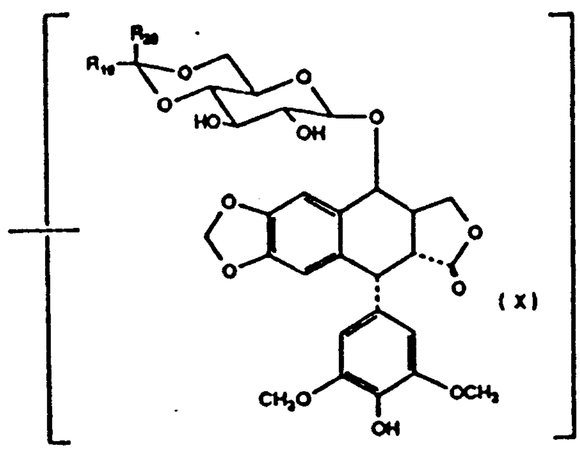

(X)

où $R_{19}$ représente un atome d'hydrogène ou un groupe méthyle; $R_{20}$ représente un groupe méthyle ou un groupe thiényle;

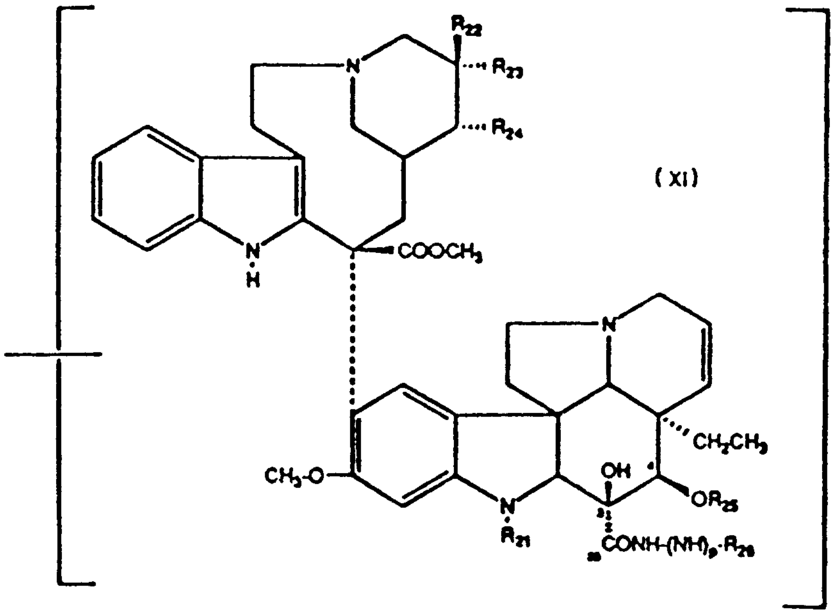

où $R_{21}$ représente H, $CH_3$ ou CHO; lorsque $R_{23}$ et $R_{24}$ sont pris séparément, $R_{24}$ représente H, et un des groupes $R_{22}$ et $R_{23}$ représente un groupe éthyle et l'autre représente H ou OH; lorsque et $R_{23}$ et $R_{24}$ sont pris ensemble avec les atomes de carbone auxquels ils sont liés, ils forment un noyau oxirane, auquel cas $R_{22}$ représente un groupe éthyle; $R_{25}$ représente un atome d'hydrogéne, un groupe (alkyle en $C_1$ à $C_3$)-CO- ou un groupe (alkyle en $C_1$ à $C_3$)-CO chloro-substitué;

p vaut 0 ou 1;

$R_{26}$ représente une liaison, un groupe -(alkyle en $C_2$ à $C_4$)-X ou un groupe qui requiert que p vaut 1 et qui est à son tour lié à un groupe carbonyloxy;

X représente -O-, -S- ou -NH-;

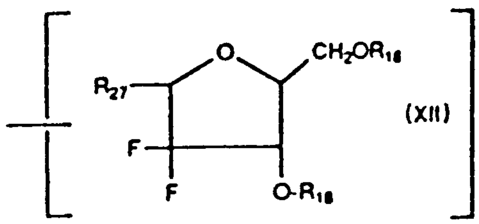

où $R_{27}$ représente une base d'une des formules :

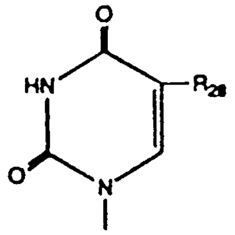

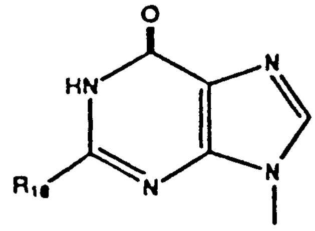

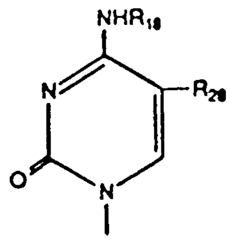

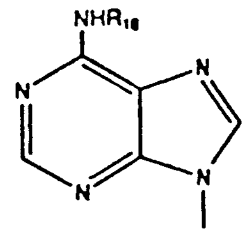

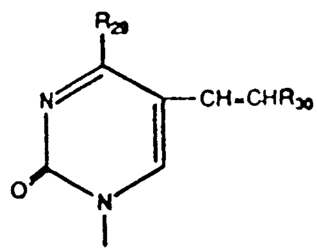

où $R_{28}$ représente un atome d'hydrogène, un groupe méthyle, un atome de brome, un atome de fluor, un atome de chlore ou un atome d'iode;

$R_{29}$ représente $-OR_{18}$ ou $-NHR_{18}$;

$R_{30}$ représente un atome d'hydrogène, un atome de brome, un atome de chlore ou un atome d'iode;

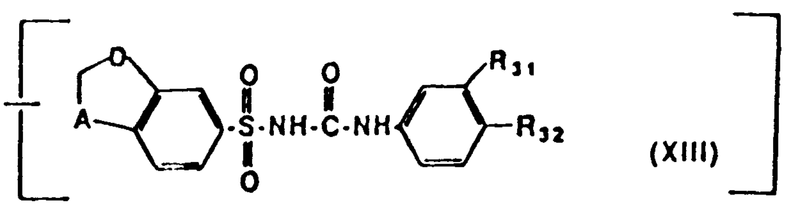

(XIII)

où A représente -O-, $-NCH_3-$, $-CH_2-$, $-CH_2CH_2-$ ou $-CH_2O-$;

D représente $-CH_2-$ ou -O-;

$R_{31}$ représente un atome d'hydrogène ou un atome d'halogène;

$R_{32}$ représente un atome d'halogène ou un groupe trifluorométhyle;

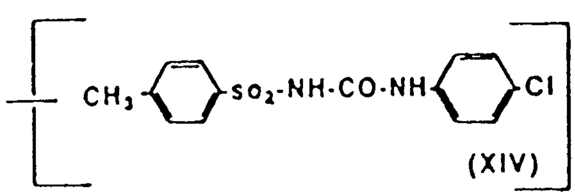

(XIV)

ou 5-uracile.

5. Composé selon la Revendication 4, où $R_1$ représente un groupe acétyle, un groupe propionyle, un groupe butanoyle, un groupe isopropionyle, un groupe pivaloyle, un groupe méthoxyméthyle, un groupe phénylacétyle, un groupe phénoxyacétyle, un groupe 2-(aminométhyl)phénylacétyle, un groupe 2-phényl-2-hydroxyacétyle, un groupe 2-phényl-2-(sulfonato sodique)acétyle, un groupe 2-phényl-2-2-carboxyacétyle, un groupe 2-(4-hydroxyphényl)-2-carboxyacétyle, un groupe 2-phényl-2-aminoacétyle, un groupe 2-(4-hydroxyphényl)-2-aminoacétal, un groupe 2-(3-(N-(méthyl-sulfonyl-amino))-phényl)-2-aminoacétyle, un groupe 2-phényl-2-(5-indanyl-carboxylate)acétyle, un groupe 2-phényl-2-(phénylcarboxylate)acétyle, un groupe 2-phényl-2-azidoacétyle, un groupe 2-phénoxypropionyle, un groupe 2,5-diméthoxybenzoyle, un groupe 2-(formyloxy)-2-phénylacétyle, un groupe 2-(2-éthoxynapht-1-yl)acétyle, un groupe 2-(napht-1-yl)-2-aminoacétyle, un groupe 2-(napht-2-yl)-2-aminoacétyle, un groupe 2-(2,5-dichlorophénylthio)acétyle, un groupe 2-(3,4-dichlorophénylthio)acétyle, un groupe 2-(1-tétrazolyl)-acétyle, un groupe 2-(N-(3,5-dichloropyrid-4-oxyl))acétyle, un groupe 2-(2-aminothiazol-4-yl)acétyle, un groupe 2-(2-thiényl)acétyle, un groupe 2-(4-pyridylthio)acétyle, un groupe 2-(N-méthyl-4-pyridiniumthio)acétyle, un groupe 2-(2-amino-4-phénylthiazol-5-yl)acétyle, un groupe 2-(3-hydroxy-4-carboxyisothiazol-5-ylthio)acétyle, un groupe 3-phényl-5-méthyl-isoxazolyl-3-formyle, 3-(2-chlorophényl)-5-méthylisoxazolyl-3-formyle, un groupe 3-(2,5-dichlorophényl)-5-méthylisoxazolyl-3-formyle, un groupe 3-(2-fluoro-5-chlorophényl)-5-méthylisoxazolyl-3-formyle,un groupe 2-(2-thiényl)-2-aminoacétyle, un groupe 2-(2-thiényl)-2-(carboxylate sodique)acétyle, un groupe 2-(N-(4-pyridinium))acétyle, un groupe 2-(2-benzothiényl)acétyle, un groupe 2-(3-benzothiényl)acétyle, un groupe 2-(2-benzofuryl)acétyle et un groupe 2-(3-benzofuryl)acétyle.

6. Composé, tel que revendiqué à la Revendication 5, où l'Agent Cytotoxique est le méthotrexate, le 5-fluorouracile, le désacétylvinblastine-hydrazide, le désacétylvinblastine-aminoéthanethiol une fraction désacétylvinblastinehydrazodécarboxyle, une fraction 7-(carboxyamino)désacétylcolchicine, la N-(p-tosyl)-N'-(p-chlorophényl)urée ou la N-(((4-chlorophényl)amino)carbonyl)-2,3-dihydro-1H-indéne-5-sulfonamide.

7. Composé, tel que revendiqué à la Revendication 6, où $R_1$ représente un groupe 2-(thién-2-yl)acétyle et ZZ vaut 0.

8. Composé, tel que revendiqué à la Revendication 7, où l'Agent Cytotoxique est le méthotrexate.

9. Composé, tel que revendiqué à la Revendication 8, où le méthotrexate est lié par l'intermédiaire de son groupe carboxyle au Substrat céphalosporine.

10. Composé, tel que revendiqué à la Revendication 7, où l'Agent Cytotoxique est le 5-fluorouracile.

11. Composé, tel que revendiqué à la Revendication 10, où le 5-fluorouracile est lié par l'intermédiaire de son atome d'azote $N_1$ au Substrat céphalosporine.

12. Composé, tel que revendiqué à la Revendication 7, où l'Agent Cytotoxique est le désacétylvinblastine-hydrazide.

13. Composé, tel que revendiqué à la Revendication 12, où le désacétylvinblastine-hydrazide est lié par l'intermédiaire de l'atome d'azote, par ailleurs non-lié, de son groupe hydrazido au Substrat céphalosporine.

14. Composé, tel que revendiqué à la Revendication 7, où l'Agent Cytotoxique est le désacétylvinblastine-aminoéthanethiol.

15. Composé, tel que revendiqué à la Revendication 14, où le désacétylvinblastine-aminoéthanethiol est lié par l'intermédiaire du groupe thiol de sa fraction aminoéthanethiol au Substrat céphalosporine.

16. Composé, tel que revendiqué à la Revendication 7, où l'Agent Cytotoxique est une fraction désacétylvinblastine-hydrazidocarboxyle.

**17.** Composé, tel que revendiqué à la Revendication 16, où la fraction désacétylvinblastine est liée par l'intermédiaire d'un atome d'oxygène du groupe hydrazidocarboxyle au substrat céphalosporine.

**18.** Composé, tel que revendiqué à la Revendication 7, où l'Agent Cytotoxique est la N-(p-tosyl)N'-(p-chlorophényl)urée.

**19.** Composé, tel que revendiqué à la Revendication 18, où la N-(p-tosyl)-N'-(p-chlorophényl)urée est liée par l'intermédiaire de l'atome d'azote p-tosyle au Substrat céphalosporine.

**20.** Composé, tel que revendiqué à la Revendication 6, où $R_1$ représente un groupe 2-(thién-2-yl)acétyle et ZZ vaut 1.

**21.** Composé, tel que revendiqué à la Revendication 20, où l'Agent Cytotoxique est une fraction 7-(carboxyamino)désacétylcolchicine.

**22.** Composé, tel que revendiqué à la Revendication 21, où la fraction 7-(carboxyamino)désacétylcolchicine est liée par l'intermédiaire d'un atome d'oxygène du groupe 7-(carboxyamino) au Substrat céphalosporine.

**23.** Composé, tel que revendiqué à la Revendication 20, où l'Agent Cytotoxique est le désacétylvinblastine-aminoéthanethiol.

**24.** Composé, tel que revendiqué à la Revendication 23, où le désacétylvinblastine-aminoéthanethiol est lié par l'intermédiaire du groupe thiol du groupe aminoéthanethiol au Substrat céphalosporine.

**25.** Composé, tel que revendiqué à la Revendication 20, où l'Agent Cytotoxique est la fraction désacétylvinblastine-hydrazidocarboxyle.

**26.** Composé, tel que revendiqué à la Revendication 25, où la fraction désacétylvinblastine-hydrazidocarboxyle est liée par l'intermédiaire d'un atome d'oxygène du groupe hydrazidocarboxyle à la fraction céphalosporine.

**27.** Composé, tel que revendiqué à la Revendication 6, où $R_1$ représente un groupe phénoxyacétyle et ZZ vaut 1.

**28.** Composé, tel que revendiqué à la Revendication 27, où l'Agent Cytotoxique est la fraction désacétylvinblastine-hydrazidocarboxyle.

**29.** Composé, tel que revendiqué à la Revendication 28, où la fraction désacétylvinblastine-hydrazidocarboxyle est liée par l'intermédiaire d'un atome d'oxygène du groupe hydrazidocarboxyle au Substrat céphalosporine.

**30.** Composé conjugué anticorps-enzyme répondant à la Formule (I) :

Anticorps-Enzyme (I)

où :

l'Enzyme réagit avec un composé selon l'une quelconque des Revendications 1 à 29 et provoque la séparation de l'Agent Cytotoxique du Substrat; et

l'Anticorps présente une spécificité pour un antigène exprimé par le tissu cible et où l'enzyme est la béta-lactamase.

**31.** Composé, tel que revendiqué à la Revendication 30, où l'Anticorps se complexe avec des antigènes exprimés uniquement, ou en quantités surnaturelles, par des cellules tumorales malignes.

**32.** Composé, tel que revendiqué à la Revendication 31, où l'Anticorps présente une spécificité pour l'antigène carcino-embryonnaire.

**33.** Composé, tel que revendiqué à la Revendication 32, où l'Anticorps est le ZCE025.

**34.** Composé, tel que revendiqué à la Revendication 32, où l'Anticorps est le CEM 231.6.7.

**35.** Composé, tel que revendiqué à la Revendication 31, où l'Anticorps est le T101.

**36.** Composé, tel que revendiqué à la Revendication 31, où l'Anticorps présente une spécificité pour l'antigène KS1/4.

**37.** Composé, tel que revendiqué dans l'une quelconque des Revendications 30 à 36, où l'Anticorps est un fragment F(ab').

**38.** Formulation pharmaceutique, qui comprend comme ingrédient un composé selon l'une quelconque des Revendications 1 à 29, associé avec un support, diluant ou excipient pharmaceutiquement acceptable pour celui-ci.

**39.** Formulation pharmaceutique, qui comprend comme ingrédient un composé selon l'une quelconque des Revendications 30 à 37, associé avec un support, diluant ou excipient pharmaceutiquement acceptable pour celui-ci.

**40.** Système prêt-à-l'emploi adapté pour le traitement d'une maladie néoplastique, qui comprend un conjugué Anticorps-Enzyme répondant à la Formule (I), tel que défini dans l'une quelconque des Revendications 30 à 37 et un composé Substrat-Agent Cytotoxique répondant à la Formule (II), tel que défini dans l'une quelconque des Revendications 1 à 29.

**41.** Composé répondant à la Formule (I), tel que défini dans l'une quelconque des Revendications 30 à 37, destiné à l'utilisation dans le traitement d'une maladie néoplastique.

**42.** Composé répondant à la Formule (II), tel que défini dans l'une quelconque des Revendications 1 à 29, destiné à l'utilisation dans le traitement d'une maladie néoplastique.

**Revendications pour l'Etat contractant suivant : ES**

**1.** Procédé pour la préparation d'un composé répondant à la Formule (II) :

Substrat-Agent Cytotoxique (II)

où le Substrat représente une molécule qui est un substrat pour une enzyme, où ladite enzyme clive, et par conséquent sépare, le substrat de l'Agent Cytotoxique, comprenant les étapes de :

a) protection de tout groupe carboxyle, amino, thiol ou hydroxyle sur le Substrat ou l'Agent Cytotoxique,

b) déplacement d'un groupe sortant soit sur le Substrat, soit sur l'Agent Cytotoxique, avec un groupe nucléophile de l'autre composé, Substrat ou Agent Cytotoxique, sous conditions $S_n1$ ou $S_n2$, et

c) déprotection des groupes protégés, où le Substrat est une molécule qui est un substrat pour la béta-lactamase.

**2.** Procédé, tel que revendiqué à la Revendication 1, où le Substrat est une céphalosporine répondant à la formule :

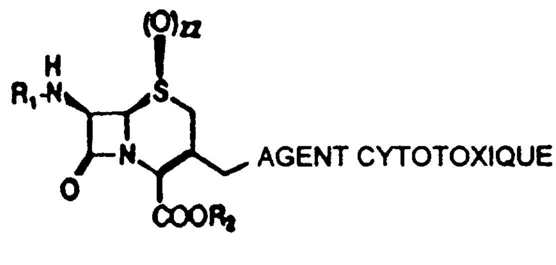

où ZZ vaut 0 ou 1, $R_1$ représente un groupe acyle dérivé d'un groupe alkyle en $C_1$ à $C_{30}$, et $R_2$ représente un atome d'hydrogène, un cation organique ou inorganique, un groupe carboxy-protecteur

ou un groupe formant un ester, non-toxique et métaboliquement instable.

**3.** Procédé selon la Revendication 2, où $R_1$ représente un groupe acyle répondant à la formule $-COR_3$ où $R_3$ représente :

i) un groupe alkyle en $C_1$ à $C_6$, un groupe alkyle en $C_1$ à $C_6$ substitué, un groupe alcoxy en $C_1$ à $C_4$, un groupe alkylthio en $C_1$ à $C_4$ ou un groupe répondant aux formules :

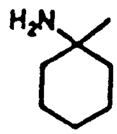 ;

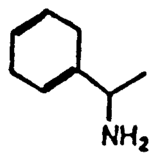 ;

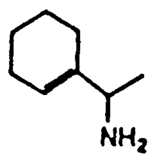 ;

$F_3C-S-CH_2-$ ;

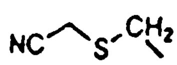 ;

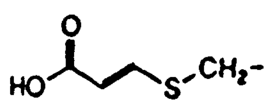 ; ou

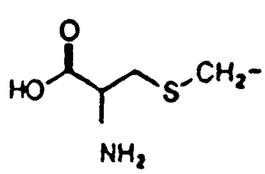 ;

ou un groupe amino protégé et/ou un dérivé carboxyle protégé de ceux-ci;

ii) un groupe répondant aux formules :

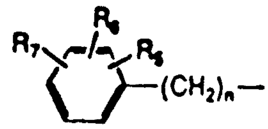
;

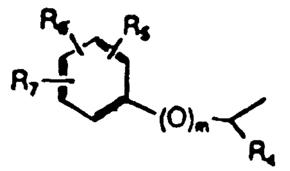
;

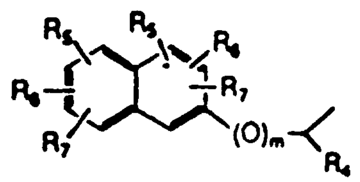
;

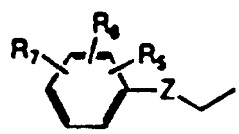
;

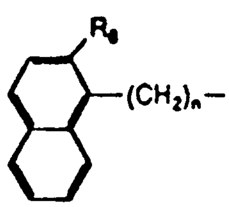
;

où chacun des groupes $R_5$, $R_6$ et $R_7$ représente, indépendamment l'un de l'autre, un atome d'hydrogène, un atome d'halogène, un groupe hydroxyle, un groupe hydroxyle protégé, un groupe nitro, un groupe amino, un groupe amino protégé, un sel d'amine, un groupe cyano, un groupe trifluorométhyle, un groupe aminométhyle, un groupe aminométhyle protégé, un groupe N-(méthyl- ou éthyl-sulfonyl)amino, un groupe alkyle en $C_1$ à $C_6$ ou un groupe alcoxy en $C_1$ à $C_4$, $R_4$ représente un groupe hydroxyle, un groupe hydroxyle protégé, un groupe formyloxy, un groupe amino, un groupe amino protégé, un sel d'amine, un groupe carboxyle, un sel carboxylate, un groupe carboxyle protégé, un groupe phénylcarboxylate, un groupe (5-indanyl)carboxylate, un acide sulfonique, un sel sulfonate, un groupe azido, un atome d'halogène ou un groupe alkyle en $C_1$ à $C_6$; $R_8$ représente un groupe alcoxy en $C_1$ à $C_4$; Z représente un atome d'oxygène ou de soufre; n vaut 0, 1, 2 ou 3; et m vaut 0 ou 1;

iii) un groupe répondant à la formule :

$$R_9-(CH_2)_n- \quad ;$$

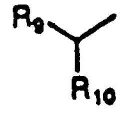
;

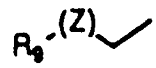

ou

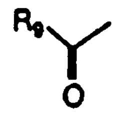

où $R_9$ représente un noyau hétérocyclique; $R_{10}$ représente un groupe hydroxyle, un groupe hydroxyle protégé, un groupe formyloxy, un groupe amino, un groupe amino protégé, un sel d'amine, un groupe carboxyle, un sel carboxylate, un groupe carboxyle protégé, un groupe phénylcarboxylate, un groupe (5-indanyl)carboxylate, un acide sulfonique, un sel sulfonate, un groupe azido, un atome d'halogène ou un groupe alkyle en $C_1$ à $C_6$; n vaut 0, 1, 2 ou 3; et Z représente un atome d'oxygène ou de soufre.

**4.** Procédé selon la Revendication 3, où l'Agent Cytotoxique est :

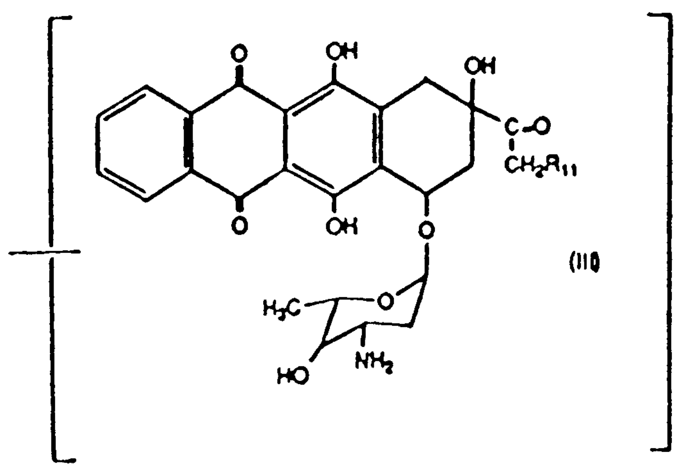

(III)

où $R_{11}$ représente un atome d'hydrogène ou un groupe hydroxyle;

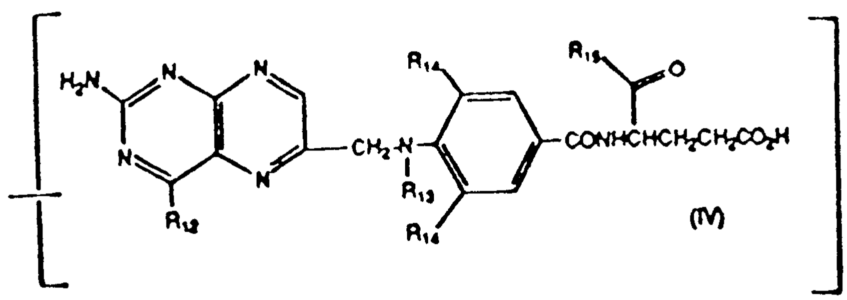

(IV)

où $R_{12}$ représente un groupe amino ou un groupe hydroxyle; $R_{13}$ représente un atome d'hydrogène ou un groupe méthyle; $R_{14}$ représente un atome d'hydrogène, un atome de fluor, un atome de chlore, un atome de brome, un atome d'iode; $R_{15}$ représente un groupe hydroxyle ou une fraction qui complète un sel de l'acide carboxylique;

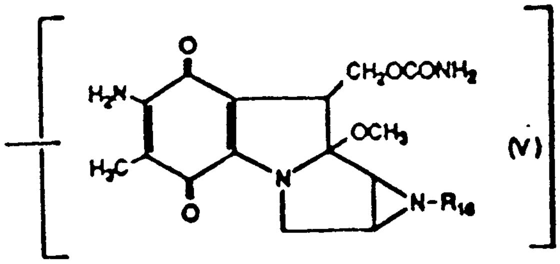

(V)

où $R_{16}$ représente un atome d'hydrogène ou un groupe méthyle;

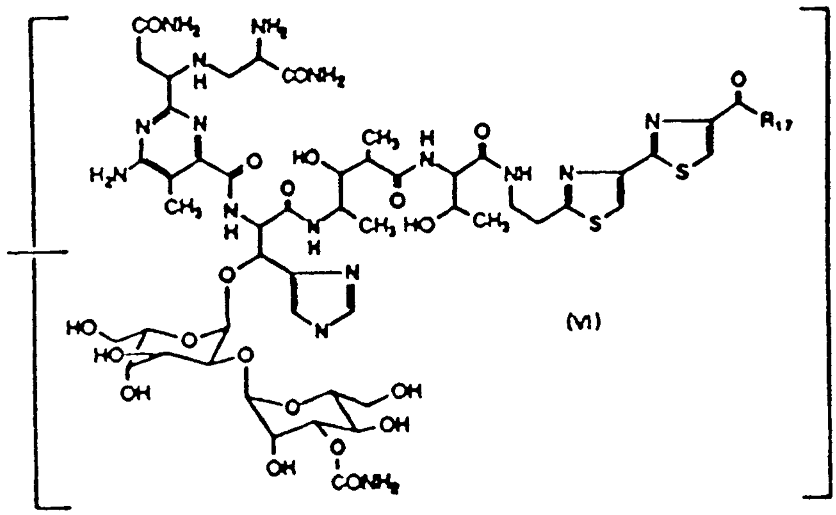

(VI)

où $R_{17}$ représente un groupe amino, un groupe alkylamino en $C_1$ à $C_3$, un groupe di-(alkyle en $C_1$ à $C_3$)amino ou un groupe (polyméthylène en $C_4$ à $C_6$)amino;

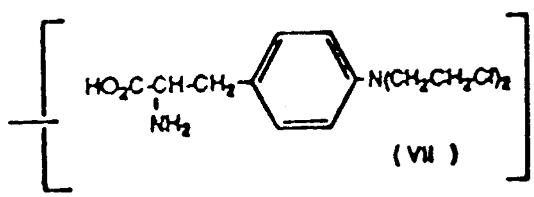

(VII)

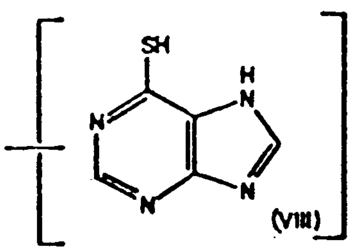

(VIII)

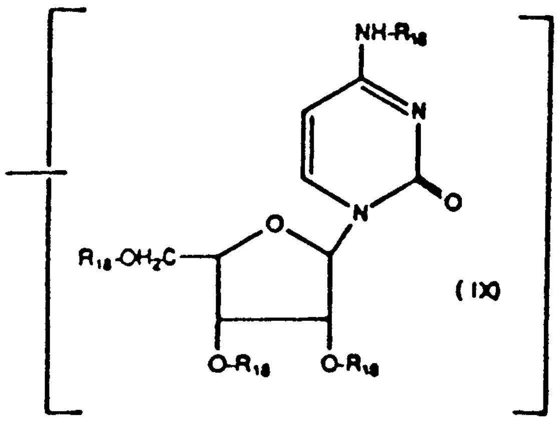

(IX)

où une des fractions $R_{18}$ représente une liaison et les autres sont des atomes d'hydrogène;

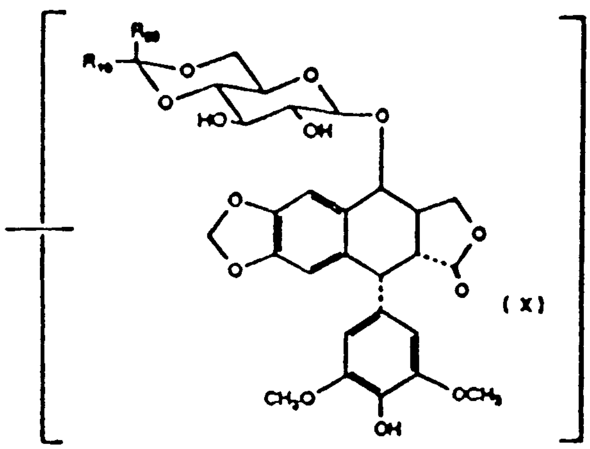

(X)

où $R_{19}$ représente un atome d'hydrogène ou un groupe méthyle; $R_{20}$ représente un groupe méthyle ou un groupe thiényle;

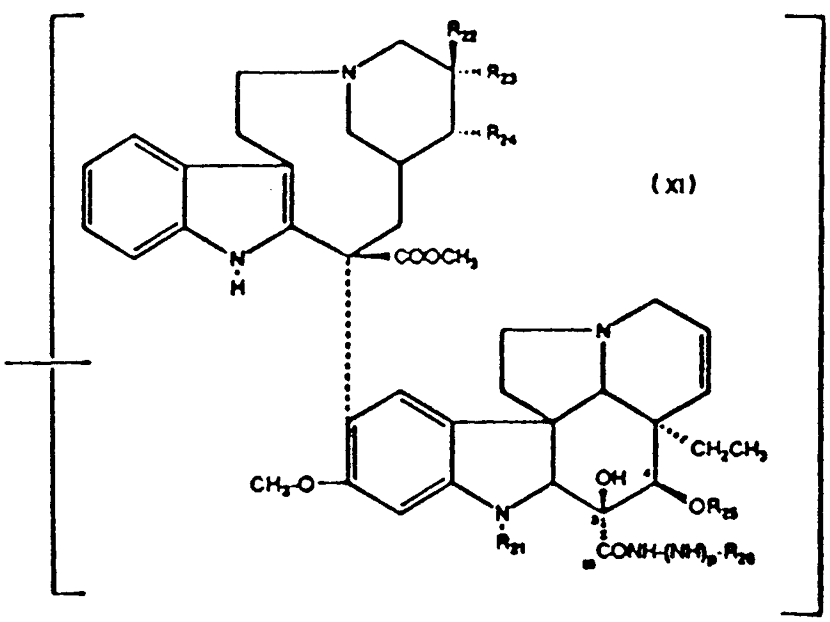

(XI)

où $R_{21}$ représente H, $CH_3$ ou CHO; lorsque $R_{23}$ et $R_{24}$ sont pris séparément, $R_{24}$ représente H, et un des groupes $R_{22}$ et $R_{23}$ représente un groupe éthyle et l'autre représente H ou OH; lorsque et $R_{23}$ et $R_{24}$ sont pris ensemble avec les atomes de carbone auxquels ils sont liés, ils forment un noyau oxirane, auquel cas $R_{22}$ représente un groupe éthyle; $R_{25}$ représente un atome d'hydrogène, un groupe (alkyle en $C_1$ à $C_3$)-CO- ou un groupe (alkyle en $C_1$ à $C_3$)-CO chloro-substitué;

p vaut 0 ou 1;

$R_{26}$ représente une liaison, un groupe -(alkyle en $C_2$ à $C_4$)-X ou un groupe qui requiert que p vaut 1 et qui est à son tour lié à un groupe carbonyloxy;

X représente -O-, -S- ou -NH-;

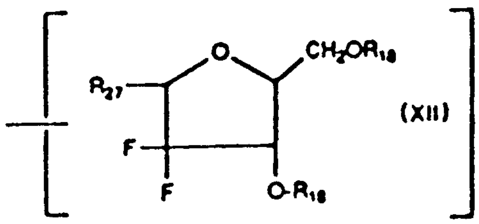
(XII)

où $R_{27}$ représente une base d'une des formules :

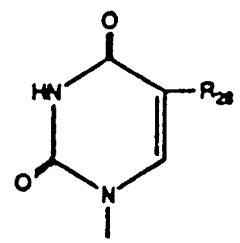
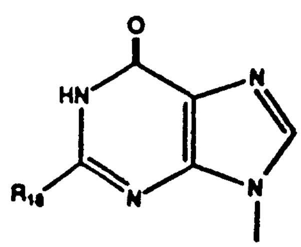

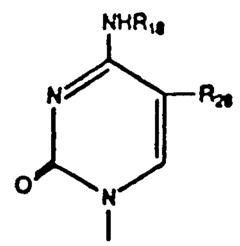
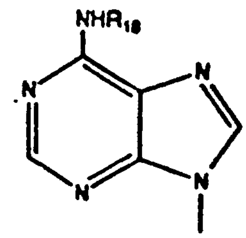

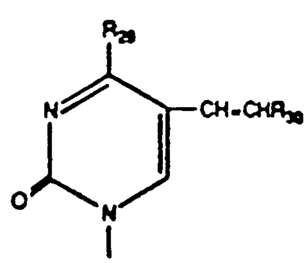

où $R_{28}$ représente un atome d'hydrogène, un groupe méthyle, un atome de brome, un atome de fluor, un atome de chlore ou un atome d'iode;

$R_{29}$ représente $-OR_{18}$ ou $-NHR_{18}$;

$R_{30}$ représente un atome d'hydrogène, un atome de brome, un atome de chlore ou un atome d'iode;

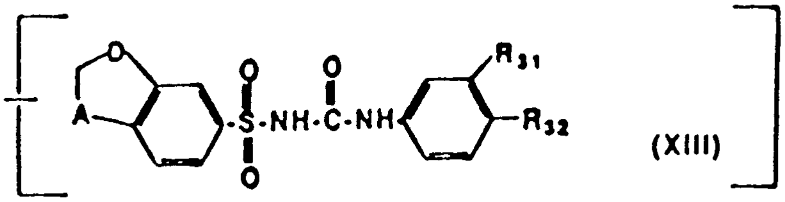

(XIII)

où A représente -O-, $-NCH_3-$, $-CH_2-$, $-CH_2CH_2-$ ou $-CH_2O-$;
D représente $-CH_2-$ ou -O-;
$R_{31}$ représente un atome d'hydrogène ou un atome d'halogène;
$R_{32}$ représente un atome d'halogène ou un groupe trifluorométhyle;

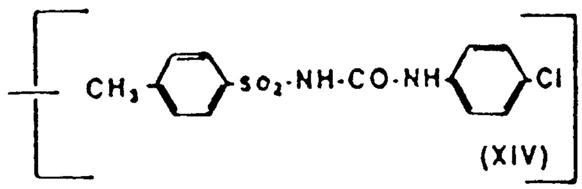

(XIV)

ou 5-uracile.

**5.** Procédé selon la Revendication 4, où $R_1$ représente un groupe acétyle, un groupe propionyle, un groupe butanoyle, un groupe isopropionyle, un groupe pivaloyle, un groupe méthoxyméthyle, un groupe phénylacétyle, un groupe phénoxyacétyle, un groupe 2-(aminométhyl)phénylacétyle, un groupe 2-phényl-2-hydroxyacétyle, un groupe 2-phényl-2-(sulfonato sodique)acétyle, un groupe 2-phényl-2-2-carboxyacétyle, un groupe 2-(4-hydroxyphényl)- 2-carboxyacétyle, un groupe 2-phényl-2-aminoacétyle, un groupe 2-(4-hydroxyphényl)-2-aminoacétal, un groupe 2-(3-(N-(méthyl-sulfonyl-amino))phényl)-2-aminoacétyle, un groupe 2-phényl-2-(5-indanyl-carboxylate)acétyle un groupe 2-phényl-2-(phénylcarboxylate)acétyle, un groupe 2-phényl-2-azidoacétyle, un groupe 2-phénoxypropionyle, un groupe 2,5-diméthoxybenzoyle, un groupe 2-(formyloxy)-2-phénylacétyle, un groupe 2-(2-éthoxynapht-1-yl)acétyle, un groupe 2-(napht-1-yl)-2-aminoacétyle, un groupe 2-(napht-2-yl)-2-aminoacétyle, un groupe 2-(2,5-dichlorophénylthio)acétyle, un groupe 2-(3,4-dichlorophénylthio)acétyle, un groupe 2-(1-tétrazolyl)acétyle, un groupe 2-(N-(3,5-dichloropyrid-4-oxyl))acétyle, un groupe 2-(2-aminothiazol-4-yl)acétyle, un groupe 2-(2-thiényl)acétyle, un groupe 2-(4-pyridylthio)acétyle, un groupe 2-(N-méthyl-4-pyridiniumthio)acétyle, un groupe 2-(2-amino-4-phénylthiazol-5-yl)acétyle, un groupe 2-(3-hydroxy-4-carboxyisothiazol-5-ylthio)-acétyle, un groupe 3-phényl-5-méthyl-isoxazolyl-3-formyle, un groupe 3-(2-chlorophényl)-5-méthylisoxazolyl-3-formyle, un groupe 3-(2,5-dichlorophényl)-5-méthylisoxazolyl-3-formyle, un groupe 3-(2-fluoro-5-chlorophényl)-5-méthylisoxazolyl-3-formyle,un groupe 2-(2-thiényl)-2-aminoacétyle un groupe 2-(2-thiényl)-2-(carboxylate sodique)acétyle, un groupe 2-(N-(4-pyridinium))acétyle, un groupe 2-(2-benzothiényl)acétyle, un groupe 2-(3-benzothiényl)acétyle, un groupe 2-(2-benzofuryl)acétyle et un groupe 2-(3-benzofuryl)acétyle.

**6.** Procédé, tel que revendiqué à la Revendication 5, où l'Agent Cytotoxique est le méthotrexate, le 5-fluorouracile, le désacétylvinblastine-hydrazide, le désacétylvinblastine-aminoéthanethiol, une fraction désacétylvinblastine-hydrazodécarboxyle, une fraction 7-(carboxyamino)désacétylcolchicine, la N-(p-tosyl)-N'-(p-chlorophényl)urée ou la N-(((4-chlorophényl)amino)carbonyl)-2,3-dihydro-1H-indène-5-sulfonamide.

**7.** Procédé, tel que revendiqué à la Revendication 6, où $R_1$ représente un groupe 2-(thién-2-yl)acétyle et ZZ vaut 0.

**8.** Procédé, tel que revendiqué à la Revendication 7, où l'Agent Cytotoxique est le méthotrexate.

**9.** Procédé, tel que revendiqué à la Revendication 8, où le méthotrexate est lié par l'intermédiaire de son groupe carboxyle au Substrat céphalosporine.

**10.** Procédé, tel que revendiqué à la Revendication 7, où l'Agent Cytotoxique est le 5-fluorouracile.

**11.** Procédé, tel que revendiqué à la Revendication 10, où le 5-fluorouracile est lié par l'intermédiaire de son atome d'azote $N_1$ au Substrat céphalosporine.

**12.** Procédé, tel que revendiqué à la Revendication 7, où l'Agent Cytotoxique est le désacétylvinblastine-hydrazide.

**13.** Procédé, tel que revendiqué à la Revendication 12, où le désacétylvinblastine-hydrazide est lié par l'intermédiaire de l'atome d'azote, par ailleurs non-lié, de son groupe hydrazido au Substrat céphalosporine.

**14.** Procédé, tel que revendiqué à la Revendication 7, où l'Agent Cytotoxique est le désacétylvinblastine-aminoéthanethiol.

**15.** Procédé, tel que revendiqué à la Revendication 14, où le désacétylvinblastine-aminoéthanethiol est lié par l'intermédiaire du groupe thiol de sa fraction aminoéthanethiol au Substrat céphalosporine.

**16.** Procédé, tel que revendiqué à la Revendication 7, où l'Agent Cytotoxique est une fraction désacétylvinblastine-hydrazidocarboxyle.

**17.** Procédé, tel que revendiqué à la Revendication 16, où la fraction désacétylvinblastine est liée par l'intermédiaire d'un atome d'oxygène du groupe hydrazidocarboxyle au substrat céphalosporine.

**18.** Procédé, tel que revendiqué à la Revendication 7, où l'Agent Cytotoxique est la N-(p-tosyl)-N'-(p-chlorophényl)urée.

**19.** Procédé, tel que revendiqué à la Revendication 18, où la N-(p-tosyl)-N'-(p-chlorophényl)urée est liée par l'intermédiaire de l'atome d'azote p-tosyle au Substrat céphalosporine.

**20.** Procédé, tel que revendiqué à la Revendication 6, où $R_1$ représente un groupe 2-(thién-2-yl)acétyle et ZZ vaut 1.

**21.** Procédé, tel que revendiqué à la Revendication 20, où l'Agent Cytotoxique est une fraction 7-(carboxyamino)désacétylcolchicine.

**22.** Procédé, tel que revendiqué à la Revendication 21, où la fraction 7-(carboxyamino)désacétylcolchicine est liée par l'intermédiaire d'un atome d'oxygène du groupe 7-(carboxyamino) au Substrat céphalosporine.

**23.** Procédé, tel que revendiqué à la Revendication 20, où l'Agent Cytotoxique est le désacétylvinblastine-aminoéthanethiol.

**24.** Procédé, tel que revendiqué à la Revendication 23, où le désacétylvinblastine-aminoéthanethiol est lié par l'intermédiaire du groupe thiol du groupe aminoéthanethiol au Substrat céphalosporine.

**25.** Procédé, tel que revendiqué à la Revendication 20, où l'Agent Cytotoxique est la fraction désacétylvinblastine-hydrazidocarboxyle.

**26.** Procédé, tel que revendiqué à la Revendication 25, où la fraction désacétylvinblastine-hydrazidocarboxyle est liée par l'intermédiaire d'un atome d'oxygène du groupe hydrazidocarboxyle à la fraction céphalosporine.

**27.** Procédé, tel que revendiqué à la Revendication 6, où $R_1$ représente un groupe phénoxyacétyle et ZZ vaut 1.

**28.** Procédé, tel que revendiqué à la Revendication 27, où l'Agent Cytotoxique est la fraction désacétylvinblastine-hydrazidocarboxyle.

**29.** Procédé, tel que revendiqué à la Revendication 28, où la fraction désacétylvinblastine-hydrazidocarboxyle est liée par l'intermédiaire d'un atome d'oxygène du groupe hydrazidocarboxyle au Substrat céphalosporine.

**30.** Procédé pour la préparation d'un composé conjugué anticorps-enzyme répondant à la Formule (I) :

Anticorps-Enzyme (I)

où :

l'Enzyme réagit avec un composé préparé par un procédé selon l'une quelconque des Revendications 1 à 31 et provoque la séparation de l'Agent Cytotoxique du Substrat; et

l'Anticorps présente une spécificité pour un antigène exprimé par le tissu cible, qui comprend la conjugaison d'une Enzyme et d'un Anticorps en utilisant un réactif couplant protéine-protéine, où l'Enzyme est une béta-lactamase.

**31.** Procédé, tel que revendiqué à la Revendication 30, où l'Anticorps se complexe avec des antigènes exprimés uniquement, ou en quantités surnaturelles, par des cellules tumorales malignes.

**32.** Procédé, tel que revendiqué à la Revendication 31, où l'Anticorps présente une spécificité pour l'antigène carcino-embryonnaire.

**33.** Procédé, tel que revendiqué à la Revendication 32, où l'Anticorps est le ZCE025.

**34.** Procédé, tel que revendiqué à la Revendication 32, où l'Anticorps est le CEM 231.6.7.

**35.** Procédé, tel que revendiqué à la Revendication 31, où l'Anticorps est le T101.

**36.** Procédé, tel que revendiqué à la Revendication 31, où l'Anticorps présente une spécificité pour l'antigène KS1/4.

**37.** Procédé, tel que revendiqué dans l'une quelconque des Revendications 30 à 36, où l'Anticorps est un fragment F(ab').

**38.** Procédé pour la préparation d'une composition contenant un composé Anticorps-Enzyme répondant à la Formule (I), tel que défini dans l'une quelconque des Revendications 30 à 37, qui comprend la combinaison du composé avec un support pharmaceutiquement acceptable de façon à procurer une formulation solide ou liquide pour une administration par voie orale, par voie topique ou par injection.

**39.** Procédé pour la préparation d'une composition contenant un composé Substrat-Agent Cytotoxique répondant à la Formule (II), tel que défini dans l'une quelconque des Revendications 1 à 29, qui comprend la combinaison du composé avec un support pharmaceutiquement acceptable de façon à procurer une formulation solide ou liquide pour une administration par voie orale, par voie topique ou par injection.

**40.** Système prêt-à-l'emploi formé de parties adaptées pour le traitement d'une maladie néoplastique, comprenant une composition préparée par le procédé de la Revendication 38 ou la Revendication 39.